(19) Europäisches Patentamt
European Patent Office
Office européen des brevets

(11) Veröffentlichungsnummer: **0 243 903 B1**

(12) **EUROPÄISCHE PATENTSCHRIFT**

(45) Veröffentlichungstag der Patentschrift: **18.03.92**

(51) Int. Cl.$^5$: **C07D 295/08**, C07D 295/18, A61K 31/495

(21) Anmeldenummer: **87106032.3**

(22) Anmeldetag: **28.04.87**

Die Akte enthält technische Angaben, die nach dem Eingang der Anmeldung eingereicht wurden und die nicht in dieser Patentschrift enthalten sind.

# (54) Gegebenenfalls substituierte, 1-(w[bis-(Phenyl)-alkoxy]-alkyl)-4-(alkenyl)-piperazine und -4-(alkinyl)-piperazine, Verfahren zu ihrer Herstellung und diese enthaltende Arzneimittel.

(30) Priorität: **28.04.86 HU 174986**

(43) Veröffentlichungstag der Anmeldung:
**04.11.87 Patentblatt 87/45**

(45) Bekanntmachung des Hinweises auf die Patenterteilung:
**18.03.92 Patentblatt 92/12**

(84) Benannte Vertragsstaaten:
**AT BE CH DE FR GB IT LI LU NL SE**

(56) Entgegenhaltungen:
**GB-A- 837 986**

**CHEMICAL ABSTRACTS, Band 104, Nr. 17, 28. April 1986, Seite 17, Zusammenfassung Nr. 141709r, Columbus, Ohio, US; P. VAN DER ZEE et al.: "Interaction between substituted 1-[2-(diphenylmethoxy)ethyl]piperazines and dopamin e receptors"**

(73) Patentinhaber: **Richter Gedeon Vegyészeti Gyár R.T.**
**Gyömröi ut 19-21**
**H-1475 Budapest X(HU)**

(72) Erfinder: **Toth, Edit**
**Szabolcska M. u. 7**
**H 1114 Budapest(HU)**
Erfinder: **Kiss, Béla**
**Vöröshadsereg utja 197/c**
**H 2220 Vecsés(HU)**
Erfinder: **Törley, Jozsef**
**Katona J. u. 41**
**H 1137 Budapest(HU)**
Erfinder: **Pálosi, Eva, Dr.**
**Vend u. 21**
**H 1025 Budapest(HU)**
Erfinder: **Hajdu, István**
**Tátra tér B/4**
**H 1205 Budapest(HU)**
Erfinder: **Szporny, Lászlo, Dr.**
**M. u. 7.**
**H 1114 Szabolcska(HU)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**INDUSTRIE CHIMIQUE BELGE, Band T.XIX, Nr. 11, 1954, Seiten 1176-1196; H. MQRREN et al.: "Nouveaux dérivés 1.4-disubstitués de la pipérazine; Etude de leurs propriétés antihistaminiques"**

**NEURQPHARMACQLQGY, 1985, 24 (12), pages 1171-1174**

Erfinder: **Groo, Dora, Dr.**
**Napraforgo u. 17**
**H 1021 Budapest(HU)**
Erfinder: **Lapis, Erzsébet, Dr.**
**Abaliget u. 86**
**H 1172 Budapest(HU)**
Erfinder: **Laszlovszky, István, Dr.**
**Bartok B. u. 16**
**H 1111 Budapest(HU)**

(74) Vertreter: **Beszédes, Stephan G., Dr. Patentanwalt**
**Münchener Strasse 80a Postfach 1168**
**W-8060 Dachau(DE)**

## Beschreibung

Die Erfindung betrifft neue, gegebenenfalls substituierte, 1-{ω-[bis-(Phenyl)-alkoxy]-alkyl}-4-{alkenyl}-piperazine und -4-{alkinyl}-piperazine, ein Verfahren zu ihrer Herstellung und diese Verbindungen enthaltende Arzneimittel, insbesondere mit dopaminerger Wirkung auf das Zentralnervensystem.

Die Herstellung von, gegebenenfalls substituierten, 1-{ω-[bis-(Phenyl)-alkoxy]-alkyl}-4-{alkyl}-piperazine, -4-{alkenyl}-piperazine, -4-{alkinyl}-piperazine beziehungsweise -4-{acyl}-piperazine ist in CA 52:12873i [H.G. Morren: Ind. Chim. belge 22, 409-20 (1957)], CA 53:20101f und 54:12169a [belgische Patentschriften 551 032 und 549 420] sowie CA 85:160163p [französische Patentschrift 2 276 824] beschrieben. Gemäß den pharmakologischen Untersuchungen haben diese Verbindungen neben einer schwachen Antihistaminwirkung eine starke Hemmwirkung auf Magengeschwüre, darüber hinaus jedoch keinerlei pharmakologische Wirkung, und die in der französischen Patentschrift 2 276 824 beschriebenen Verbindungen wirken hustenstillend.

Aus Neuropharmakology 24 (12), pp. 1171-1174 (1985) ist die Interaktion zwischen substituierten 1-[2-(Diphenylmethoxy)-äthyl]-piperazinen und Dopaminrezeptoren bekannt. Es wurde auch eine Korrelation zwischen den $IC_{50}$-Werten der Bindungstests und den $IC_{50}$-Werten für die Hemmung bei der Dopaminaufnahme an synaptosomalen Präparationen des Striatums der Ratte gefunden.

Der Erfindung liegt die Aufgabe zugrunde, überlegene pharmakologische Wirkungen aufweisende neue 1,4-disubstituierte Piperazinderivate, ein Verfahren zu ihrer Herstellung und diese Verbindungen enthaltende Arzneimittel zu schaffen.

Das Obige wurde überraschenderweise durch die Erfindung erreicht.

Gegenstand der Erfindung sind, gegebenenfalls substituierte, 1-{ω-[bis-(Phenyl)-alkoxy]-alkyl}-4-{alkenyl}-piperazine und -4-{alkenyl}-piperazine der allgemeinen Formel

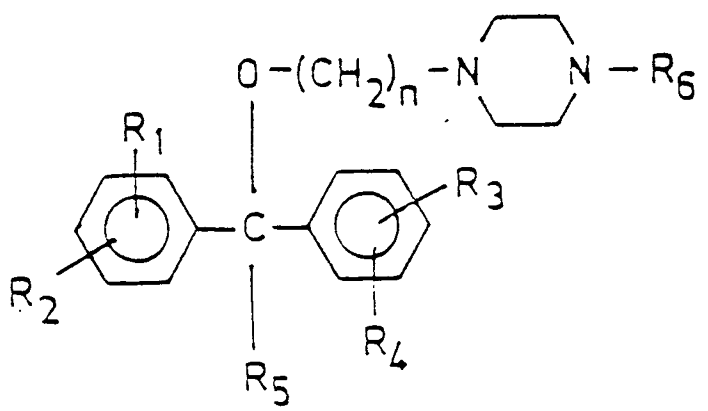

I ,

worin

| | |
|---|---|
| $R_1$, $R_2$, $R_3$ und $R_4$ | unabhängig voneinander für Wasserstoffatome, Halogenatome, Trihalogenmethylreste, $C_1$-$C_4$-Alkylreste, $C_1$-$C_4$-Alkoxyreste, Nitrogruppen, Hydroxygruppen, 1-(Prop-2'-enyl)piperazin-4-ylreste oder Phenyl-$C_1$-$C_4$-alkoxyreste stehen, |
| $R_5$ | ein Wasserstoffatom oder einen Alkylrest mit 1 bis 4 Kohlenstoffatom(en) bedeutet, |
| $R_6$ | ein Alkenyl- oder Alkinylrest mit 3 bis 6 Kohlenstoffatomen ist und |
| n | gleich 2 oder 3 ist; |

sowie ihre Säureadditionssalze, quaternären Salze und optisch aktiven Isomere und Solvate.

Die erfindungsgemäßen 1-{ω-[bis-(Phenyl)-alkoxy]-alkyl}-4-{alkenyl}-piperazine und -4-{alkinyl}-piperazine können 1 oder mehrere asymmetrische Kohlenstoffatome haben und daher in verschiedenen Sterooisomerformen vorkommen. Sie können also Basen, Säureadditionssalze, quaternäre Salze, insbesondere quaternäre Ammoniumsalze, Racemate, getrennte optische Isomere und deren Gemische sowie Solvate, zum Beispiel Hydrate, sein.

Vorzugsweise ist beziehungsweise sind das beziehungsweise die Halogenatom(e), für welche[s] $R_1$ , $R_2$ , $R_3$ und/oder $R_4$ stehen kann beziehungsweise können, und/oder die Halogenatome des beziehungsweise der Trihalogenmethylreste[s], für welche[n] $R_1$ , $R_2$ , $R_3$ und/oder $R_4$ stehen kann beziehungsweise können, Fluor, Chlor und/oder Brom, insbesondere Fluor und/oder Chlor.

Von den $C_1$-$C_4$-Alkylresten, $C_1$-$C_4$-Alkoxyresten sowie den Alkoxyteilen von Phenyl-$C_1$-$C_4$-alkoxyresten

von $R_1$, $R_2$, $R_3$ und/oder $R_4$ sind solche mit 1 oder 2 Kohlenstoffatom(en) bevorzugt. Von den $C_1$-$C_4$-Alkylresten ist der Methylrest besonders bevorzugt. Als $C_1$-$C_4$-Alkoxyrest ist der Äthoxyrest besonders bevorzugt und von den Phenyl-$C_1$-$C_4$-Alkoxyresten ist der Benzyloxyrest besonders bevorzugt.

Ferner ist es bevorzugt, daß der Alkylrest, für welchen $R_5$ stehen kann, ein solcher mit 1 bis 3, insbesondere 1 oder 3, Kohlenstoffatom(en) ist.

Weiterhin ist es bevorzugt, daß der Alkyl-, Alkenyl- oder Alkinylrest, für welchen $R_6$ stehen kann, ein solcher mit 3 oder 4, insbesondere 3, Kohlenstoffatomen ist.

Weiterhin ist es bevorzugt, daß der (die) an einem oder mehreren Phenylring(en) gegebenenfalls vorliegende(n) Substituent(en) in der (den) 3- und/oder 4-Stellung(en) ist beziehungsweise sind.

Besonders bevorzugte erfindungsgemäße 1-{ω-[bis-Phenyl)-alkoxy]-alkyl}-4-{alkenyl}-piperazine oder -4-{alkinyl}-piperazine sind

1-{2'-[bis-⟨4''-(Fluor)-phenyl⟩-methoxy]-äthyl}-4-{prop-2'''-enyl}-piperazin,
1-{2'-[⟨4''-(Fluor)-phenyl⟩-⟨phenyl⟩-methoxy]-äthyl}-4-{prop-2'''-enyl}-piperazin,
1-{2'-[⟨4''-(Chlor)-phenyl⟩-⟨4'''-(fluor)-phenyl⟩-methoxy]-äthyl}-4-{prop-2''''-enyl}-piperazin,
1-{2'-[⟨4''-(Brom)-phenyl⟩-⟨4'''-(fluor)-phenyl⟩-methoxy]-äthyl}-4-{prop-2''''-enyl}-piperazin,
1-{2'-[bis-⟨4''-(Chlor)-phenyl⟩-methoxy]-äthyl}-4-{prop-2'''-enyl}-piperazin,
1-{2'-[⟨3''',4''-Di-(chlor)-phenyl⟩-⟨phenyl⟩-methoxy]-äthyl}-4-{prop-2'''-enyl}-piperazin,
1-{2'-[1''-bis-⟨4'''-(Fluor)-phenyl⟩-äthoxy]-äthyl}-4-{prop-2''''-enyl}-piperazin,
1-{2'-[bis-⟨4''-(Fluor)-phenyl⟩-methoxy]-äthyl}-4-{prop-2'''-inyl}-piperazin,
1-{2'-[bis-⟨4''-(Fluor)-phenyl⟩-methoxy]-äthyl}-4-{2'''-(methyl)-prop-2'''-enyl}-piperazin,
1-{2'-[⟨3''-(Nitro)-4''-(1'''-[prop-2''''-enyl]-piperazin-4'''-yl)-phenyl⟩-⟨phenyl⟩-methoxy]-äthyl}-4-{prop-2'''''-enyl}-piperazin,
1-{2'-[1''-⟨2''',5'''-Di-(methyl)-phenyl⟩-1''-⟨phenyl⟩-propoxy]-äthyl}-4-{prop-2''''-enyl}-piperazin,
1-{2'-[⟨Di-(phenyl)⟩-methoxy]-äthyl}-4-{1''-(oxo)-prop-2''-enyl}-piperazin,
1-{2'-[⟨Di-(phenyl)⟩-methoxy]-äthyl}-4-{prop-2''-enyl}-piperazin,
1-{2'-[⟨2''-(Chlor)-phenyl⟩-⟨4'''-(fluor)-phenyl⟩-methoxy]-äthyl}-4-{prop-2''''-enyl}-piperazin,
1-{2'-[⟨4''-(Trifluormethyl)-phenyl⟩-⟨4'''-(fluor)-phenyl⟩-methoxy]-äthyl}-4-{prop-2''''-enyl}-piperazin,
1-{2'-[⟨2''-(Methyl)-phenyl⟩-⟨4'''-(fluor)-phenyl⟩-ethoxy]-äthyl}-4-{prop-2''''-enyl}-piperazin,
1-{2'-[⟨3''-(Äthoxy)-4''-(hydroxy)-phenyl⟩-⟨4'''-(fluor)-phenyl⟩-methoxy]-äthyl}-4-{prop-2''''-enyl}-piperazin,
1-{2'-[⟨3''-(Trifluormethyl)-phenyl⟩-⟨4'''-(fluor)-phenyl⟩-methoxy]-äthyl}-4-{prop-2''''-enyl}-piperazin,
1-{2'-[⟨3''-(Trifluormethyl)-phenyl⟩-⟨phenyl⟩-methoxy]-äthyl}-4-{prop-2'''-enyl}-piperazin,
1-{2'-[⟨4''-(Benzyloxy)-phenyl⟩-⟨4'''-(fluor)-phenyl⟩-methoxy]-äthyl}-4-{prop-2''''-enyl}-piperazin,
1-{2'-[⟨4''-(Chlor)-phenyl⟩-⟨4'''-(fluor)-phenyl⟩-methoxy]-äthyl}-4-{prop-2''''-inyl}-piperazin,
1-{2'-[⟨4''-(Fluor)-phenyl⟩-⟨phenyl⟩-methoxy]-äthyl}-4-{prop-2'''-inyl}-piperazin,
1-{2'-[⟨3'',4''-Di-(chlor)-phenyl⟩-⟨phenyl⟩-methoxy]-äthyl}-4-{prop-2'''-inyl}-piperazin,
1-{2'-[⟨4''-(Brom)-phenyl⟩-⟨4'''-(fluor)-phenyl⟩-methoxy]-äthyl}-4-{prop-2''''-inyl}-piperazin,
1-{2'-[⟨4''-(Trifluormethyl)-phenyl⟩-⟨4'''-(fluor)-phenyl⟩-methoxy]-äthyl}-4-{prop-2''''-inyl}-piperazin,
1-{2'-[bis-⟨4''-(Chlor)-phenyl⟩-methoxy]-äthyl}-4-{prop-2'''-inyl}-piperazin, und
1-{2'-[bis-⟨4''-(Chlor)-phenyl⟩-methoxy]-äthyl}-4-{prop-2'''-enyl}-piperazin.

Die Säureadditionssalze können solche mit anorganischen oder organischen Säuren sein. Zweckmäßig sind sie solche mit physiologisch brauchbaren Säuren. Beispiele für solche sind Halogenwasserstoffsäuren, wie Salzsäure und Bromwasserstoffsäure, Schwefelsäure, Phosphorsäuren, Ameisensäure, Essigsäure, Propionsäure, Oxalsäure, Glykolsäure, Maleinsäure, Fumarsäure, Bernsteinsäure, Weinsäure, Ascorbinsäure, Citronensäure, Apfelsäure, Salicylsäure, Milchsäure, Benzoesäure, Zimtsäure, Asparaginsäure, Glutaminsäure, N-(Acetyl)-asparaginsäure, N-(Acetyl)-glutaminsäure, Alkylsulfonsäuren, zum Beispiel Methansulfonsäure, und Arylsulfonsäuren, zum Beispiel p-Toluolsulfonsäure.

Die quaternären Salze sind vorzugsweise quaternäre Ammoniumsalze. Zweckmäßig sind sie solche mit physiologisch brauchbaren Quaternisierungsmitteln. Beispiele für solche sind Benzylhalogenide und niedere Alykl- und Alkenylhalogenide und Alkylsulfate.

Gegenstand der Erfindung ist auch ein Verfahren zur Herstellung der erfindungsgemäßen Verbindungen, welches dadurch gekennzeichnet ist, daß,

a) gegebenenfalls substituierte, Benzhydrole beziehungsweise ihre Alkoholate der allgemeinen Formel

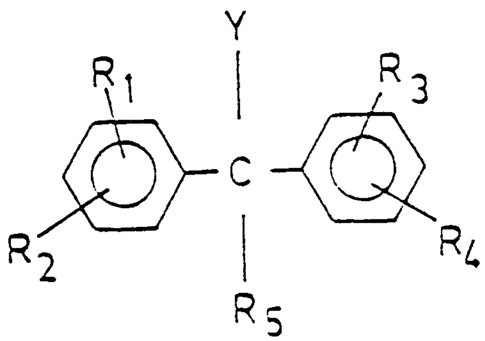

II ,

worin

$R_1$ , $R_2$ , $R_3$ , $R_4$ und $R_5$ die oben angegebenen Bedeutungen haben und

Y für eine Hydroxygruppe oder einen Rest der allgemeinen Formel

-OM,

in welchletzterer

M ein Alkalimetallatom oder einen Rest der Formel

-MgHlg,

in welchletzterer

Hlg ein Halogenatom ist,

bedeutet,

steht,

mit 1-(Alkenyl)- oder 1-(Alkinyl)-piperazin-4-ylalk-ω-ylhalogeniden beziehungsweise -4-ylalkan-ω-olen beziehungsweise 4-(Alkyl-beziehungsweise Arylsulfonyloxyalk-ω-yl)-piperazin-1-ylalkenen, -1-ylalkinen oder 1-ylalken- beziehungsweise -alkincarbonsäuren der allgemeinen Formel

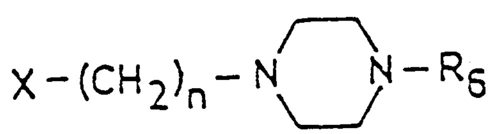

III ,

worin

$R_6$ und n die oben angegebenen Bedeutungen haben und

X für ein Halogenatom, eine Hydroxygruppe, einen Alkylsulfonyloxyrest oder einen Arylsulfonyloxyrest steht,

mit der Einschränkung, daß

X von einer Hydroxygruppe verschieden ist,

wenn Y einen Rest der allgemeinen Formel -OM bedeutet,

umgesetzt werden, oder,

b) gegebenenfalls substituierte, Benzhydrylhalogenide der allgemeinen Formel

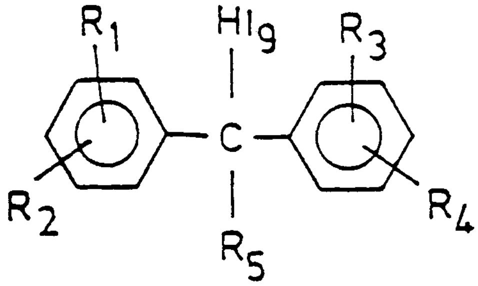

IV ,

worin

$R_1$ , $R_2$ , $R_3$ , $R_4$ und $R_5$ sowie n die oben angegebenen Bedeutungen haben,

mit 1-(Alkenyl)- oder 1-(Alkinyl)-piperazin-4-ylalkan-ω-olen beziehungsweise ihren Alkoholaten der allgemeinen Formel

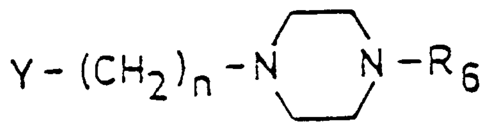

V ,

worin

$R_6$ und n die oben angegebenen Bedeutungen haben und

Y für eine Hydroxygruppe oder einen Metalloxyrest der allgemeinen Formel

-OM' ,

in welchletzterer

M' ein Alkalimetallatom bedeutet,

steht,

umgesetzt werden oder,

c) gegebenenfalls substituierte, [Di-(phenyl)]-[alkyl- beziehungsweise arylsulfonyloxyalk-ω-oxy]-alkane beziehungsweise [Di-(phenyl)]-[halogenalk-ω-oxy]-alkane der allgemeinen Formel

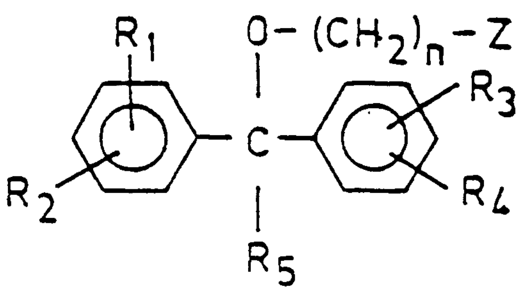

VI ,

worin

$R_1$ , $R_2$ , $R_3$ , $R_4$ und $R_5$ sowie n die oben angegebenen Bedeutungen haben und

Z für ein Halogenatom oder einen Alkyl- oder Arylsulfonyloxyrest steht,

mit 1-(Alkenyl)- oder 1-(Alkinyl)-piperazinen der allgemeinen Formel

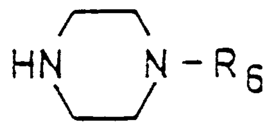

VII.

worin

$R_6$ die oben angegebenen Bedeutungen hat,

umgesetzt werden oder,

d) gegebenenfalls substituierte, 1-{ω-[bis-(Phenyl)-alkoxy]-alk-ω-yl}-piperazine der allgemeinen Formel

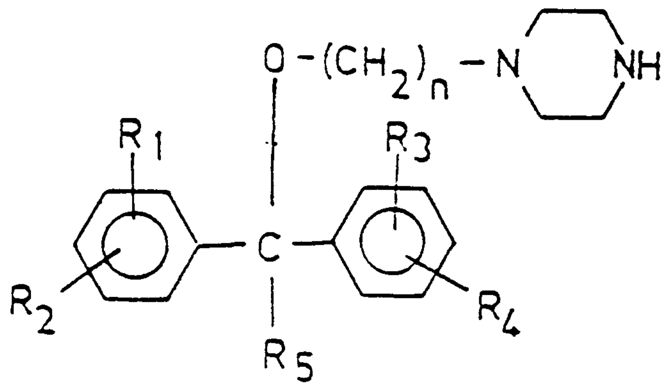

VIII,

worin

$R_1$ , $R_2$ , $R_3$ , $R_4$ und $R_5$ sowie n die oben angegebenen Bedeutungen haben,

mit Alkenyl- oder Alkinyl- beziehungsweise Alkyl- beziehungsweise Arylsulfonyloxy-alkenen, -alkinen beziehungsweise -alkencarbonsäuren beziehungsweise -alkincarbonsäuren der allgemeinen Formel

$Z-R_6$ IX,

worin

$R_6$ und Z die oben angegebenen Bedeutungen haben,

umgesetzt werden oder,

e) gegebenenfalls substituierte, 1-{ω-[bis-(Phenyl)-alkoxy]-alkanoyl}-4-{allenyl}-piperazine oder -4-{alkinyl}-piperazine der allgemeinen Formel

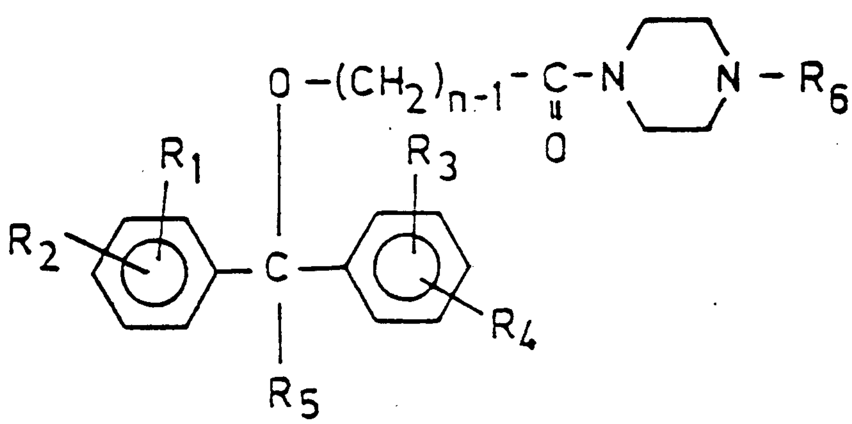

XI ,

worin

$R_1$ , $R_2$ , $R_3$ , $R_4$ und $R_5$ sowie n die oben angegebenen Bedeutungen haben und

$R_6$ für einen Alkenyl- oder Alkinylrest mit 3 bis 6 Kohlenstoffatomen steht,

reduziert werden oder

f) gegebenenfalls substituierte, 1-{ω-[bis-(Phenyl)-alkoxy]-alkyl}-4-{alkinyl}-piperazine der allgemeinen Formel I, bei welchen $R_1$ , $R_2$ , $R_3$ , $R_4$ und $R_5$ sowie n die oben angegegebenen Bedeutungen haben und $R_6$ für einen Alkinylrest steht, zu 1-{ω-[bis-(Phenyl)-alkoxy]-alkyl}-4-{alkenyl}-piperazinen der allgemeinen Formel I, bei welchen $R_6$ für einen Alkenylrest steht, reduziert werden oder,

g) durch 1 oder mehr Benzyloxyrest(e) substituierte 1-{ω-[bis-(Phenyl)-alkoxy]-alkyl} -4-{alkenyl}-piperazine oder -4-{alkinyl}-piperazine der allgemeinen Formel

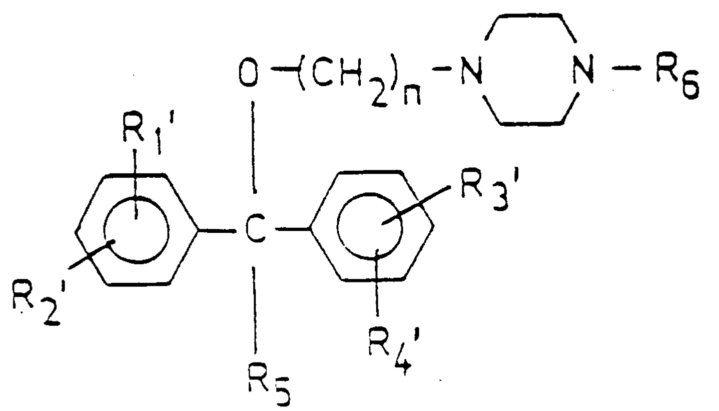

Ia ,

worin

| | |
|---|---|
| $R_5$ , $R_6$ und n | die oben angegebenen Bedeutungen haben und |
| mindestens 1 von $R_1'$ , $R_2'$ , $R_3'$ und $R_4'$ | für einen Benzyloxyrest steht und |
| die etwaigen anderen von $R_1'$ , $R_2'$ , $R_3'$ und $R_4'$ | die oben für $R_1$ , $R_2$ , $R_3$ und $R_4$ angegebenen Bedeutungen haben, |

zu den entsprechenden 1-{ω-[bis-(Phenyl)-alkoxy]-alkyl}-4-{alkenyl}-piperazinen, oder -4-{alkinyl}-piperazinen der allgemeinen Formel I, bei welchen mindestens 1 von $R_1$ , $R_2$ , $R_3$ und $R_4$ für eine Hydroxygruppe steht, reduziert werden

sowie in an sich bekannter Weise gegebenenfalls die nach einer der Varianten a) bis g) erhaltenen 1-{ω-[bis-(Phenyl)-alkoxy]-alkyl} -4-{alkenyl}-piperazine oder -4-{alkinyl}-piperazine der allgemeinen Formel I mit anorganischen oder organischen Säuren in Säureadditionssalze oder mit Quaternisierungsmitteln in quaternäre Salze überführt und/oder die erhaltenen Säureadditionssalze oder quaternären Salze der 1-{ω-[bis-(Phenyl)-alkoxy]-alkyl} -4-{alkenyl}-piperazine oder -4-{alkinyl}-piperazine der allgemeinen Formel I in die entsprechenden freien 1-{ω-[bis-(Phenyl)-alkoxy]-alkyl} -4-{alkenyl}-piperazine oder -4-{alkinyl}-piperazine der allgemeinen Formel I oder in andere Säureadditionssalze und/oder quaternäre Salze überführt werden und/oder gegebenenfalls eine Spaltung der erhaltenen racemischen 1-{ω-[bis-(Phenyl)-alkoxy]-alkyl -4-{alkenyl}-piperazine oder -4-{alkinyl}-piperazine der allgemeinen Formel I beziehungsweise von deren Säureadditionsalzen beziehungsweise quaternären Salzen beziehungsweise eine Racemisierung der entsprechenden optisch aktiven Verbindungen in Stereoisomere vorgenommen wird.

Als Alkalimetall, für welches M beziehungsweise M' stehen kann, wird bevorzugt Lithium, Kalium oder Natrium verwendet.

Sofern im erfindungsgemäßen Verfahren Ausgangssubstanzen mit Alkylsulfonyloxygruppen eingesetzt werden, sind diese vorteilhaft solche mit 1 bis 4, insbesondere 1 oder 2, Kohlenstoffatom(en).

Sofern im erfindungsgemäßen Verfahren Ausgangssubstanzen mit Arylsulfonyloxygruppen eingesetzt werden, sind diese vorteilhaft, gegebenenfalls durch 1 oder mehr Methylrest(e) substituierte, Phenylsulfonyloxygruppen.

Vorzugsweise wird im erfindungsgemäßen Verfahren in für die Reaktionsteilnehmer inerten Lösungsmitteln gearbeitet.

Im Sinne der Verfahrensvariante a) werden Verbindungen der allgemeinen Formel (II), in denen Y für die Gruppe -OM steht, in einem wasserfreien, inerten organischen Lösungsmittel mit Piperazinderivaten der allgemeinen Formel (III), in denen X für Alkylsulfonyloxy-, Arylsulfonyloxygruppe oder Halogen steht, umgesetzt. Besonders geeignete Verbindungen der allgemeinen Formel (III) sind die entsprechenden Mesylate und Tosylate, besonders bevorzugt jedoch die Chloride und Bromide. Die Umsetzung wird zweckmäßig unter Inertgasatmosphäre vorgenommen, zum Beispiel unter Stickstoff oder Argon. Als Lösungsmittel kommen zum Beispiel aliphatische oder alicyclische Äther, zum Beispiel Di-n-butyläther, Tetrahydrofuran, Dioxan, aliphatische oder aromatische Kohlenwasserstoffe, zum Beispiel n-Hexan, Ligroin,

Benzol, Toluol, Xylol, oder Dimethylsulfoxyd, Hexamethylphosphoramid oder Gemische der aufgeführten Lösungsmittel in Frage.

Wenn Y für Hydroxylgruppe steht und als Verbindung der allgemeinen Formel (III) eine der oben genannten Verbindungen verwendet wird, so ist es zweckmäßig, zum Binden der Säure ein organisches oder anorganisches Säurebindemittel zuzusetzen. Als Säurebindemittel ist auch ein Überschuß der Verbindung der allgemeinen Formel (III) geeignet. Die Reaktion kann in einem inerten organischen Lösungsmittel, jedoch auch ohne Lösungsmittel vorgenommen werden.

Wenn sowohl Y wie auch X für Hydroxylgruppe stehen, wird die Kondensation bevorzugt in Gegenwart der bei der Ätherbildung üblichen anorganischen oder organischen Säuren beziehungsweise sauren Salze vorgenommen. Es kann unter atmosphärischem und unter vermindertem Druck gearbeitet werden, und des entstehende Wasser wird azeotrop abdestilliert. Als Lösungsmittel kommen aliphatische oder aromatische Kohlenwasserstoffe, zum Beispiel n-Heptan, Toluol, Xylol, aliphatische oder alicyclische Äther, wie Di-n-butyläther und Dioxan, in Frage.

Gemäß dem erfindungsgemäßen Verfahren b) werden die Benzhydrylhalogenide, vorzugsweise -chloride oder -bromide, der allgemeinen Formel (IV) mit den Piperazinderivaten der allgemeinen Formel (V) umgesetzt. Die Reaktion wird unter den gleichen Bedingungen vorgenommen wie das Verfahren a). Zur Aufarbeitung kann das Reaktionsgemisch zum Beispiel auf Wasser gegossen und das Produkt mit einem Lösungsmittel extrahiert werden. Die organische Phase wird mit Wasser halogenfrei gewascher, getrocknet und dann eingedampft. Das Rohprodukt kann durch Chromatographie und/oder Kristallisation gereinigt werden.

Im Sinne des erfindungsgemäßen Verfahrens c) werden Verbindungen der allgemeinen Formel (VI), vorzugsweise die entsprechenden Mesylate, Tosylate,Bromide oder Chloride, mit den, 1-(Alkenyl)- oder 1-(Alkinyl)-piperazinen der allgemeinen Formel (VII) umgesetzt. Die Umsetzung wird vorzugsweise in einem organischen Lösungsmittel und in Gegenwart einer zum Binden der entstehenden Säure geeigneten Base vorgenommen. Als Lösungsmittel kommen Kohlenwasserstoffe, zum Beispiel Ligroin, Benzol, Toluol, Xylol, oder halogenierte Kohlenwasserstoffe, zum Beispiel Chloroform, ferner Äther, wie Dioxan, oder Alkohole, wie Äthanol, Ester, zum Beispiel Äthylacetat, Säureamide wie Dimethylformamid, Ketone, zum Beispiel Aceton oder Methylisobutylketon, oder Gemische der aufgeführten Lösungsmittel in Frage. Bei dieser Variante c) kann auch 1 Mol der Verbindung der allgemeinen Formel VI, bei welcher $R_1$ , $R_2$ , $R_3$ und/oder $R_4$ für [ein] Halogenatom(e) steht beziehungsweise stehen, mit 2 bis 5 Mol der Verbindung der allgemeinen Formel VII zu einer Verbindung der allgemeinen Formel I, bei welcher $R_1$ , $R_2$ , $R_3$ und/oder $R_4$ für einen 1-(Prop-2'-enyl)-piperazin-4-yl-rest steht, umgesetzt werden. Als Säurebindemittel werden anorganische oder organische Basen, zum Beispiel Alkalimetallcarbonate und -hydroxyde, ferner Triäthylamin und Pyridin eingesetzt. Als Säurebindemittel kann auch der Überschuß des Piperazinderivates der allgemeinen Formel (VII) dienen. Wird als Säurebindemittel eine tertiäre Base oder der Überschuß des Piperazinderivates verwendet, so ist ein zusätzliches Lösungsmittel nicht erforderlich. Die Umsetzung wird bei Temperaturen zwischen 20 °C und dem Siedepunkt des Reaktionsgemisches vorgenommen. Gegebenenfalls kann ein Katalysator, zum Beispiel ein Alkalimetalljodid, eingesetzt werden.

Gemäß dem Verfahren d) werden 1-(Benzhydryloxyalkyl)-piperazine der allgemeinen Formel (VIII) mit Verbindungen der allgemeinen Formel (IX) umgesetzt. Die Reaktionsbedingungen sind vorzugsweise die gleichen wie die des Verfahrens c).

Vorzugsweise werden bei der Variante f) beziehungsweise g) die Reduktion zum Alkylrest als katalytische Hydrierung durchgeführt.

Im Sinne des Verfahrens f) werden diejenigen Verbindungen der allgemeinen Formel (I), in denen $R_6$ für Alkinylgruppe mit 3-6 Kohlenstoffatomen steht, durch partielle Reduktion der Dreifachbindung zu den entsprechenden Alkenylderivaten umgesetzt. Zur Herstellung der Alkenylderivate wird in Gegenwart eines zur partiellen Sättigung der Dreifachbindung geeigneten Katalysators, bis zur Aufnahme der moläquivalenten Menge Wasserstoff hydriert. Als Katalysator kann zum Beispiel mit Zinkacetat vergiftetes Raney-Nickel in Gegenwart von Piperidin oder Lindlar-Katalysator (Pd/CaCo/PbO) in Gegenwart von Chinolin verwendet werden. Als Hydrierkatalysator kommen zum Beispiel Ruthenium, Palladium, Platin, Nickel, Eisen, Kupfer, Kobalt, Chrom, Zink, Molybdän und Wolfram sowie deren Oxyde und Sulfide in Frage. Der zum Hydrieren verwendete Katalysator kann auch durch Fällung auf die Oberfläche eines geeigneten Trägers aufgebracht sein. Solche Träger sind zum Beispiel Kohle, Siliciumdioxyd, Aluminiumoxyd, die Carbonate und Sulfate der Alkali- und Erdalkalimetalle. Bevorzugt wird die Hydrierung in Gegenwart von Palladium, Platin oder Raney-Nickel in einem inerten Lösungsmittel vorgenommen. Als Lösungsmittel kommen zum Beispiel niedere aliphatische Alkohole, Äther, Ester, aliphatische, cycloaliphatische und aromatische Kohlenwasserstoffe oder Gemische der aufgeführten Lösungsmittel in Frage. Die Hydrierung wird bei atmosphärischem Druck oder Überdruck und bei zwischen 20 °C und dem Siedepunkt des Reaktionsgemisches liegenden Temperaturen

vorgenommen. Nach Aufnahme der berechneten Menge Wasserstoff wird der Katalysator abfiltriert, das Filtrat eingedampft und das Produkt durch Destillation und/oder Kristallisieren gereinigt. Vorzugsweise werden bei der Verfahrensvariante e) als Reduktionsmittel Aluminiumkomplexe verwendet. Dabei werden die Amide der allgemeinen Formel (XI) reduziert. Diese Reduktion wird zweckmäßig mit Lithiumaluminiumhydrid in einem inerten Lösungsmittel, zum Beispiel aliphatischen oder cycloaliphatischen Äthern, wie Diäthyläther, Tetrahydrofuran oder Gemischen dieser Lösungsmittel, unter einer inerten Gesatmosphäre, zum Beispiel unter Stickstoff oder Argon, vorgenommen, und anschließend wird der gebildete Komplex hydrolysiert. Bei dieser Reduktion können auch Substituenten der Phenylringe, wie Halogenatome, mit wegreduziert werden.

Die Reduktion der Variante g) wird zweckmäßig durch katalytische Hydrierung durchgeführt, wobei als Katalysator beispielsweise Palladium verwendet werden kann.

Das Überführen der erfindungsgemäßen 1-{ω[bis-(Phenyl)-alkoxy]-alkyl}-4-{alkenyl}-piperazine oder -4-{alkinyl}-piperazine der allgemeinen Formel I in Säureadditionssalze kann nach an sich bekannten Verfahrensweisen durchgeführt werden.

Beispielsweise wird zur Salzbildung die Verbindung der allgemeinen Formel (I) in einem inerten Lösungsmittel gelöst und zu der zum Beispiel äthanolischen Lösung der entsprechenden Säure gegeben. Das Salz wird vorzugsweise mit einem nicht wassermischbaren organischen Lösungsmittel, zum Beispiel Diäthyläther, ausgefällt.

Die Umsetzung zur Überführung der erfindungsgemäßen 1-{ω[bis-(Phenyl)-alkoxy]-alkyl}-4-{alkenyl}-piperazine oder -4-{alkinyl}-piperazine der allgemeinen Formel I in quaternäre Salze kannn in einem organischen Lösungsmittel, zweckmäßig zum Beispiel Aceton, Acetonitril, Äthanol oder deren Gemischen, bei zwischen der Raumtemperatur und dem Siedepunkt des Gemisches liegenden Temperaturen vorgenommen werden. Das gebildete quaternäre Salz kann zum Beispiel durch Filtrieren isoliert und erforderlichenfalls durch Kristallisieren gereinigt werden.

Die Ausgangsstoffe sind bekannt oder können nach literaturbekannnten Verfahren hergestellt werden. So können die Benzhydrole der allgemeinen Formel (II), in denen Y für Hydroxylgruppe oder eine Gruppe -OM mit M = MgHlg (worin Hlg Halogen bedeutet) steht, aus den entsprechenden Carbonylverbindungen mit Grignard-Reagenzien hergestellt werden [z.B.: M. S. Kharasch et al.: Grignard reactions of nonmetallic substances, Ed. Prentice-Hall Inc. 138-143 (1954)].

Die Alkohole der allgemeinen Formel (V) können zum Beispiel durch Alkylieren der monosubstituierten Piperazine der allgemeinen Formel (VII) hergestellt werden, wobei als Alkylierungsmittel Halogenalkanole der Formel $Hlg-(CH_2)_n-OH$ zum Einsatz kommen, in denen die Bedeutung von Hlg und n die gleiche wie oben ist.

Die Halogenderivate der allgemeinen Formel (III) können zum Beispiel durch Umsetzen der Alkohole der allgemeinen Formel (V) mit Thionylhalogeniden hergestellt werden [s. zum Beispiel O. Hromatka et al.: Monatshefte 87, 701-7 (1956)].

Die Alkoholate der allgemeinen Formeln (II) und (V), in denen Y für -OM' mit M' = Alkalimetall steht, können durch Umsetzen der entsprechenden Alkohole mit Alkalimetallen, Alkalimetallhydriden oder -amiden erhalten werden [z.B.: Houben Weyl: Methoden der Organischen Chemie VI/2 6-34 (1963)].

Die Verbindungen der allgemeinen Formel (IV) sind zum Beispiel nach der Methode von K. E. Hamlin et al. [J. Am. Chem. Soc. 71, 2731-4 (1949)] beziehungsweise nach R. Baltzly et al. [J. Org. Chem. 14, 775-82 (1949)] zugänglich.

Die Äther der allgemeinen Formel (VI) können zum Beispiel nach der Methode von Sugasawa [Org. Synthesis 33, 11 (1953)] hergestellt werden.

Zur Synthese der monosubstituierten Piperazinderivate der allgemeinen Formeln (VII) und (VIII) können die Methoden von Kiichi Fujii [J. Pharm. Soc. Japan 74, 1049-51 (1954)], H. W. Steward [J. Org. Chem. 13, 134-43 (1948)], T. Irikura [J. Med. Chem. 11(4) 801-4 (1968)] oder das in der belgischen Patentschrift Nr. 549 420 beschriebene Verfahren herangezogen werden.

Die Verbindungen der allgemeinen Formel (XI) können zum Beispiel durch Umsetzen der Alkoholate der allgemeinen Formel (II) mit der allgemeinen Formel (III) entsprechenden, jedoch in 1-Stellung statt der Gruppe $X-(CH_2)_n$-eine Gruppe $Hlg-(CH_2)_{n-1}-CO-$ enthaltenden Piperazinderivaten hergestellt werden; die Reaktionsbedingungen sind wie unter a) angegeben. Die genannten Piperazinderivate können zum Beispiel gemäß US-PS 3 041 341 hergestellt werden.

Gegenstand der Erfindung sind auch Arzneimittel, welche 1 oder mehr der erfindungsgemäßen Verbindungen als Wirkstoff(e), gegebenenfalls zusammen mit 1 oder mehr üblichen pharmazeutischen Träger- und/oder Hilfsstoff(en), enthalten.

Gegenstand der Erfindung ist ferner die Verwendung von 1-ω-[bis-(Phenyl)-alkoxy]-alkyl-4-alkyl und -4-{acyl}-piperazinen der allgemeinen Formel

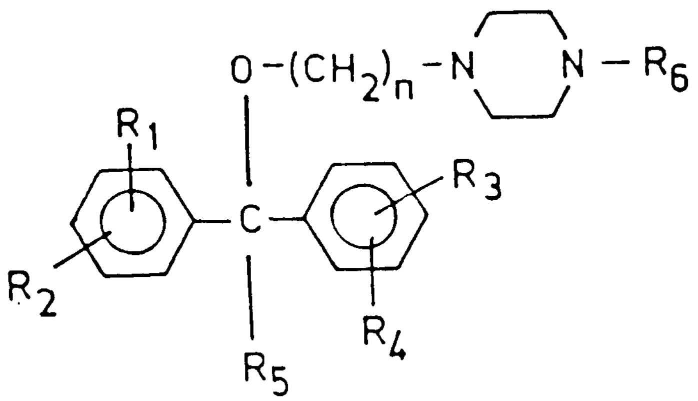

I ,

worin

| | |
|---|---|
| $R_1$ | für ein Wasserstoffatom oder ein Halogenatom in der o- oder p-Stellung steht, |
| $R_2$, $R_3$, $R_4$ und $R_5$ | Wasserstoff bedeuten und |
| $R_6$ | ein Alkylrest mit 3 bis 6 Kohlenstoffatomen oder ein Acylrest der allgemeinen Formel |

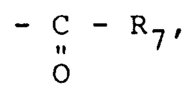

ist, in welchletzterer $R_7$ für einen Alkylrest mit 2 bis 5 Kohlenstoffatomen steht,

zur Herstellung von Arzneimitteln mit dopaminerger Wirkung auf das zentrale Nervensystem.

In "Neuropharmacology" 1985, 24(12), 1171-4 wird die Wechselwirkung zwischen von den erfindungsgemäßen Piperatzinderivaten verschiedenen Phenylalkyl- und Cinnamylpiperazinderivaten mit Dopamin-Rezeptoren beschrieben.

Die britische Patentschrift 837 986 beschreibt Piperazinderivate der oben angegebenen Formel (I), bei denen $R_1$ für ein Wasserstoffatom oder ein Halogenatom in der o- oder p-Stellung steht, $R_2$, $R_3$, $R_4$ und $R_5$ Wasserstoff bedeuten und $R_6$ Alkyl ist. Diese Verbindungen besitzen eine Antihistaminwirkung sowie eine Wirkung gegen Magengeschwüre.

Ferner beschreibt diese britische Patentschrift Verbindungen der obigen allgemeinen Formel (I), in denen $R_1$ für ein Halogenatom in der o-Stellung steht, $R_2$, $R_3$, $R_4$ und $R_5$ Wasserstoff bedeuten und $R_6$ ein Alkyl- oder Alkanoylrest ist. Diese Verbindungen besitzen eine Wirkung gegen Magengeschwüre und nur eine geringe Antihistaminwirkung.

Die Literaturstelle "Industrie Chimique Belge", Band T.XIX, Nr. 11, 1954, Seiten 1176 bis 1196 beschreibt Piperazinderivate, die den in der vorstehend erwähnten britischen Patentschrift 837 986 beschriebenen Derivaten entsprechen.

Die dopaminerge Aktivität der Verbindungen wurde in vitro und in vivo in Tierexperimenten nachgewiesen. Im Zuge der in vivo vorgenommenen Versuche wurde darüber hinaus geprüft, in welchem Maße die neuen Verbindungen die neurotoxische Wirkung des 1-Methyl-4-phenyl-1,2,3,6-tetrahydropyridins (MPTP) zu kompensieren vermögen. Von MPTP wurde 1979 bekannt, daß es an Menschen und Affen eine Degeneration des dopaminergen Systems verursacht [Davis, G. C. et al.: Psychiat. Res. 1, 249-254 (1979); Burns, R. S. et al.: Proc. Natl. Acad. Sci. (USA) 80, 4546-4550 (1983)], und später wurde die selektive, das dopaminerge System schädigende Wirkung der Verbindung auch an Mäusen nachgewiesen [z.B.: Hallman H. et al.: Eur. J. Pharmacol. 97, 133-136 (1984); Pileblad E. et al.: Neuropharmacol. 24, 689-694 (1985)]. Die mit MPTP an Versuchstieren ausgelöste selektive, das dopaminerge System schädigende Wirkung kann als den Degenerations- und Hypofunktionszuständen des menschlichen dopaminergen Systems analoger Prozeß betrachtet werden und ist deshalb ein geeignetes Modell für die Untersuchung von Verbindungen, die zur Therapie von mit einer pathologischen Funktion des dopaminergen Systems zusammenhängenden Krankheiten dienen können [Bradbury A.J. et al.: The Lancet, 1444-45 (1985); Przuntek, H. et al.: Life Sci. 37, 1195-1200 (1985)].

Zu den Untersuchungen wurden männliche CFY-Mäuse eines Gewichtes von 20-25 g verwendet. Die Verbindungen wurden in 1 %igem Tween® 80 homogenisiert und in einer Dosis von 0,1 mMol/kg auf die in der Tabelle angegebene Weise den Tieren eine Stunde vor Verabreichung des MPTP appliziert. Das MPTP

wurde in Form einer mit physiologischer Kochsalzlösung frisch bereiteten Lösung subcutan in einer Dosis von 70 mg/kg verabreicht. 72-96 Stunden nach der Applikation des MPTP wurden die Tiere durch Enthaupten getötet, das Gehirn wurde schnell entfernt und in eiskalter physiologischer Kochsalzlösung gekühlt. Das Striatum wurde entfernt und in Trockeneis eingefroren.

Die Gewebe wurden im gefrorenen Zustand gewogen und dann in 1 ml 0,4 n eiskalter Perchlorsäure, die 0,5 % $Na_2S_2O_5$, 0,25 % $Na_2$EDTA und 100 ng N-Methyldopamin enthält (internationaler Standard zur Bestimmung der Catecholamine), mit einem Ultra-Turrax®-Gerät homogenisiert. Anschließend wurde bei +4 °C mit 20 000 g 10 Minuten lang zentrifugiert, und vom Überstand wurden 0,8 ml abgenommen. Nach Zusatz von 20 mg aktivierten Aluminiumoxyds wurde der pH-Wert durch Zusatz von 0,8 ml 0,5 m Tris auf 8 gestellt, und die Röhrchen wurden 20 Minuten lang geschüttelt. Das Aluminiumoxyd wurde sich absetzen gelassen und nach Absaugen des Überstandes mit 3x5 ml destilliertem Wasser gewashen. Die am Aluminiumoxyd adsorbierten Catecholamine wurden mit 1 ml 0,05 n Perchlorsäure eluiert. In einem Teil des Eluats wurde das Dopamin mittels HPLC und elektrochemischer Anzeige bestimmt (Pumpe Labor MIM OE-320, Füllung der Säulen: 4x150 mm Nucleosil 5 C-18 analytisch sowie Vorsatzfüllung 4x20 mm Nucleosil 5 C-18 Vorsatz, mit glassy-carbon Arbeitselektroden und Ag/$AgCl_2$-Referenzelektroden ausgestatteter elektrochemischer Detektor, Eltron®-Potentiostat, Zweikanal-Recorder LKB 2110,
Oxydationspotential: 600 mV, mobile Phase: 0,1 m $NaH_2PO_4$, 1 mM $Na_2$EDTA, 1 mM Octansulfonsäure, enthaltend 8,5 % Acetonitril, Fließgeschwindigkeit: 1 ml/min).

Mit der angewendeten Methode kann der Dopaminspiegel im Striatum um 50-60 % vermindert werden. Die Kompensierung des durch MPTP induzierten Absinkens des Dopaminspiegels wurde wie folgt berechnet:

$$\text{Hemmung \%} = \frac{(\text{Behandl. Verbindung + MPTP}) - (\text{Behandl. MPTP})}{(\text{Kontrolle}) - (\text{Behandl. MPTP})} \cdot 100$$

Als Vergleichssubstanz wurde Trihexyphenidyl.HCl (3-[1'-piperidyl]-1-[phenyl]-1-[cyclohexyl]-propan-1-ol.HCl) in einer Dosis von 10 mg/kg (<0,1 mMol/kg) verwendet. Eine diesen Wert überschreitende Dosis war für die Tiere tödlich. Die Ergebnisse sind in der folgenden Tabelle zusammengefaßt, in der die hier aufgeführten Abkürzungen verwendet werden.

MPTP = 1-Methyl-4-phenyl-1,2,3,6-tetrahydropyridin.HCl
DA = Dopamin
n = Anzahl der Tiere
i.p. = intraperitoneal
p.o. = oral

Verbindungen:

1 1-{2'-[bis-⟨4''-(Fluor)-phenyl⟩-methoxy]-äthyl}-4-{prop-2'''-enyl}-piperazin,
2 1-{2'-[⟨4''-(Fluor)-phenyl⟩-⟨phenyl⟩-methoxy]-äthyl}-4-{prop-2'''-enyl}-piperazin,
3 1-{2'-[⟨4''-(Chlor)-phenyl⟩-⟨4'''-(fluor)-phenyl⟩-methoxy]-äthyl}-4-{prop-2''''-enyl}-piperazin,
4 1-{2'-[⟨4''-(Brom)-phenyl⟩-⟨4'''-(fluor)-phenyl⟩-methoxy]-äthyl}-4-{prop-2''''-enyl}-piperazin,
5 1-{2'-[bis-⟨4''-(Chlor)-phenyl⟩-methoxy]-äthyl}-4-{prop-2'''-enyl}-piperazin,
6 1-{2'-[⟨3'',4''-Di-(chlor)-phenyl⟩-⟨phenyl⟩-methoxy]-äthyl}-4-{prop-2'''-enyl}-piperazin,
7 1-{2'-[1''-bis-⟨4'''-(Fluor)-phenyl⟩-äthoxy]-äthyl}-4-{prop-2''''-enyl}-piperazin,
8 1-{2'-[bis-⟨4''-(Fluor)-phenyl⟩-methoxy]-äthyl}-4-{prop-2'''-inyl}-piperazin,
9 1-{2'-[bis-⟨4''-(Fluor)-phenyl⟩-methoxy]-äthyl}-4-{2'''-(methyl)-prop-2'''-enyl}-piperazin.

Aus der folgenden Tabelle ist ersichtlich, daß die neuen Verbindungen der allgemeinen Formel I bei ihrer Verabreichung vor der Behandlung mit MPTP oral und/oder intraperitoneal die neurotoxische Wirkung des MPTP ganz oder doch zum großen Teil kompensieren. Deshalb sind die erfindungsgemäßen Verbindungen ein wertvolles therapeutisches Mittel zur Behandlung aller Krankheitsbilder, die auf die Degeneration des dopaminergen Systems oder seine aus anderen Gründen eingetretene Hypofunktion zurückzuführen sind.

Tabelle

| Nr. der Verbindung | 0,1 mMol/kg Applikationsweise | | Hemmung der durch MPTP induzierten DA-Verringerung, % i.p. | p.o. | n |
|---|---|---|---|---|---|
| 1 | i.p. | p.o. | 98 | 100 | je 7 |
| 2 | | p.o. | | 100 | |
| 3 | i.p. | | 100 | | 7 |
| 4 | i.p. | | 100 | | 7 |
| 5 | i.p. | | 100 | | 7 |
| 6 | i.p. | p.o. | 100 | 98 | je 7 |
| 7 | i.p. | | 89 | | 7 |
| 8 | | p.o. | | 94 | 7 |
| 9 | | p.o. | | 95 | 7 |
| Trihexyphenidyl .HCl | i.p. | | 7 | | 7 |

Die erfindungsgemäßen Verbindungen können zu Arzneimittelpräparaten formuliert werden, die zur oralen, rektalen oder parenteralen Applikation geeignet sind. Zur oralen Verabreichung werden zum Beispiel Tabletten, Dragees, Kapseln hergestellt, wobei als Füllstoffe zum Beispiel Milchzucker oder Stärke und als Binde- und Granuliermittel zum Beispiel Gelatine, Carboxymethylcellulose-Na, Methylcellulose, Polyvinylpyrrolidon oder Stärkekleister verwendet werden. Als Sprengmittel kommen in erster Linie Kartoffelstärke oder mikrokristalline Cellulose in Frage, jedoch können auch Ultraamylopektin und Formaldehydkasein verwendet werden. Als Antihaft- und Gleitmittel kommen Talk, kolloidale Kieselsäure, Stearin, Calcium- und Magnesiumstearat zur Verwendung.

Die Tabletten können zum Beispiel durch nasses Granulieren und anschließendes Pressen hergestellt werden. Das Gemisch aus Wirk- und Trägerstoffen sowie gegebenenfalls ein Teil des Sprengmittels wird mit der wäßrigen, alkoholischen oder wäßrig-alkoholischen Lösung des Bindemittels in einer geeigneten Vorrichtung granuliert und das Granulat getrocknet. Der Rest des Sprengmittels sowie die Gleitmittel und übrigen Hilfsstoffe werden dem trockenen Granulat zugemischt, und die fertige Mischung wird zu Tabletten gepreßt. Zur Erleichterung der Dosierung können die Tabletten mit Teilungsmarken versehen sein. Die Tabletten können auch durch unmittelbares Verpressen des Wirk- und Hilfsstoffgemisches hergestellt werden. Gewünschtenfalls können die Tabletten mit einem Überzug versehen werden, der die üblichen Schutz-, Geschmacks- beziehungsweise Farbstoffe, zum Beispiel Zucker, Cellulosederivate (Methyl- oder Äthylcellulose, Carboxymethylcellulose-Na), Polyvinylpyrrolidon, Calciumphosphat, Calciumcarbonat, Lebensmittelfarbstoffe, Lebensmittelfarblacke, Aromastoffe, Eisenoxydpigmente enthält. Zur Herstellung von Kapseln wird das Gemisch aus Träger- und Wirkstoffen in Kapseln gefüllt.

Zur rektalen Anwendung werden Suppositorien hergestellt. Diese enthalten außer dem Wirkstoff eine Trägermasse, sog. Adeps pro suppositorio. Diese Masse besteht aus pflanzlichen Fetten, zum Beispiel gehärteten pflanzlichen Fetten, den Triglyceriden der Fettsäuren mit 12-18 Kohlenstoffatomen. Vorzugsweise wird die Masse der Markenbezeichnung Witepsol® verwendet. Der Wirkstoff wird in der geschmolzenen Trägermasse homogen verteilt, und aus der Masse werden Suppositorien gegossen.

Zur parenteralen Applikation werden Injektionslösungen hergestellt. Dazu werden die Wirkstoffe, gegebenenfalls zusammen mit Lösungsvermittlern wie Polyoxyäthylensorbitan-monolaurat, -monooleat oder -monostearat (Tween® 20, Tween® 60, Tween® 80) in destilliertem Wasser und/oder organischen Lösungsmitteln, zum Beispiel Glykoläthern, gelöst. Die Injektionslösungen können darüber hinaus noch unterschiedliche Hilfsstoffe, zum Beispiel Konservierungsmittel, wie Benzylalkohol, p-Oxybenzoesäuremethyl- oder -propylester, Benzalkoniumchlorid oder Phenylmercuriborat, ferner Antioxydantien, zum Beispiel Ascorbinsäure, Tocopherol, Natriumpyrosulfat und gegebenenfalls zum Binden von Metallspuren geeignete Komplexbildner wie Äthylendiamin-tetraacetat, zum Einstellen des pH-Wertes und zum Puffern dienende Stoffe, Lokalanaesthetika, zum Beispiel Lidocain, enthalten. Die Injektionslösungen werden vor dem Abfüllen in Ampullen filtriert, die gefüllten Ampullen werden sterilisiert.

Die tägliche Dosis hängt vom Zustand des Patienten und der Schwere des zu behandelnden Leidens ab und beträgt für Erwachsene im Falle oraler Verabreichung 5-200 mg.

Die Herstellung der erfindungsgemäßen Verbindungen wird durch die folgenden Beispiele näher veranschaulicht.

## Beispiel 1

1-{2'-[bis-⟨4''-(Fluor)-phenyl⟩-methoxy]-äthyl}-4-{prop-2'''-enyl}-piperazindihydrochlorid

2,4 g einer 50 %igen öligen Dispersion von Natriumhydrid und 11,0 g 4,4'-Difluorbenzhydrol werden in 60 ml wasserfreiem Toluol suspendiert. Die Suspension wird unter Rühren, in einer Argonatmosphäre 15 Minuten lang am Rückfluß gekocht und dann mit der Lösung von 9,4 g 1-(2-Chloräthyl)-4-(2-propenyl)-piperazin in 70 ml wasserfreiem Toluol versetzt. Das Gemisch wird weitere 2 Stunden lang am Rückfluß gekocht und nach dem Erkalten mit 40 ml Wasser versetzt. Die organische Phase wird abgetrennt, mit Wasser chloridfrei gewaschen und über Natriumsulfat getrocknet. Der nach Eindampfen unter vermindertem Druck zurückbleibende Rückstand wird mit einem Gemisch aus Benzol und Methanol als Elutionsmittel an einer Silikagelsäule gereinigt. Die entsprechenden Fraktionen werden eingedampft, der Rückstand wird in wasserfreiem Isopropanol gelöst und durch Zusatz ätherischer Salzsäure das Dihydrochlorid gebildet, das bei 189-191 °C schmilzt.

| Elementaranalyse für $C_{22}H_{26}F_2N_2O$ (Base) | | | | |
|---|---|---|---|---|
| berechnet, %: | C 70,94 | H 7,04 | F 10,20 | N 7,52; |
| gefunden, %: | C 70,77 | H 7,11 | F 10,40 | N 7,63. |

## Beispiel 2

1-{2'-[⟨4''-(Chlor)-phenyl⟩-⟨4'''-(fluor)-phenyl⟩-methoxy]-äthyl}-4-{prop-2''''-enyl}-piperazindihydrochlorid

Ein Gemisch aus 18,0 g 2-[(4-Chlorphenyl)-(4-fluorphenyl)-methoxy]-äthylchlorid, 6,3 g 1-Allylpiperazin, 8,3 g wasserfreiem, pulverförmigem Kaliumcarbonat, 0,83 g Kaliumjodid und 170 ml Methylisobutylketon wird unter Rühren 15 Stunden lang am Rückfluß gekocht. Nach dem Abkühlen wird das Gemisch unter vermindertem Druck eingedampft. Der Rückstand wird mit Wasser versetzt und mit Benzol extrahiert. Die Benzolphase wird mit Wasser gewaschen, über wasserfreiem Natriumsulfat getrocknet, zur Trockne eingedampft und der Rückstand in wasserfreien Äther aufgenommen. Aus der ätherischen Lösung wird durch Zusatz ätherischer Salzsäure das Dihydrochlorid ausgefällt. Schmelzpunkt: 199-200 °C.

| Elementaranalyse für $C_{22}H_{26}ClFN_2O$ (Base) | | | | | |
|---|---|---|---|---|---|
| berechnet, %: | C 67,94 | H 6,74 | Cl 9,12 | F 4,89 | N 7,20; |
| gefunden, %: | C 68,10 | H 6,53 | Cl 9,30 | F 5,10 | N 7,08. |

Auf die beschriebene Weise werden noch die folgenden Verbindungen hergestellt:

a) 1-{2'-[⟨3''-(Nitro)-4''-(1'''-/prop-2''''-enyl/-piperazin-4'''-yl)-phenyl⟩-⟨phenyl⟩-methoxy]-äthyl}-4-{prop-2'''''-enyl}-piperazintetramaleat, Schmelzpunkt: 109-112°C, aus 2-[(3-Nitro-4-chlorphenyl)-phenylmethoxy]-äthylchlorid und 1-(2-Propenyl)-piperazin;

| Elementaranalyse für $C_{29}H_{39}N_5O_3$ (Base) | | | |
|---|---|---|---|
| berechnet, %:<br>gefunden, %: | C 68,88<br>C 68,67 | H 7,77<br>H 7,84 | N 13,85;<br>N 13,97. |

b) 1-{2'-[⟨4''-(Chlor)-phenyl⟩-⟨4'''-(fluor)-phenyl⟩-methoxy]-äthyl}-4-{prop-2''''-inyl}-piperazindihydrochlorid, Schmelzpunkt: 186-188°C, aus 2-[(4-Chlorphenyl)-(4-fluorphenyl)-methoxy]-äthylchlorid und 1-(2-Propinyl)-piperazin;

| Elementaranalyse für $C_{22}H_{24}ClFN_2O$ (Base) | | | | | |
|---|---|---|---|---|---|
| berechnet, %:<br>gefunden, %: | C 68,29<br>C 68,47 | H 6,25<br>H 6,38 | Cl 9,16<br>Cl 9,00 | F 4,91<br>F 4,77 | N 7,24;<br>N 7,32. |

c) 1-{2'-[⟨3'',4''-Di-(chlor)-phenyl⟩-⟨phenyl⟩-methoxy]-äthyl}-4-{prop-2'''-inyl}-piperazindihydrochlorid, Schmelzpunkt: 201-203 °C, aus 2-[(3,4-Dichlorophenyl)-phenylmethoxy]-äthylbromid und 1-(2-Propinyl)-piperazin;

| Elementaranalyse für $C_{22}H_{24}Cl_2N_2O$ (Base) | | | | |
|---|---|---|---|---|
| berechnet, %:<br>gefunden, %: | C 65,51<br>C 65,60 | H 6,00<br>H 6,11 | Cl 17,58<br>Cl 17,33 | N 6,95;<br>N 7,13. |

d) 1-{2'-[⟨4''-(Brom)-phenyl⟩-⟨4'''-(fluor)-phenyl⟩-methoxy]-äthyl}-4-{prop-2''''-inyl}-piperazindihydrochlorid, Schmelzpunkt: 179-181°C, aus 2-[(4-Bromphenyl)-(4-(fluor-phenyl)-methoxy]-äthylbromid und 1-(2-Propinyl)-piperazin;

| Elementaranalyse für $C_{22}H_{24}BrFN_2O$ (Base) | | | | | |
|---|---|---|---|---|---|
| berechnet, %:<br>gefunden, %: | C 61,26<br>C 61,17 | H 5,61<br>H 5,83 | Br 18,53<br>Br 18,44 | F 4,40<br>F 4,61 | N 6,49;<br>N 6,40. |

e) 1-{2'-[⟨4''-(Fluor)-phenyl⟩-⟨phenyl⟩-methoxy]-äthyl}-4-{prop-2'''-inyl}-piperazindihydrochlorid, Schmelzpunkt: 186-187 °C, aus 2-[(4-Fluorphenyl)-phenylmethoxy]-äthylchlorid und 1-(2-Propinyl)-piperazin;

| Elementaranalyse für $C_{22}H_{25}FN_2O$ (Base) | | | | |
|---|---|---|---|---|
| berechnet, %:<br>gefunden, %: | C 74,97<br>C 75,21 | H 7,15<br>H 7,34 | F 5,39<br>F 5,55 | N 7,95;<br>N 7,79. |

f) 1-{2'-[bis-⟨4''-(Chlor)-phenyl⟩-methoxy]-äthyl}-4-{prop-2'''-inyl}-piperazindihydrochlorid, Schmelzpunkt: 194-196 °C, aus 2-[bis(4-Chlorphenyl)-methoxy]-äthyltosylat und 1-(2-Propinyl)-piperazin;

| Elementaranalyse für $C_{22}H_{24}Cl_2N_2O$ (Base) | | | | |
|---|---|---|---|---|
| berechnet, %: | C 65,51 | H 6,00 | Cl 17,58 | N 6,95; |
| gefunden, %: | C 65,69 | H 5,87 | Cl 17,45 | N 6,84. |

Beispiel 3

1-{2'-[1''-⟨2''',5'''-Di-(methyl)-phenyl⟩-1''-⟨phenyl⟩-propoxy]-äthyl}-4-(prop-2''''-enyl)-piperazindihydrofumarat

Zu einem Gemisch aus 10,6 g 1-{2-[1-(2,5-Dimethylphenyl)-1-phenylpropoxy]-äthyl}-piperazin und 4,6 g wasserfreiem, pulverförmigem Kaliumcarbonat in 90 ml wasserfreiem Benzol werden unter schwachem Sieden 3,6 g Allylbromid in 10 ml Benzol tropfenweise zugegeben. Das Reaktionsgemisch wird noch eine Stunde lang gekocht und dann mit Wasser versetzt. Die organische Phase wird abgetrennt, mit Wasser neutral gewaschen, über Natriumsulfat getrocknet und unter vermindertem Druck eingedampft. Der Rückstand wird in wasserfreiem Äthanol gelöst und mit äthanolischer Fumarsäurelösung behandelt. Das ausfallende Salz wird abfiltriert und aus Methanol umkristallisiert. Es schmilzt bei 202-204 °C.

| Elementaranalyse für $C_{26}H_{36}N_2O$ (Base) | | | |
|---|---|---|---|
| berechnet, %: | C 79,54 | H 9,24 | N 7,14; |
| gefunden, %: | C 79,31 | H 9,28 | N 7,30. |

Beispiel 4

1-{2'-[⟨Di-(phenyl)⟩-methoxy]-äthyl}-4-{1''-(oxo)-prop-2''-enyl}-piperazinhydrochlorid

Zu der Lösung von 7,2 g 1-{2-[⟨Di-(phenyl)⟩-methoxy]-äthyl}-piperazin und 4,2 g Triäthylamin in 80 ml 1,2-Dichloräthan wird bei 10°C tropfenweise die Lösung von 2,2 g Acrylsäurechlorid in 10 ml 1,2-Dichloräthan gegeben. Das Reaktionsgemisch wird noch 30 Minuten lang gerührt und dann mit Wasser versetzt. Die organische Phase wird abgetrennt, mit Wasser gewaschen, über wasserfreiem Magnesiumsulfat getrocknet und unter vermindertem Druck eingedampft. Der Rückstand wird in Äthanol gelöst und mit ätherischer Salzsäure zum Hydrochlorid umgesetzt. Das Salz wird abgetrennt und aus Äthanol umkristallisiert. Schmp.: 189-190°C.

Durch entsprechende Wahl der Ausgangsstoffe wird in analoger Weise die folgende Verbindung hergestellt:
1-{2'-[⟨Di-(phenyl)⟩-methoxy]-äthyl}-4-{1''-(oxo)-prop-2''-enyl}-piperazinhydrochlorid.

1-{2'-[⟨Di-(phenyl)⟩-methoxy]-äthyl}-4-{prop-2''-enyl}-piperazindihydrochlorid (physikalische Daten s. Beispiel 9).

Beispiel 5

1-{2'-[1''-bis-⟨4'''-(Fluor)-phenyl⟩-äthoxy]-äthyl}-4-{prop-2''''-enyl}-piperazindihydrochlorid

Zu 20 ml einer 1,5 molaren, mit Tetrahydrofuran bereiteten Lösung von 4-Fluorphenyl-magnesiumbromid wird in einer Stickstoffatmosphäre unter Rühren die Lösung von 4,6 g 4'-Fluoracetophenon in 10 ml Tetrahydrofuran tropfenweise zugesetzt. Das Reaktionsgemisch wird eine Stunde lang gekocht und dann auf Raumtemperatur abgekühlt. Zu diesem die Ausgangssubstanz [1-bis-⟨4'-(Fluor)-phenyl⟩-äthoxy]-magnesiumbromid enthaltenden Reaktionsgemisch wird die Lösung von 5,7 g 1-(2-Chloräthyl)-4-(2-propenyl)-piperazin in 45 ml wasserfreiem Xylol zugegeben und weitere 3 Stunden lang gekocht. Nach dem Abkühlen wird das Gemisch auf Wasser gegossen, die organische Phase wird abgetrennt. Die wäßrige Phase wird mit 2x25 ml Xylol extrahiert. Dann wird die saure wäßrige Lösung mit Ammoniak alkalisch gemacht und mit Äther ausgezogen. Die ätherische Phase wird über wasserfreiem Magnesiumsulfat getrocknet und dann eingedampft. Der Rückstand wird unter Verwendung eines im Verhältnis 98:2 bereiteten Gemisches aus Chloroform und Methanol an einer Silikagelsäule gereinigt. Die entsprechenden Fraktionen werden unter vermindertem Druck eingedampft. Das Hydrochlorid wird mit ätherischer Salzsäure ausgefällt, abfiltriert und

getrocknet. Schmp.: 187-189,5 °C.

| Elementaranalyse für $C_{23}H_{28}F_2N_2O$ (Base) | | | | |
|---|---|---|---|---|
| berechnet, %:<br>gefunden, %: | C 71,47<br>C 71,60 | H 7,30<br>H 7,37 | F 9,83<br>F 9,75 | N 7,25;<br>N 7,30. |

Beispiel 6

1-{2'-[bis-‹4''-(Chlor)-phenyl›-methoxy]-äthyl}-4-{prop-2'''-enyl}-piperazindihydrochlorid

8,5 g 1-(2-Propenyl)-4-(2-hydroxyäthyl)-piperazin werden in 30 ml Methanol mit 2,7 g Natriummethylat umgesetzt, dann wird das Methanol aus dem Reaktionsgemisch abdestilliert. Der Rückstand wird mit 100 ml Toluol versetzt und durch azeotrope Destillation von Methanolspuren befreit (etwa 20 ml Destillat werden genommen). Dann wird dem Gemisch unter schwachem Sieden am Rückfluß tropfenweise die Lösung von 14,1 g 4,4'-Dichlorbenzhydrylchlorid in 30 ml wasserfreiem Toluol zugesetzt. Das Gemisch wird weitere 4 Stunden lang gekocht und nach dem Abkühlen mit Wasser versetzt. Die organische Phase wird abgetrennt, mit Wasser gewaschen, über wasserfreiem Magnesiumsulfat getrocknet und unter vermindertem Druck eingedampft. Der Rückstand wird in wasserfreiem Äther gelöst und die Lösung mit ätherischer Salzsäure versetzt. Das ausgefallene Dihydrochlorid wird aus einem Metharol-Äther-Gemisch umkristallisiert und schmilzt dann bei 218,5-220 °C.

| Elementaranalyse für $C_{22}H_{26}Cl_2N_2O$ (Base) | | | | |
|---|---|---|---|---|
| berechnet, %:<br>gefunden, %: | C 65,18<br>C 65,31 | H 6,47<br>H 6,52 | Cl 17,49<br>Cl 17,29 | N 6,91;<br>N 6,83. |

Beispiel 7

Ein entsprechendes Benzhydrylchlorid-Derivat und ein 4-Alkenyl-(ω-hydroxyalkyl)-piperazin-Derivat werden unter Stickstoffatmosphäre auf 160-170°C erwärmt und für 30 Minuten bei dieser Temperatur belassen. Dann wird das Reaktionsgemisch auf 90-100°C gekühlt, mit Wasser versetzt und nach dem Abkühlen auf Raumtemperatur mit Benzol extrahiert. Die Benzolphase wird mit Wasser neutral gewaschen, über Magnesiumsulfat getrocknet und dann eingedampft. Der Rückstand wird in wasserfreiem Äthanol gelöst und aus der Lösung mit ätherischer Salzsäure das Dihydrochlorid gefällt. Es wird mit Äther gewaschen und dann getrocknet.

Durch entsprechende Wahl der Ausgangsstoffe werden auf die in den Beispielen 6 und 7 beschriebene Weise die folgenden Verbindungen hergestellt ($R_5$ = H, n = 2, $R_6$ = = 2-Propenyl):

| | $R_1$ | $R_2$ | $R_3$ | $R_4$ | Salz | Schmp. °C |
|---|---|---|---|---|---|---|
| b) | H | 2-Cl | H | 4-F | 2HCl | 207-209 |
| c) | 4-$CF_3$ | H | H | 4-F | 2HCl | 198-200 |
| d) | 4-Cl | 3-Cl | H | H | 2HCl | 200-202 |
| e) | 4-F | H | 3-$C_2H_5O$ | 4-OH | 2HCl | 156-158 |
| f) | H | 3-$CF_3$ | H | 4-F | 2HCl | 192-193 |
| g) | H | 2-$CH_3$ | H | 4-F | 2HCl | 199-201 |
| h) | 4-F | H | 4-Benzyloxy | H | 2-Maleat | 172-174 |
| i) | 4-Br | H | H | 4-F | 2HCl | 197-198,5 |
| j) | H | H | 3-$CF_3$ | H | 2-Maleat | 171-172 |

Beispiel 8

a) 1-{2'-[bis-‹4''-(Fluor)-phenyl›-methoxy]-äthyl}-4-{2'''-(methyl)-prop-2'''-enyl}-piperazindihydrochlorid

Ein Gemisch aus 6,6 g 1-{2-[bis(4-Fluorphenyl)-methoxy]-äthyl}-piperazin, 3,0 g pulverisiertem wasserfreiem Kaliumcarbonat, 2,0 g 3-Chlor-2-methylprop-1-en und 70 ml wasserfreiem Aceton wird unter Rühren 6 Stunden lang am Rückfluß gekocht. Dann wird das Aceton unter vermindertem Druck abdestilliert, der Rückstand mit Wasser versetzt und mit Benzol extrahiert. Die Benzolphase wird mit Wasser gewaschen, über wasserfreiem Kaliumcarbonat getrocknet und im Vakuum eingedampft. Der Rückstand wird an einer Silikagelsäule unter Verwendung von Chloroform als Elutionsmittel gereinigt. Die entsprechenden Fraktionen werden vereinigt und eingedampft. Das Dihydrochlorid wird mit ätherischer Salzsäure ausgefällt, es schmilzt bei 197-199 °C.

Aus der Hydrochlorid wird die Base mit verdünntem wäßrigem Ammoniak freigesetzt.

| Elementaranalyse für $C_{23}H_{28}F_2N_2O$ (Base) | | | | |
|---|---|---|---|---|
| berechnet, %:<br>gefunden, %: | C 71,48<br>C 71,60 | H 7,30<br>H 7,35 | F 9,83<br>F 9,74 | N 7,25;<br>N 7,33. |

Beispiel 9

1-{2'-[⟨Di-(phenyl)⟩-methoxy]-äthyl}-4-{prop-2''-enyl}-piperazindihydrochlorid

16,7 g 1-[2-(Diphenylmethoxy)-äthyl]-4-(2-propinyl)-piperazin und 0,4 g Zinkacetat-dihydrat werden in einem Gemisch aus 170 ml Methanol und 10 ml Piperidin gelöst und in Gegenwart von 3,0 g Raneynickel unter atmosphärischem Druck hydriert. Nach Aufnahme der berechneten Menge Wasserstoff wird der Katalysator abfiltriert und das Lösungsmittel unter vermindertem Druck abdestilliert. Der Rückstand wild in Benzol gelöst, die Lösung mit Wasser gewaschen, über Magnesiumsulfat getrocknet und dann eingedampft. Der Rückstand wird an einer Silikagelsäule mit einem im Verhältnis 98:2 bereiteten Gemisch aus Chloroform und Methanol chromatographisch gereinigt. Die entsprechenden Fraktionen werden mit ätherischer Salzsäure versetzt, worauf das bei 204-206 °C schmelzende Dihydrochlorid ausfällt.

| Elementaranalyse für $C_{22}H_{28}N_2O$ | | | |
|---|---|---|---|
| berechnet, %:<br>gefunden, %: | C 78,53<br>C 78,67 | H 8,39<br>H 8,29 | N 8,33;<br>N 8,35. |

Beispiel 10

1-{2'-[bis-⟨4''-(Fluor)-phenyl⟩-methoxy]-äthyl}-4-{prop-2'''-inyl}-piperazindihydrochlorid

Ein Gemisch aus 33,2 g 1-{2-[bis(4-Fluorphenyl)-methoxy]-äthyl}-piperazin, 15,5 g wasserfreiem, pulverisiertem Kaliumcarbonat, 13,1 g einer mit Toluol bereiteten 80 %igen Propargylbromidlösung und 330 ml wasserfreiem Aceton wird eine Stunde lang bei Raumtemperatur gerührt und dann unter vermindertem Druck eingedampft. Der Rückstand wird mit Wasser versetzt und mit Benzol extrahiert. Die Benzollösung wird mit Wasser gewaschen, über wasserfreiem Natriumsulfat getrocknet und dann eingedampft. Das Produkt wird an einer Silikagelsäule mit Chloroform gereinigt. Die Base wird in Äther gelöst und mit ätherischer Salzsäure zum Dihydrochlorid umgesetzt. Schmp.: 187-188 °C.

| Elementaranalyse für $C_{22}H_{24}F_2N_2O$ (Base) | | | | |
|---|---|---|---|---|
| berechnet, %:<br>gefunden, %: | C 71,33<br>C 71,41 | H 6,53<br>H 6,47 | F 10,26<br>F 10,12 | N 7,56;<br>N 7,58. |

Durch entsprechende Wahl der Ausgangsstoffe können auf analoge Weise folgende Verbindungen hergestellt werden:

a) 1-{2'-[⟨Di-(phenyl)⟩-methoxy]-äthyl}-4-{prop-2''-inyl}-piperazindihydrochlorid, Schmp.: 205-206°C,

| Elementaranalyse für $C_{22}H_{26}N_2O$ (Base) | | | |
|---|---|---|---|
| berechnet, %:<br>gefunden, %: | C 79,00<br>C 79,20 | H 7,84<br>H 7,78 | N 8,38;<br>N 8,19. |

b) 1-{2'-[⟨4''-(Trifluormethyl)-phenyl⟩-⟨4'''-(fluor)-phenyl⟩-methoxy]-äthyl}-4-{prop-2''''-inyl}-piperazin (Öl),

Elementaranalyse für $C_{23}H_{24}F_4N_2O$ (Base)

| | | | | |
|---|---|---|---|---|
| berechnet, %:<br>gefunden, %: | C 65,70<br>C 65,86 | H 5,75<br>H 5,76 | F 18,08<br>F 18,20 | N 6,66;<br>N 6,51. |

Beispiel 11

1-{2'-[⟨4''-(Fluor)-phenyl⟩-⟨phenyl⟩-methoxy]-äthyl}-4-{prop-2'''-enyl}-piperazindihydrochlorid

Zu der Lösung von 7,9 g 4-Fluorbenzhydrol in 150 ml Toluol werden 12,0 g p-Toluolsulfonsäuremonohydrat und die Lösung von 5,1 g 1-(2-Hydroxyäthyl)-4-(2-propenyl)-piperazin in 45 ml Dimethylformamid gegeben. Das Reaktionsgemisch wird gekocht und dabei das gebildete Wasser azeotrop abdestilliert. Nach Ablauf der Reaktion (die dünnschichtchromatographisch verfolgt wird) wird das Reaktionsgemisch abgekühlt und dann mit Wasser extrahiert. Die wäßrige Phase wird mit konzentriertem wäßrigem Ammoniak alkalisch gemacht und dann mit Benzol extrahiert. Die Benzolphase wird mit Wasser gewaschen, über wasserfreiem Magnesiumsulfat getrocknet, durch eine Schicht aus 50 g Aluminiumoxyd filtriert und dann eingedampft. Der Rückstand wird in wasserfreiem Äther gelöst und die Lösung mit ätherischer Salzsäure auf pH 2,5-3 angesäuert. Das Dihydrochlorid wird abfiltriert und aus Methanol umkristallisiert. Es schmilzt bei 191-192 °C.

| Elementaranalyse für $C_{22}H_{27}FN_2O$ | | | | |
|---|---|---|---|---|
| berechnet, %:<br>gefunden, %: | C 74,55<br>C 74,41 | H 7,68<br>H 7,73 | F 5,36<br>F 5,41 | N 7,90;<br>N 7,88. |

Beispiel 12

Aus den erfindungsgemäßen Verbindungen können zum Beispiel die folgenden Arzneimittelpräparate hergestellt werden:

Tabletten

50 g Wirkstoff, 92 g Lactose, 40 g Kartoffelstärke, 4 g Polyvinylpyrrolidon, 6 g Talk, 1 g Magnesiumstearat, 1 g kolloidales Siliziumdioxyd (Aerosil) und 6 g Ultraamylopektin werden miteinander vermischt, naß granuliert und dann zu Tabletten des Gewichtes von 200 mg verpreßt.
Wirkstoff: 1-{2'-[bis-⟨4''-(Fluor)-phenyl⟩-methoxy]-äthyl}-4-{prop-2'''-enyl}-piperazindihydrochlorid

Dragees

Die auf die beschriebene Weise hergestellten Tabletten werden mit einer aus Zucker und Talk bestehenden Schicht überzogen und dann mit einem Gemisch aus Bienenwachs uns Carnaubawachs poliert.
Gewicht eines Dragees: 250 mg

**Patentansprüche**

**Patentansprüche für folgende Vertragsstaaten : BE, CH, DE, FR, GB, IT, LI, LU, NL, SE**

1. Gegebenenfalls substituierte, 1-{ω-[bis-(Phenyl)-alkoxy]-alkyl}-4-{alkenyl}-piperazine und -4-{alkinyl}-piperazine der allgemeinen Formel I

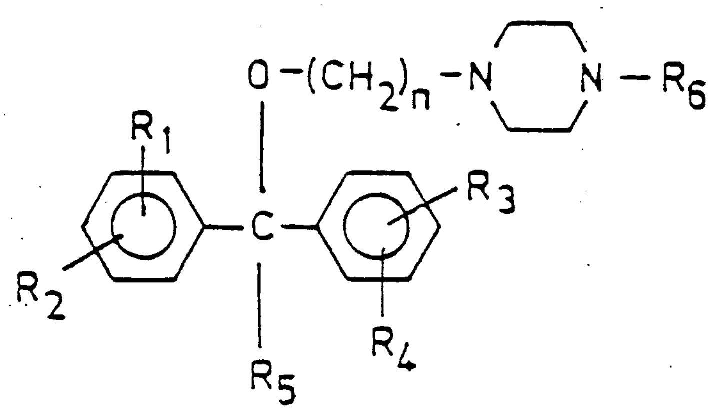

I.,

worin

| | |
|---|---|
| $R_1$, $R_2$, $R_3$ und $R_4$ | unabhängig voneinander für Wasserstoffatome, Halogenatome, Trihalogenmethylreste, $C_1$-$C_4$-Alkylreste, $C_1$-$C_4$-Alkoxyreste, Nitrogruppen, Hydroxygruppen, 1-(Prop-2'-enyl)-piperazin-4-ylreste oder Phenyl-$C_1$-$C_4$-alkoxyreste stehen, |
| $R_5$ | ein Wasserstoffatom oder einen Alkylrest mit 1 bis 4 Kohlenstoffatom(en) bedeutet, |
| $R_6$ | ein Alkenyl- oder Alkinylrest mit 3 bis 6 Kohlenstoffatomen ist und |
| n | gleich 2 oder 3 ist; |

sowie ihre Säureadditionssalze, quaternären Salze und optisch aktiven Isomere und Solvate.

2. Piperazine nach Anspruch 1, dadurch gekennzeichnet, daß das (die) Halogenatom(e) und/oder die Halogenatome des (der) Trihalogenmethylreste(s) von $R_1$, $R_2$, $R_3$ und/oder $R_4$ Fluor, Chlor und/oder Brom ist (sind).

3. Piperazine nach Anspruch 1 oder 2, dadurch gekennzeichnet, daß der (die) $C_1$-$C_4$-Alkylrest(e), $C_1$-$C_4$-Alkoxyrest(e) und/oder der Alkylteil des (der) Phenyl-$C_1$-$C_4$-Alkoxyreste(s) von $R_1$, $R_2$, $R_3$ und/oder $R_4$ -[ein] $C_1$- oder $C_2$-Rest(e) ist (sind).

4. Piperazine nach Anspruch 1 bis 3, dadurch gekennzeichnet, daß $R_5$ ein Alkylrest mit 1 bis 3 Kohlenstoffatomen ist.

5. Piperazine nach Anspruch 1 bis 4, dadurch gekennzeichnet, daß $R_6$ ein Alkenyl- oder Alkinylrest mit 3 oder 4 Kohlenstoffatomen ist.

6. Piperazine nach Anspruch 1 bis 5, dadurch gekennzeichnet, daß der Phenyl-$C_1$-$C_4$-alkoxyrest ein Benzyloxyrest ist.

7. 1-{2'-[bis-⟨4''-(Fluor)-phenyl⟩-methoxy]-äthyl}-4-{prop-2'''-enyl}-piperazin,

   1-{2'-[⟨4''-(Fluor)-phenyl⟩-⟨phenyl⟩-methoxy]-äthyl}-4-{prop-2'''-enyl}-piperazin,

   1-{2'-[⟨4''-(Chlor)-phenyl⟩-⟨4'''-(fluor)-phenyl⟩-methoxy]-äthyl}-4-{prop-2''''-enyl}-piperazin,

   1-{2'-[⟨4''-(Brom)-phenyl⟩-⟨4'''-(fluor)-phenyl⟩-methoxy]-äthyl}-4-{prop-2''''-enyl}-piperazin,

   1-{2'-[bis-⟨4''-(Chlor)-phenyl⟩-methoxy]-äthyl}-4-{prop-2'''-enyl}-piperazin,

1-{2'-[⟨3'',4''-Di-(chlor)-phenyl⟩-⟨phenyl⟩-methoxy]-äthyl}-4-{prop-2'''-enyl}-piperazin,

1-{2'-[1''-bis-⟨4'''-(Fluor)-phenyl⟩-äthoxy]-äthyl}-4-{prop-2''''-enyl}-piperazin,

1-{2'-[bis-⟨4''-(Fluor)-phenyl⟩-methoxy]-äthyl}-4-{prop-2'''-inyl}-piperazin,

1-{2'-[bis-⟨4''-(Fluor)-phenyl⟩-methoxy]-äthyl}-4-{2'''-(methyl)-prop-2'''-enyl}-piperazin,

1-{2'-[⟨3''-(Nitro)-4''-(1'''-[prop-2''''-enyl]-piperazin-4'''-yl)-phenyl⟩-⟨phenyl⟩-methoxy]-äthyl}-4-{prop-2'''''-enyl}-piperazin,

1-{2'-[1''-⟨2''',5'''-Di-(methyl)-phenyl⟩-1''-⟨phenyl⟩-propoxy]-äthyl}-4-{prop-2''''-enyl}-piperazin,

1-{2'-[⟨Di-(phenyl)⟩-methoxy]-äthyl}-4-{prop-2''-enyl}-piperazin,

1-{2'-[⟨2''-(Chlor)-phenyl⟩-⟨4'''-(fluor)-phenyl⟩-methoxy]-äthyl}-4-{prop-2''''-enyl}-piperazin,

1-{2'-[⟨4''-(Trifluormethyl)-phenyl⟩-⟨4'''-(fluor)-phenyl⟩-methoxy]-äthyl}-4-{prop-2''''-enyl}-piperazin,

1-{2'-[⟨2''-(Methyl)-phenyl⟩-⟨4'''-(fluor)-phenyl⟩-methoxy]-äthyl}-4-{prop-2''''-enyl}-piperazin,

1-{2'-[⟨3''-(Äthoxy)-4''-(hydroxy)-phenyl⟩-⟨4'''-(fluor)-phenyl⟩-methoxy]-äthyl}-4-{prop-2''''-enyl}-piperazin,

1-{2'-[⟨3''-(Trifluormethyl)-phenyl⟩-⟨4'''-(fluor)-phenyl⟩-methoxy]-äthyl}-4-{prop-2''''-enyl}-piperazin,

1-{2'-[⟨3''-(Trifluormethyl)-phenyl⟩-⟨phenyl⟩-methoxy]-äthyl}-4-{prop-2'''-enyl}-piperazin,

1-{2'-[⟨4''-(Benzyloxy)-phenyl⟩-⟨4'''-(fluor)-phenyl⟩-methoxy]-äthyl}-4-{prop-2''''-enyl}-piperazin,

1-{2'-[⟨4''-(Chlor)-phenyl⟩-⟨4'''-(fluor)-phenyl⟩-methoxy]-äthyl}-4-{prop-2''''-inyl}-piperazin,

1-{2'-[⟨4''-(Fluor)-phenyl⟩-⟨phenyl⟩-methoxy]-äthyl}-4-{prop-2'''-inyl}-piperazin,

1-{2'-[⟨3'',4''-Di-(chlor)-phenyl⟩-⟨phenyl⟩-methoxy]-äthyl}-4-{prop-2'''-inyl}-piperazin,

1-{2'-[⟨4''-(Brom)-phenyl⟩-⟨4'''-(fluor)-phenyl⟩-methoxy]-äthyl}-4-{prop-2''''-inyl}-piperazin,

1-{2'-[⟨4''-(Trifluormethyl)-phenyl⟩-⟨4'''-(fluor)-phenyl⟩-methoxy]-äthyl}-4-{prop-2''''-inyl}-piperazin,

1-{2'-[bis-⟨4''-(Chlor)-phenyl⟩-methoxy]-äthyl}-4-{prop-2'''-inyl}-piperazin, und

1-{2'-[bis-⟨4''-(Chlor)-phenyl⟩-methoxy]-äthyl}-4-{prop-2'''-enyl}-piperazin.

**8.** Verfahren zur Herstellung von Piperazin-Verbindungen nach Anspruch 1 bis 7, dadurch gekennzeichnet, daß man,
a) gegebenenfalls substituierte, Benzhydrole oder ihre Alkoholate der allgemeinen Formel

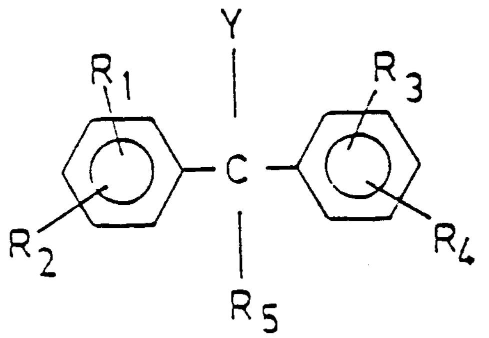

II ,

worin

| | |
|---|---|
| $R_1$ , $R_2$ , $R_3$ , $R_4$ und $R_5$ | die in den Ansprüchen 1 bis 6 angegebenen Bedeutungen haben und |
| Y | für eine Hydroxygruppe oder einen Rest der allgemeinen Formel<br><br>-OM,<br><br>in welchletzterer |
| M | ein Alkalimetallatom oder einen Rest der Formel<br><br>- MgHlg,<br><br>in welchletzterer |
| Hlg | ein Halogenatom ist,<br><br>bedeutet,<br><br>steht, |

mit einer Piperazinverbindung der allgemeinen Formel

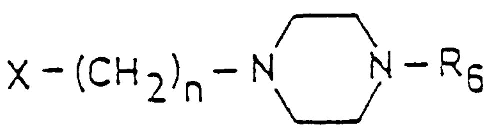

III,

worin

| | |
|---|---|
| $R_6$ und n | die in den Ansprüchen 1 oder 5 angegebenen Bedeutungen haben und |
| X | für ein Halogenatom, eine Hydroxygruppe, einen Alkylsulfonyloxyrest oder einen Arylsulfonyloxyrest steht,<br>mit der Einschränkung, daß |
| X | von einer Hydroxygruppe verschieden ist, |
| wenn Y | einen Rest der allgemeinen Formel -OM bedeutet, |

umsetzt, oder,

b) gegebenenfalls substituierte, Benzhydrylhalogenide der allgemeinen Formel

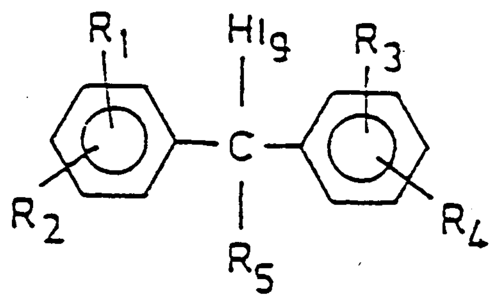

IV ,

worin

$R_1$ , $R_2$ , $R_3$ , $R_4$ und $R_5$ die in den Ansprüchen 1 bis 6 angegebenen Bedeutungen haben und

Hlg die obige Bedeutung hat,

mit einer Piperazinverbindung der allgemeinen Formel oder einem Alkoholat einer solchen

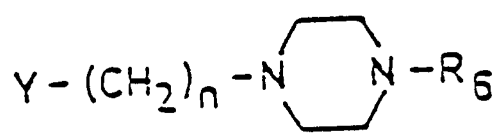

V ,

worin

$R_6$ und n die in den Absprüchen 1 oder 5 angegebenen Bedeutungen haben und

Y für eine Hydroxygruppe oder einen Metalloxyrest der allgemeinen Formel

-OM' ,

in welchletzterer

M' ein Alkalimetallatom bedeutet,

steht,

umsetzt oder;

c) gegebenenfalls substituierte, Diphenyl-Verbindungen der allgemeinen Formel VI

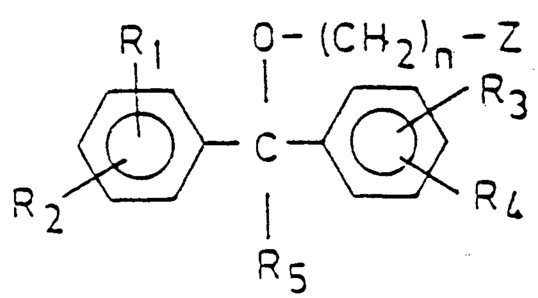

VI ,

worin

$R_1$ , $R_2$ , $R_3$ , $R_4$ und $R_5$ sowie n die in den Ansprüchen 1 bis 6 angegebenen Bedeutungen haben und

Z für ein Halogenatom oder einen Alkyl- oder Arylsulfonyloxyrest steht,

mit 1-(Alkenyl)- oder 1-(Alkinyl)-P iperazinen der allgemeinen Formel

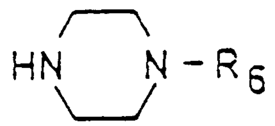 (VII)

worin

$R_6$ die in den Ansprüchen 1 oder 5 angegebenen Bedeutungen hat,

umsetzt oder,

d) gegebenenfalls substituierte, 1-{ω-[bis-(Phenyl)-alkoxy]-alk-ω-yl}-piperazine der allgemeinen Formel

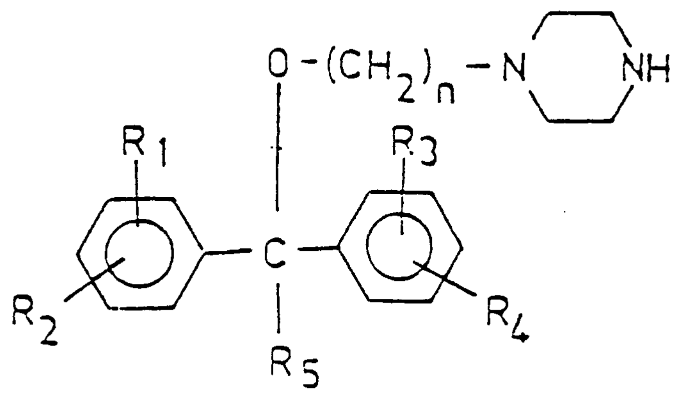 VIII,

worin

$R_1$, $R_2$, $R_3$, $R_4$ und $R_5$ sowie n die in den Ansprüchen 1 bis 6 angegebenen Bedeutungen haben,

mit einer Verbindung der allgemeinen Formel

$Z - R_6$ IX

worin

$R_6$ die in den Ansprüchen 1 oder 5 angegebenen Bedeutungen hat und

Z die oben angegebene Bedeutung hat,

umsetzt oder,

e) gegebenenfalls substituierte 1-{ω[bis-(Phenyl)-alkoxy]-alkanoyl}-4-{alkenyl}-piperazine oder -4-{alkinyl}-piperazine der allgemeinen Formel

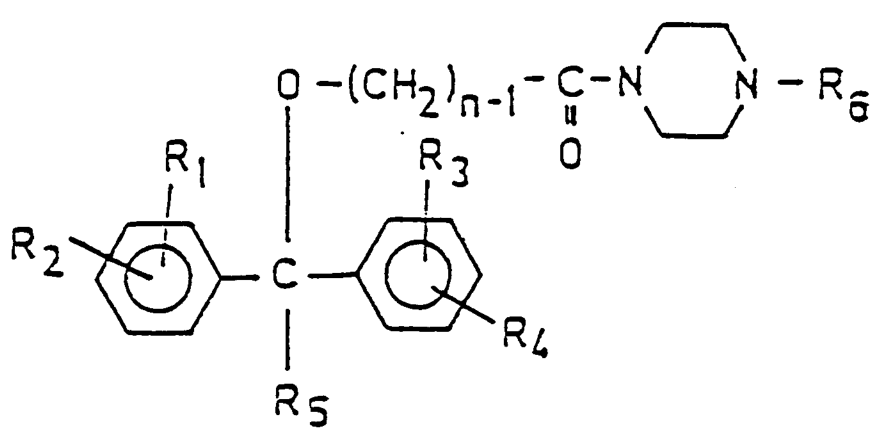 XI ,

worin

$R_1$, $R_2$, $R_3$, $R_4$ und $R_5$ sowie n die in den Ansprüchen 1 bis 6 angegebenen Bedeutungen haben und

$R_6$ für einen Alkenyl- oder Alkinylrest mit 3 bis 6 Kohlenstoffatomen steht,

reduziert

sowie in an sich bekannter Weise gegebenenfalls die nach einer der Varianten a) bis e) erhaltenen 1-{ω-[bis-(Phenyl)-alkoxy]-alkyl}-4-{alkenyl}-piperazine oder -4-{alkinyl}-piperazine der allgemeinen Formel I mit anorganischen oder organischen Säuren in Säureadditionssalze oder mit Quaternisierungsmitteln in quaternäre Salze überführt und/oder die erhaltenen Säureadditionssalze oder quaternären Salze der 1-{ω-[bis-(Phenyl)-alkoxy]-alkyl}--4-{alkenyl}-piperazine oder -4-{alkinyl}-piperazine der allgemeinen Formel I in die entsprechenden freien 1-{ω-[bis-(Phenyl)-alkoxy]-alkyl}-4-{alkenyl}-piperazin oder -4-{alkinyl}-piperazinbasen der allgemeinen Formel I oder in andere Säureadditionssalze und/oder quaternäre Salze überführt und/oder gegebenenfalls eine Spaltung der erhaltenen racemischen Piperazine der allgemeinen Formel I oder von deren Säureadditionssalzen oder quaternären Salzen vornimmt oder eine Racemisierung der entsprechenden optisch aktiven Verbindungen in Stereoisomere durchführt.

**9.** Verfahren nach Anspruch 8, dadurch gekennzeichnet, daß man gegebenenfalls substituierte, 1-{ω-[bis-(Phenyl)-alkoxy]-alkyl}-4-{alkinyl}-piperazine der allgemeinen Formel I, bei welchen $R_1$ , $R_2$ , $R_3$ , $R_4$ und $R_5$ sowie n die in den Ansprüchen 1 bis 6 angegebenen Bedeutungen haben und $R_6$ für einen Alkinylrest steht, zu 1-{ω-[bis-(Phenyl)-alkoxy]-alkyl}-4-{alkenyl}-piperazinen der allgemeinen Formel I, bei welchen $R_6$ für einen Alkenylrest steht, reduziert.

**10.** Verfahren nach Anspruch 8, dadurch gekennzeichnet, daß man durch 1 oder mehr Benzyloxyrest(e) substituierte 1-{ω-[bis-(Phenyl)-alkoxy]-alkyl} -4-{alkenyl}-piperazine oder -4-{alkinyl}-piperazine der allgemeinen Formel

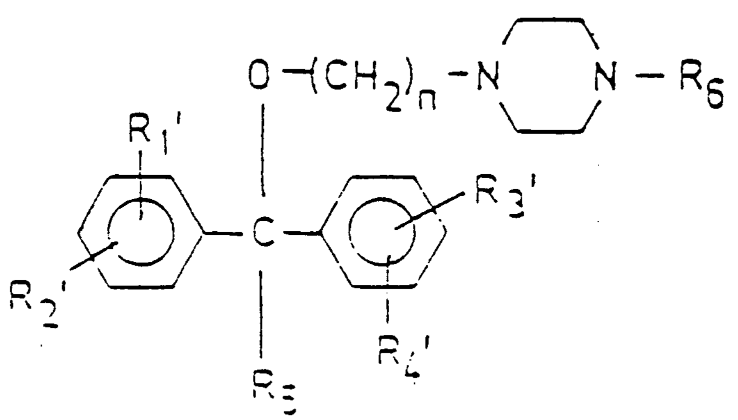

Ia ,

worin

$R_5$ , $R_6$ und n die in den Ansprüchen 1 bis 6 angegebenen Bedeutungen haben und

bei mindestens 1 von $R_1'$ , $R_2'$ , $R_3'$ und $R_4'$ der Phenyl-$C_1$-$C_4$-Alkoxyrest ein Benzyloxyrest ist und

die etwaigen anderen von $R_1'$ , $R_2'$ , $R_3'$ und $R_4'$ die in den Ansprüchen 1 bis 6 definierte Bedeutungen haben,

zu solchen Piperazinen der allgemeinen Formel I reduziert, bei welchen mindestens 1 von $R_1$ , $R_2$ , $R_3$ und $R_4$ für eine Hydroxygruppe steht.

**11.** Verfahren nach Anspruch 8, dadurch gekennzeichnet, daß man als Reduktionsmittel Aluminiumkomplexe verwendet.

**12.** Verfahren nach einem der Ansprüche 8 bis 11, dadurch gekennzeichnet, daß man in für die Reaktionsteilnehmer inerten Lösungsmitteln arbeitet.

**13.** Arzneimittel, gekennzeichnet durch einen Gehalt an einer oder mehreren Verbindung(en) nach Anspruch 1 bis 7 als Wirkstoff(en), gegebenenfalls zusammen mit einem oder mehreren üblichen

pharmazeutischen Träger- und/oder Hilfsstoff(en).

**14.** Verwendung von 1-{ω-[bis-(Phenyl)-alkoxy]-alkyl}-4-{alkyl}- und 4-{acyl}-piperazinen der allgemeinen Formel

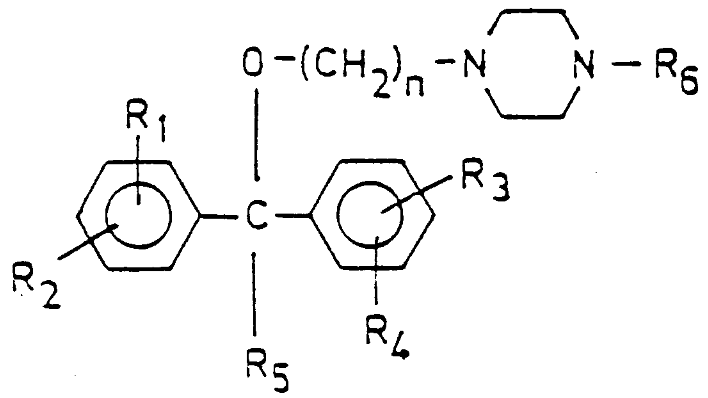

I ,

worin

| | |
|---|---|
| $R_1$ | für ein Wasserstoffatom oder ein Halogenatom in der o- oder p-Stellung steht, |
| $R_2$, $R_3$, $R_4$ und $R_5$ | Wasserstoff bedeuten und |
| $R_6$ | ein Alkylrest mit 3 bis 6 Kohlenstoffatomen oder ein Acylrest der allgemeinen Formel 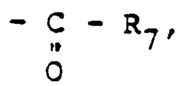 ist, in welchletzterer $R_7$ für einen Alkylrest mit 2 bis 5 Kohlenstoffatomen steht, und |
| n | 2 oder 3 ist |

zur Herstellung von Arzneimitteln mit dopaminerger Wirkung auf das zentrale Nervensystem.

**Patentansprüche für folgenden Vertragsstaat : AT**

**1.** Verfahren zur Herstellung von gegebenenfalls substituierten,1-{ω-[bis-(Phenyl)-alkoxy]-alkyl}-4-{alkenyl}-piperazinen und -4-{alkinyl}-piperazinen der allgemeinen Formel I

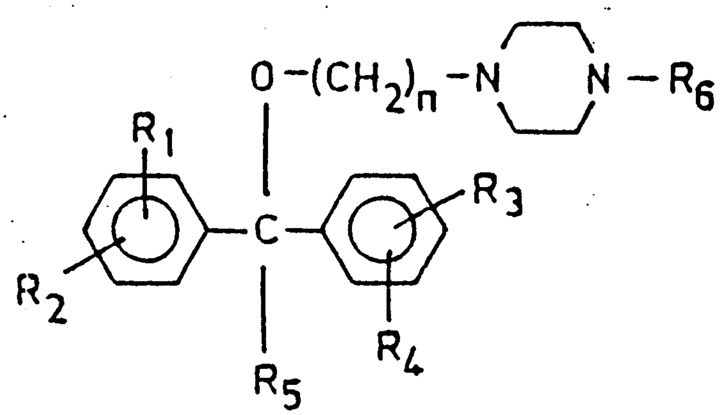

I ,

worin

| | |
|---|---|
| $R_1$, $R_2$, $R_3$ und $R_4$ | unabhängig voneinander für Wasserstoffatome, Halogenatome, Trihalogenmethylreste, $C_1$-$C_4$-Alkylreste, $C_1$-$C_4$-Alkoxyreste, Nitrogruppen, Hydroxygrup- |

pen, 1-(Prop-2'-enyl)-piperazin-4-ylreste oder Phenyl-$C_1$-$C_4$-alkoxyreste stehen,

$R_5$ ein Wasserstoffatom oder einen Alkylrest mit 1 bis 4 Kohlenstoffatom(en) bedeutet,

$R_6$ ein Alkenyl- oder Alkinylrest mit 3 bis 6 Kohlenstoffatomen ist und

n gleich 2 oder 3 ist;

sowie ihre Säureadditionssalze, quaternären Salze und optisch aktiven Isomere und Solvate, dadurch gekennzeichnet, daß man,

a) gegebenenfalls substituierte, Benzhydrole oder ihre Alkoholate der allgemeinen Formel

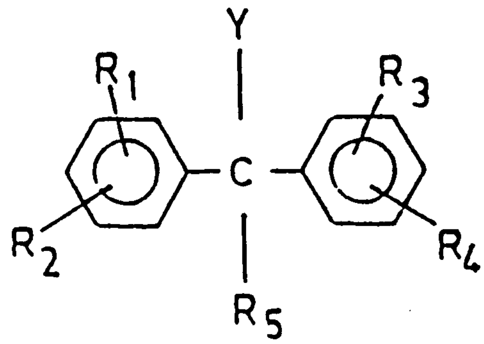

II ,

worin

$R_1$ , $R_2$ , $R_3$ , $R_4$ und $R_5$ die in den Ansprüchen 1 bis 6 angegebenen Bedeutungen haben und

Y für eine Hydroxygruppe oder einen Rest der allgemeinen Formel

-OM,

in welchletzterer

M ein Alkalimetallatom oder einen Rest der Formel

- MgHlg,

in welchletzterer

Hlg ein Halogenatom ist,

bedeutet,

steht,

mit einer Piperazinverbindung der allgemeinen Formel

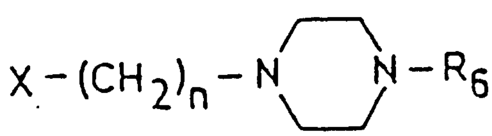

III,

worin

$R_6$ und n die in den Ansprüchen 1 oder 5 angegebenen Bedeutungen haben und

X für ein Halogenatom, eine Hydroxygruppe, einen Alkylsulfonyloxyrest oder einen Arylsulfonyloxyrest steht,

mit der Einschränkung, daß

X von einer Hydroxygruppe verschieden ist,

wenn Y einen Rest der allgemeinen Formel -OM bedeutet,

umsetzt, oder,

b) gegebenenfalls substituierte, Benzhydrylhalogenide der allgemeinen Formel

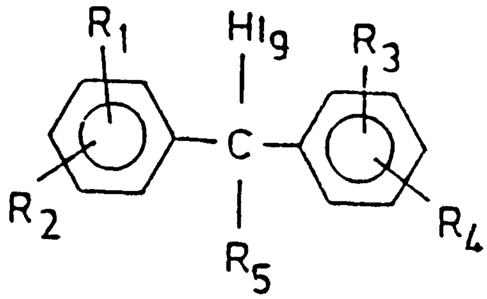

IV ,

worin

$R_1$ , $R_2$ , $R_3$ , $R_4$ und $R_5$ die in den Ansprüchen 1 bis 6 angegebenen Bedeutungen haben und

Hlg die obige Bedeutung hat,

mit einer Piperazinverbindung der allgemeinen Formel oder einen Alkohol einer solchen

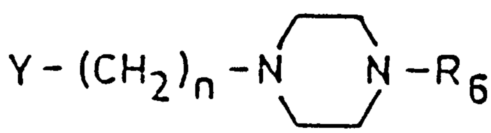

V ,

worin

$R_6$ und n die in den Ansprüchen 1 oder 5 angegebenen Bedeutungen haben und

Y für eine Hydroxygruppe oder einen Metalloxyrest der allgemeinen Formel

-OM' ,

in welchletzterer

M' ein Alkalimetallatom bedeutet,

steht,

umsetzt oder;

c) gegebenenfalls substituierte, Diphenyl-Verbindungen der allgemeinen Formel VI

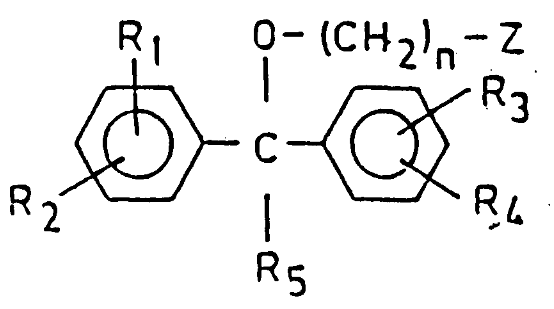

VI ,

worin

$R_1$ , $R_2$ , $R_3$ , $R_4$ und $R_5$ sowie n die in den Ansprüchen 1 bis 6 angegebenen Bedeutungen haben und

Z für ein Halogenatom oder einen Alkyl- oder Arylsulfonyloxyrest steht,

mit 1-(Alkenyl)- oder 1-(Alkinyl)-P iperazinen der allgemeinen Formel

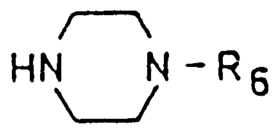

(VII)

worin

$R_6$ die in den Ansprüchen 1 oder 5 angegebenen Bedeutungen hat,

umsetzt oder,

d) gegebenenfalls substituierte, 1-{ω-[bis=(Phenyl)-alkoxy]-alk-ω-yl}-piperazine der allgemeinen Formel

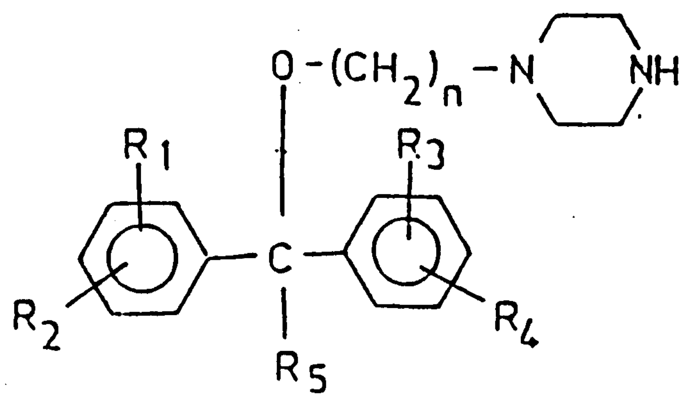

VIII,

worin

$R_1$, $R_2$, $R_3$, $R_4$ und $R_5$ sowie n die in den Ansprüchen 1 bis 6 angegebenen Bedeutungen haben,

mit einer Verbindung der allgemeinen Formel

$Z-R_6$ IX

worin

$R_6$ die in den Ansprüchen 1 oder 5 angegebenen Bedeutungen hat und

Z die oben angegebene Bedeutung hat,

umsetzt oder,

e) gegebenenfalls substituierte. 1-{ω-[bis-(Phenyl)-alkoxy]-alkanoyl}-4-{alkenyl}-piperazine oder -4-{alkinyl}-piperazine der allgemeinen Formel

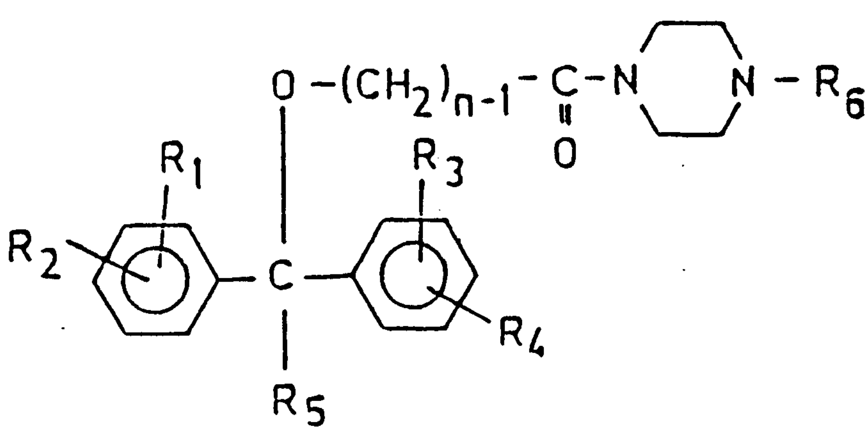

XI,

worin

| | |
|---|---|
| $R_1$ , $R_2$ , $R_3$ , $R_4$ und $R_5$ sowie n | die in den Ansprüchen 1 bis 6 angegebenen Bedeutungen haben und |
| $R_6$ | für einen Alkenyl- oder Alkinylrest mit 3 bis 6 Kohlenstoffatomen steht, |

reduziert

sowie in an sich bekannter Weise gegenenenfalls die nach einer der Varianten a) bis e) erhaltenen 1-{ω-[bis-(Phenyl)-alkoxy]-alkyl}-4-{alkenyl}-piperazine oder -4-{alkinyl}-piperazine der allgemeinen Formel I mit anorganischen oder organischen Säuren in Säurenadditionssalze oder mit Quaternisierungsmitteln in quaternäre Salze überführt und/oder die erhaltenen Säureadditionssalze oder quaternären Salze der 1-{ω-[bis-(Phenyl)-alkoxy]-alkyl} -4-{alkenyl}-piperazine oder -4-{alkinyl}-piperazine der allgemeinen Formel I in die entsprechenden freien 1-{ω-[bis-(Phenyl)-alkoxy]-alkyl}-4-{alkenyl}-piperazin oder -4-{alkinyl}-piperazinbasen der allgemeinen Formel I oder in andere Säureadditionssalze und/oder quaternäre Salze überführt und/oder gegebenenfalls eine Spaltung der erhaltenen racemischen Piperazine der allgemeinen Formel I oder von deren Säureadditionssalzen oder quaternären Salzen vornimmt oder eine Racemisierung der entsprechenden optisch aktiven Verbindungen in Stereoisomere durchführt.

**2.** Verfahren nach Anspruch 1, dadurch gekennzeichnet daß man als gegebenenfalls substituierte, Benzhydrole oder ihre Alkoholate der allgemeinen Formel

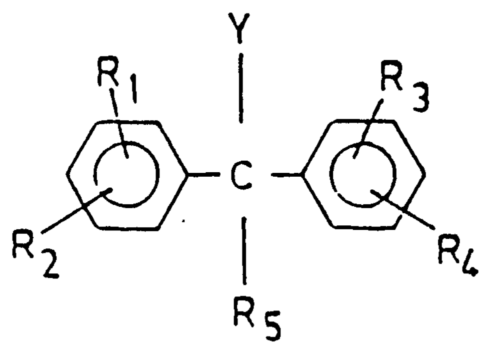

II ,

oder, als gegebenenfalls substituierte, Benzhydrylhalogenide der allgemeinen Formel

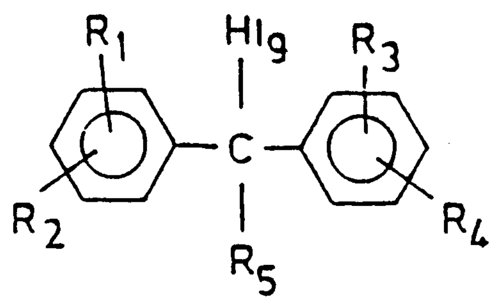

IV ,

oder, als gegebenenfalls substituierte, Diphenyl-Verbindungen der allgemeinen Formel VI

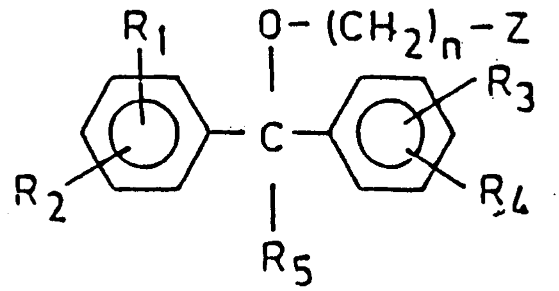

VI ,

oder als gegebenenfalls substituierte, 1-{ω-[bis-(Phenyl)-alkoxy]-alk-ω-yl} -piperazine der allgemeinen Formel

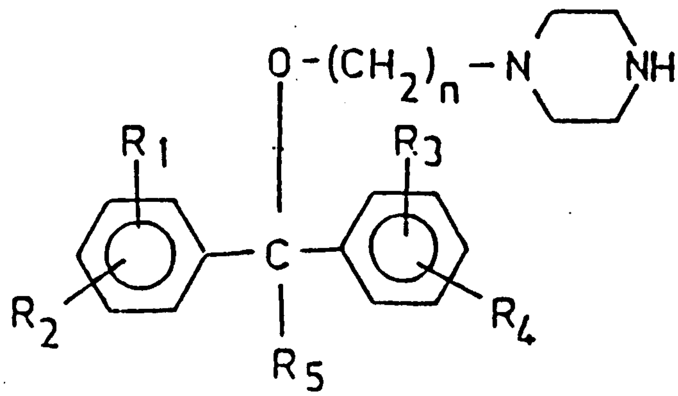

VIII,

oder als gegebenenfalls substituierte, 1-{ω-[bis-(Phenyl)-alkoxy]-alkanoyl}-4-{alkenyl}-piperazine oder -4-{alkinyl}-piperazine der allgemeinen Formel

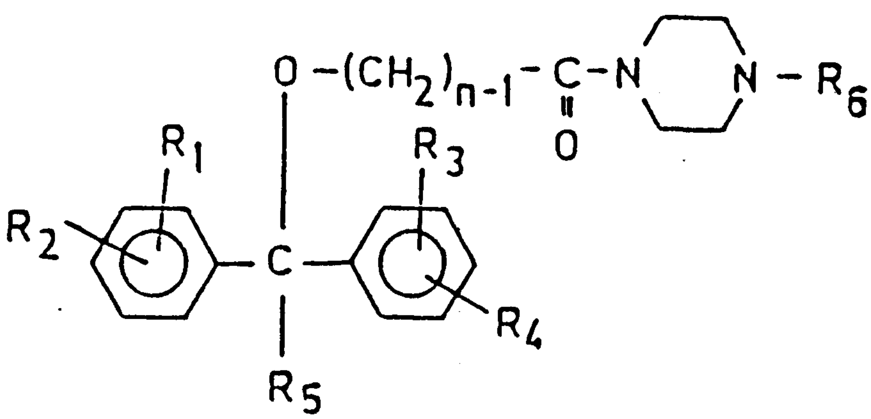

XI ,

solche verwendet, bei welchen das (die) Halogenatom(e) und/oder die Halogenatome des (der) Trihalogenmethylrest(s) von $R_1$, $R_2$, $R_3$ und/oder $R_4$ Fluor, Chlor und/oder Brom ist (sind).

**3.** Verfahren nach Anspruch 1 oder 2, dadurch gekennzeichnet, daß man als gegebenenfalls substituierte, Benzhydrole oder ihre Alkoholate der allgemeinen Formel

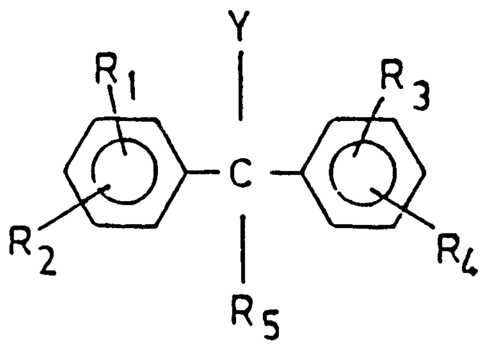

II ,

oder, als gegebenenfalls substituierte, Benzhydrylhalogenide der allgemeinen Formel

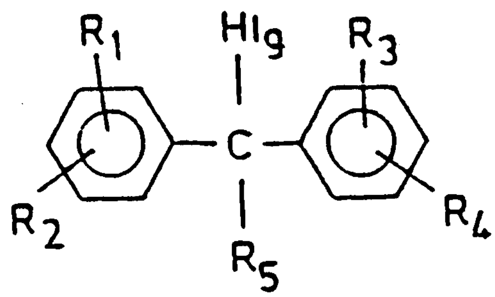

IV ,

oder, als gegebenenfalls substituierte, Diphenyl-Verbindungen der allgemeinen Formel VI

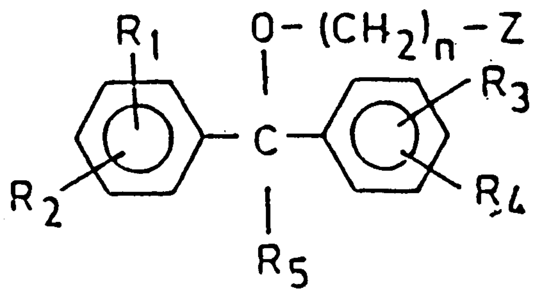

VI ,

oder als gegebenenfalls substituierte, 1-{ω-[bis-(Phenyl)-alkoxy]-alk-ω-yl}-piperazine der allgemeinen Formel

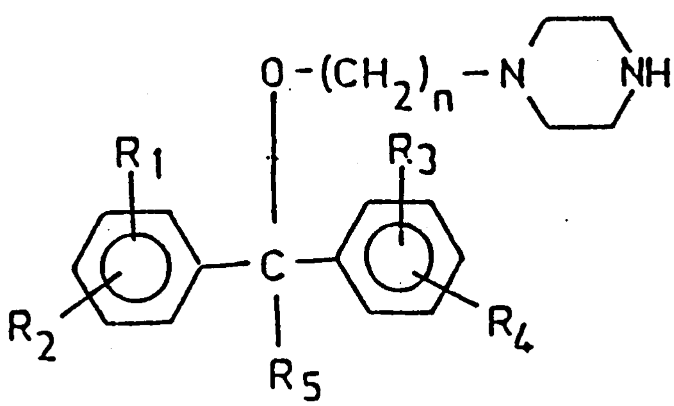

VIII,

oder als gegebenenfalls substituierte, 1-{ω-[bis-(Phenyl)-alkoxy}-alkanoyl}-4-{alkenyl}-piperazine oder

-4-{alkinyl}-piperazine der allgemeinen Formel

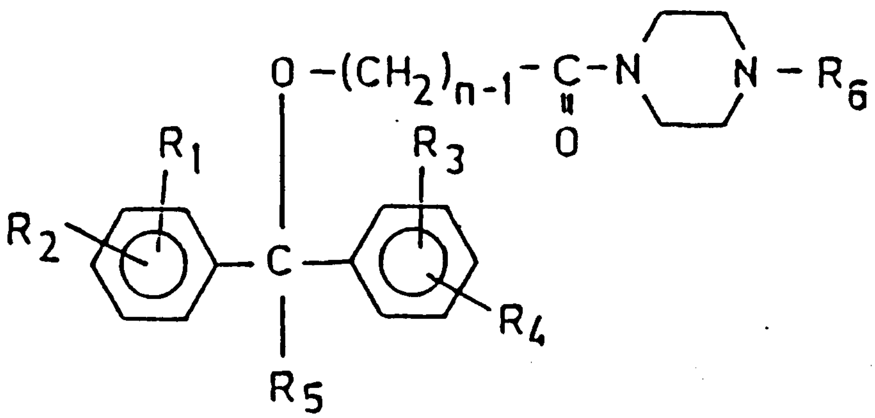

XI ,

solche verwendet, bei welchen der (die) $C_1$-$C_4$-Alkylrest(e), $C_1$-$C_4$-Alkoxyrest(e) und(oder der Alkylteil des (der) Phenyl-$C_1$-$C_4$-Alkoxyreste(s) von $R_1$, $R_2$, $R_3$ und/oder $R_4$ (ein) $C_1$- oder $C_2$Rest(e) ist (sind).

**4.** Verfahren nach Anspruch 1 bis 3, dadurch gekennzeichnet, daß man als gegebenenfalls substituierte, Benzhydrole oder ihre Alkoholate der allgemeinen Formel

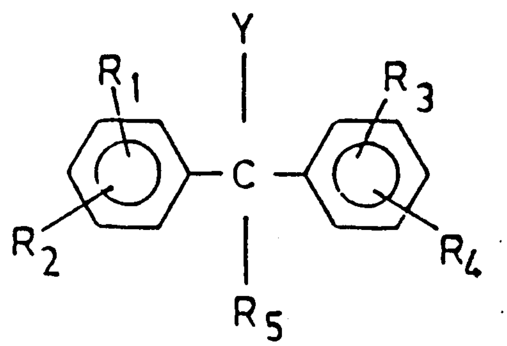

II ,

oder, als gegebenenfalls substituierte, Benzhydrylhalogenide der allgemeinen Formel

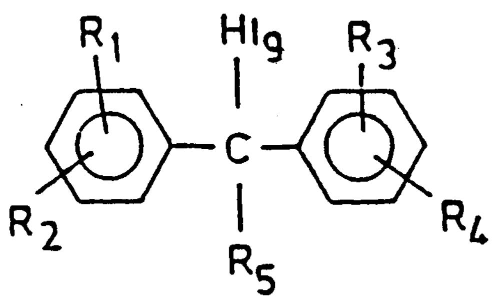

IV ,

oder, als gegebenenfalls substituierte, Diphenyl-Verbindungen der allgemeinen Formel VI

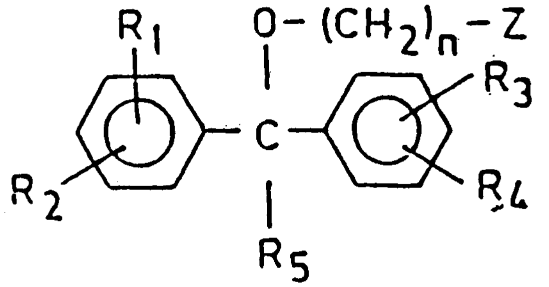

VI ,

oder als gegebenenfalls substituierte, 1-{ω-[bis-(Phenyl)-alkoxy]-alk-ω-yl}-piperazine der allgemeinen Formel

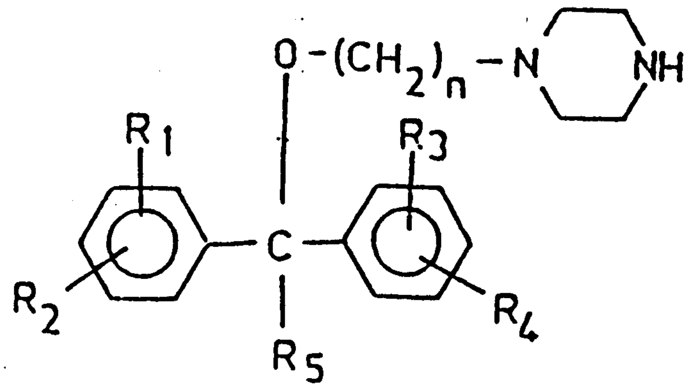

VIII,

oder als gegebenenfalls substituierte, 1-{ω-[bis-(Phenyl)-alkoxy]-alkanoyl}-4-{alkenyl}-piperazine oder -4-{alkinyl}-piperazine der allgemeinen Formel

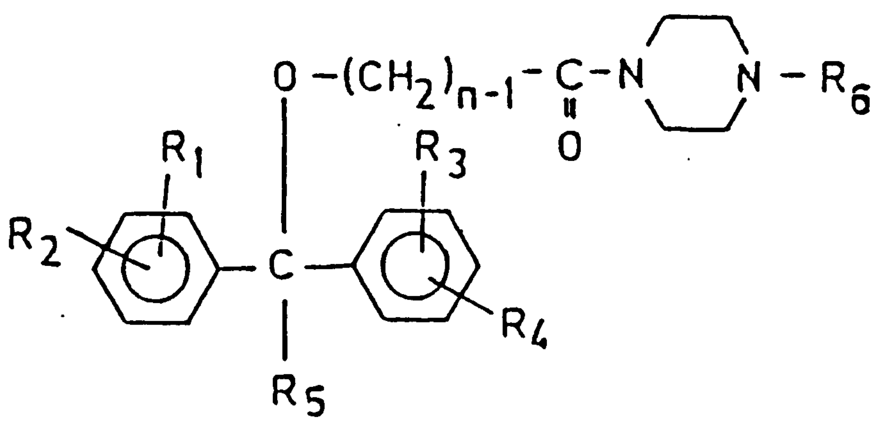

XI ,

solche verwendet, bei welchen $R_5$ ein Alkylrest mit 1 bis 3 Kohlenstoffatomen ist.

5. Verfahren nach Anspruch 1-4, dadurch gekennzeichnet daß man als gegebenenfalls substituierte, Benzhydrole oder ihre Alkoholate der allgemeinen Formel

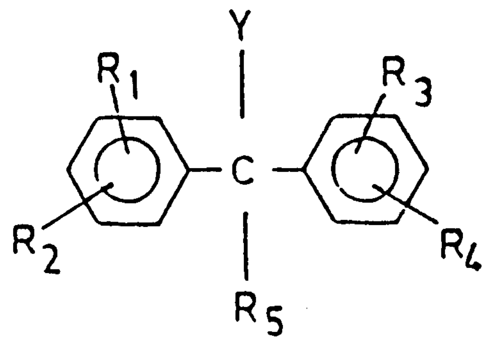

II ,

oder, als gegebenenfalls substituierte, Benzhydrylhalogenide der allgemeinen Formel

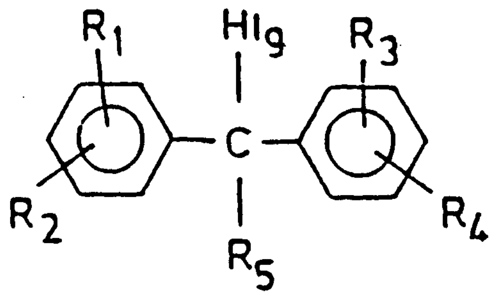

IV ,

oder, als gegebenenfalls substituierte, Diphenyl-Verbindungen der allgemeinen Formel VI

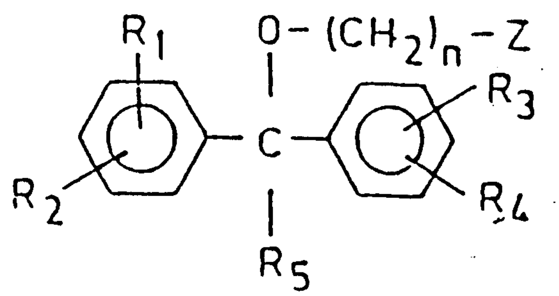

VI ,

oder als gegebenenfalls substituierte, 1-{ω-[bis-(Phenyl)-alkoxy]-alk-ω-yl}-piperazine der allgemeinen Formel

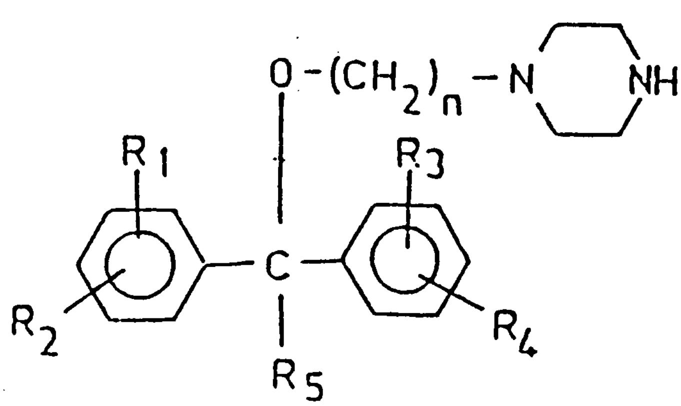

VIII,

oder als gegebenenfalls substituierte, 1-{ω-[bis-(Phenyl)-alkoxy]-alkanoyl}-4-{alkenyl}-piperazine oder -4-{alkinyl}-piperazine der allgemeinen Formel

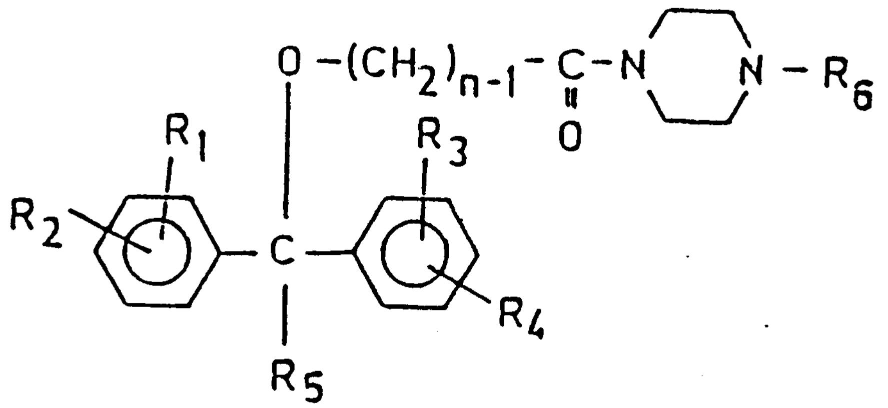

XI ,

solche verwendet werden, bei welchen $R_6$ ein Alkenyl- oder Alkinylrest mit 3 oder 4 Kohlenstoffatomen ist.

**6.** Verfahren nach Anspruch 1 bis 5, dadurch gekennzeichnet, daß man als gegebenenfalls substituierte, Benzhydrole oder ihre Alkoholate der allgemeinen Formel

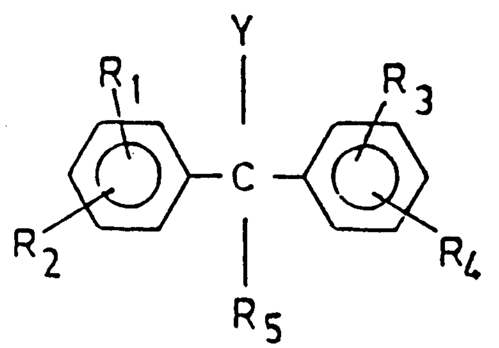

II ,

oder, als gegebenenfalls substituierte, Benzhydrylhalogenide der allgemeinen Formel

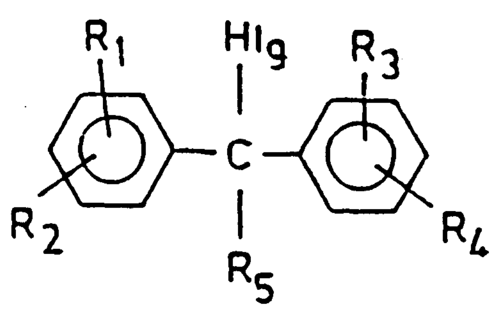

IV ,

oder, als gegebenenfalls substituierte, Diphenyl-Verbindungen der allgemeinen Formel VI

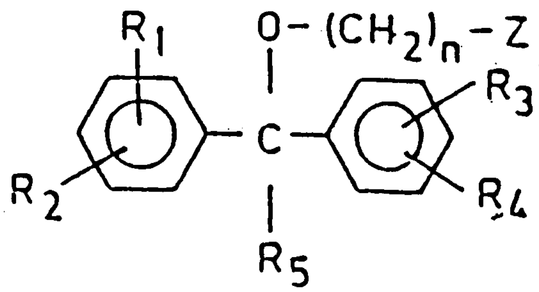

VI ,

oder als gegebenenfalls substituierte, 1-{ω-[bis-(Phenyl)-alkoxy]-alk-ω-yl}-piperazine der allgemeinen Formel

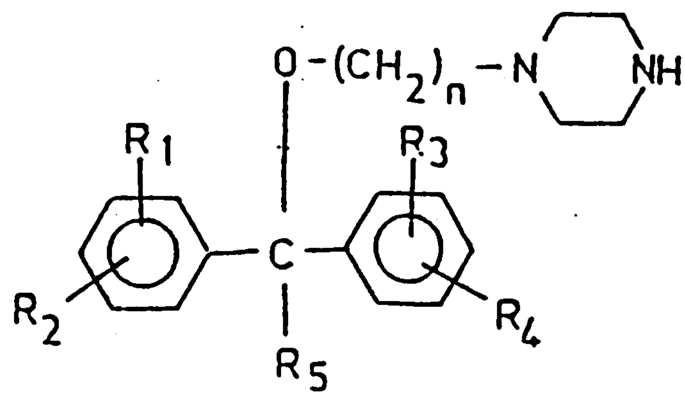

VIII,

oder als gegebenenfalls substituierte, 1-{ω-[bis-(Phenyl)-alkoxy]-alkanoyl}-4-{alkenyl}-piperazine oder -4-{alkinyl}-piperazine der allgemeinen Formel

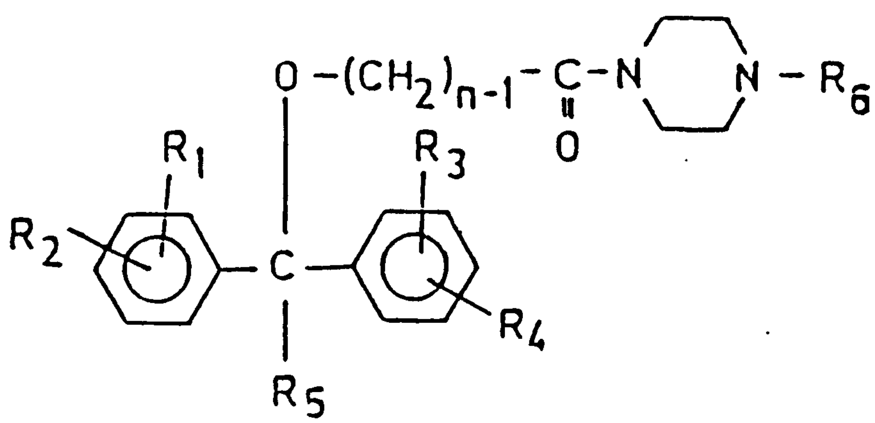

XI ,

solche verwendet, bei welchen der Phenyl-$C_1$-$C_4$-alkoxyrest ein Benzyloxyrest ist.

7. Verfahren nach Anspruch 1 bis 6, daß man als gegebenenfalls substituierte, 1-{ω-[bis-(Phenyl)-alkoxy]-alkyl}-4-{alkenyl}-piperazine und -4-{alkinyl}-piperazine

1-{2'-[bis-⟨4''-(Fluor)-phenyl⟩-methoxy]-äthyl}-4-{prop-2'''-enyl}-piperazin,

1-{2'-[⟨4''-(Fluor)-phenyl⟩-⟨phenyl⟩-methoxy]-äthyl}-4-{prop-2'''-enyl}-piperazin,

1-{2'-[⟨4''-(Chlor)-phenyl⟩-⟨4'''-(fluor)-phenyl⟩-methoxy]-äthyl}-4-{prop-2''''-enyl}-piperazin,

1-{2'-[⟨4''-(Brom)-phenyl⟩-⟨4'''-(fluor)-phenyl⟩-methoxy]-äthyl}-4-{prop-2''''-enyl}-piperazin,

1-{2'-[bis-‹4''-(Chlor)-phenyl›-methoxy]-äthyl}-4-{prop-2'''-enyl}-piperazin,

1-{2'-[‹3'',4''-Di-(chlor)-phenyl›-‹phenyl›-methoxy]-äthyl}-4-{prop-2'''-enyl}-piperazin,

1-{2'-[1''-bis-‹4'''-(Fluor)-phenyl›-äthoxy]-äthyl}-4-{prop-2''''-enyl}-piperazin,

1-{2'-[bis-‹4''-(Fluor)-phenyl›-methoxy]-äthyl}-4-{prop-2'''-inyl}-piperazin,

1-{2'-[bis-‹4''-(Fluor)-phenyl›-methoxy]-äthyl}-4-{2'''-(methyl)-prop-2'''-enyl}-piperazin,

1-{2'-[‹3''-(Nitro)-4''-(1'''-[prop-2''''-enyl]-piperazin-4'''-yl)-phenyl›-‹phenyl›-methoxy]-äthyl}-4-{prop-2'''''-enyl}-piperazin,

1-{2'-[1''-‹2''',5'''-Di-(methyl)-phenyl›-1''-‹phenyl›-propoxy]-äthyl}-4-{prop-2''''-enyl}-piperazin,

1-{2'-[‹Di-(phenyl)›-methoxy]-äthyl}-4-{prop-2''-enyl}-piperazin,

1-{2'-[‹2''-(Chlor)-phenyl›-‹4'''-(fluor)-phenyl›-methoxy]-äthyl}-4-{prop-2''''-enyl}-piperazin,

1-{2'-[‹4''-(Trifluormethyl)-phenyl›-‹4'''-(fluor)-phenyl›-methoxy]-äthyl}-4-{prop-2''''-enyl}-piperazin,

1-{2'-[‹2''-(Methyl)-phenyl›-‹4'''-(fluor)-phenyl›-methoxy]-äthyl}-4-{prop-2''''-enyl}-piperazin,

1-{2'-[‹3''-(Äthoxy)-4''-(hydroxy)-phenyl›-‹4'''-(fluor)-phenyl›-methoxy]-äthyl}-4-{prop-2''''-enyl}-piperazin,

1-{2'-[‹3''-(Trifluormethyl)-phenyl›-‹4'''-(fluor)-phenyl›-methoxy]-äthyl}-4-{prop-2''''-enyl}-piperazin,

1-{2'-[‹3''-(Trifluormethyl)-phenyl›-‹phenyl›-methoxy]-äthyl}-4-{prop-2'''-enyl}-piperazin,

1-{2'-[‹4''-(Benzyloxy)-phenyl›-‹4'''-(fluor)-phenyl›-methoxy]-äthyl}-4-{prop-2''''-enyl}-piperazin,

1-{2'-[‹4''-(Chlor)-phenyl›-‹4'''-(fluor)-phenyl›-methoxy]-äthyl}-4-{prop-2''''-inyl}-piperazin,

1-{2'-[‹4''-(Fluor)-phenyl›-‹phenyl›-methoxy]-äthyl}-4-{prop-2'''-inyl}-piperazin,

1-{2'-[‹3'',4''-Di-(chlor)-phenyl›-‹phenyl›-methoxy]-äthyl}-4-{prop-2'''-inyl}-piperazin,

1-{2'-[‹4''-(Brom)-phenyl›-‹4'''-(fluor)-phenyl›-methoxy]-äthyl}-4-{prop-2''''-inyl}-piperazin,

1-{2'-[‹4''-(Trifluormethyl)-phenyl›-‹4'''-(fluor)-phenyl›-methoxy]-äthyl}-4-{prop-2''''-inyl}-piperazin,

1-{2'-[bis-‹4''-(Chlor)-phenyl›-methoxy]-äthyl}-4-{prop-2'''-inyl}-piperazin und

1-{2'-[bis-‹4''-(Chlor)-phenyl›-methoxy]-äthyl}-4-{prop-2'''-enyl}-piperazin herstellt.

**8.** Verfahren nach Anspruch 1, dadurch gekennzeichnet, daß man gegebenenfalls substituierte, 1-{ω-[bis-(Phenyl)-alkoxy]-alkyl}-4-{alkinyl}-piperazine der allgemeinen Formel I, bei welchen $R_1$ , $R_2$ , $R_3$ , $R_4$ und $R_5$ sowie n die in den Ansprüchen 1 bis 6 angegebenen Bedeutungen haben und $R_6$ für einen Alkinylrest steht, zu 1-{ω-[bis-(Phenyl)-alkoxy]-alkyl}-4-{alkenyl}-piperazinen der allgemeinen Formel I, bei welchen $R_6$ für einen Alkenylrest steht, reduziert.

**9.** Verfahren nach Anspruch 1, dadurch gekennzeichnet, daß man durch 1 oder mehr Benzyloxyrest(e) substituierte 1-{ω-[bis-(Phenyl)-alkoxy]-alkyl} -4-{alkenyl}-piperazine oder -4-{alkinyl}-piperazine der allgemeinen Formel

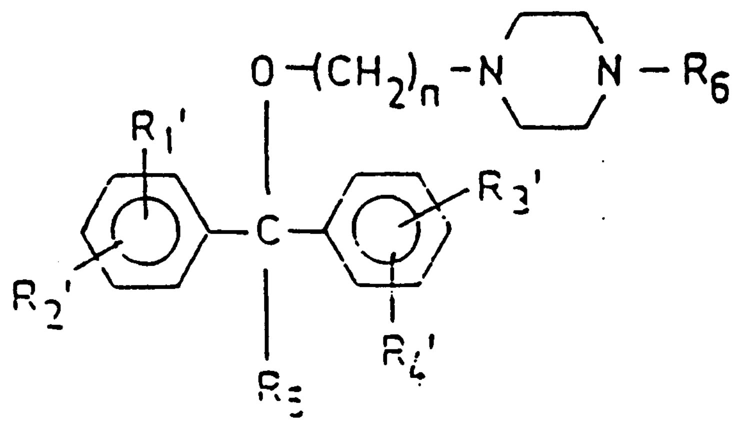

Ia ,

worin

| | |
|---|---|
| $R_5$ , $R_6$ und n | die in den Ansprüchen 1 bis 6 angegebenen Bedeutungen haben und |
| bei mindestens 1 von $R_1'$ , $R_2'$ , $R_3'$ und $R_4'$ | der Phenyl-$C_1$-$C_4$-Alkoxyrest ein Benzyloxyrest ist und |
| die etwaigen anderen von $R_1'$ , $R_2'$ , $R_3'$ und $R_4'$ | die in den Ansprüchen 1 bis 6 definierte Bedeutungen haben, |

zu solchen Piperazinen der allgemeinen Formel I reduziert, bei welchen mindestens 1 von $R_1$ , $R_2$ , $R_3$ , und $R_4$ für eine Hydroxygruppe steht.

**10.** Verfahren nach Anspruch 1, dadurch gekennzeichnet, daß man als Reduktionsmittel Aluminiumkomplexe verwendet.

**11.** Verfahren nach einem der Ansprüche 1 - 10, dadurch gekennzeichnet, daß man in für die Reaktionsteilnehmer inerten Lösungsmittel arbeitet.

**Claims**

**Claims for the following Contracting States : BE, CH, DE, FR, GB, IT, LI, LU, NL, SE**

**1.** Optionally substituted 1-[ω-[bis-[phenyl]-alkoxyl]-alkyl]-4-]alkenyl]-piperazines and -4-[alkinyl]-piperazines of the general formula I

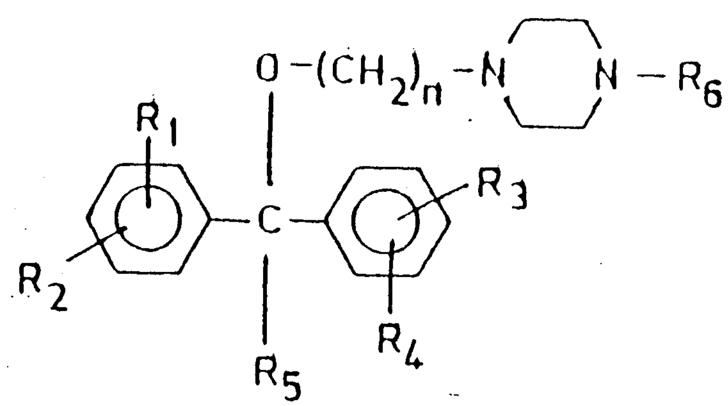

I ,

wherein

| | |
|---|---|
| $R_1$, $R_2$, $R_3$, and $R_4$ | independently of one another denote hydrogen atoms, halogen atoms, trihalomethyl radicals, $C_1$-$C_4$ alkyl radicals, $C_1$-$C_4$ alkoxy radicals, nitrogroups, hydroxy groups, 1-(prop-2'-enyl)-piperazin-4-yl radicals or phenyl $C_1$-$C_4$ alkoxy radicals, |
| $R_5$ | denotes a hydrogen atom or alkyl radical with 1 to 4 carbon atoms, |
| $R_6$ | is an alkenyl or alkinyl radical with 3 to 6 carbon atoms, and |

n is equal to 2 or 3;
as well as their acid addition salts, quaternary salts, and optically active isomers and solvates.

**2.** Piperazines according to Claim 1, characterized in that the halogen atom(s) and/or the halogen atoms of the trihalomethyl radical(s) of $R_1$, $R_2$, $R_3$ and/or $R_4$ is/are fluorine, chlorine and/or bromine.

**3.** Piperazines according to Claim 1 or 2, characterized in that the $C_1$-$C_4$ alkyl radical(s), $C_1$-$C_4$ alkoxy radical(s) and/or the alkyl part of the phenyl $C_1$-$C_4$ alkoxy radical(s) of $R_1$, $R_2$, $R_3$ and/or $R_4$ is a $C_1$ or $C_2$ radical(s).

**4.** Piperazines according to Claims 1 to 3, characterized in that $R_5$ is an alkyl radical with 1 to 3 carbon atoms.

**5.** Piperazines according to Claims 1 to 4, characterized in that $R_6$ is an alkenyl or alkinyl radical with 3 or 4 carbon atoms.

**6.** Piperazines according to Claims 1 to 5, characterized in that the phenyl $C_1$-$C_4$ alkoxy radical is a benzyloxy radical.

**7.** 1-{2'-[bis-⟨4''-(fluoro)-phenyl⟩-methoxy]-ethyl}-4-{prop-2'''-enyl}-piperazine,

1-{2'-[⟨4''-(fluoro)-phenyl⟩-⟨phenyl⟩-methoxy]-ethyl}-4-{prop-2'''-enyl}-piperazine,

1-{2'-[⟨4''-(chloro)-phenyl⟩-⟨4'''-(fluoro)-phenyl⟩-methoxy]-ethyl}-4-{prop-2''''-enyl}-piperazine,

1-{2'-[⟨4''-(bromo)-phenyl⟩-⟨4'''-(fluoro)-phenyl⟩-methoxy]-ethyl}-4-{prop-2''''-enyl}-piperazine,

1-{2'-[bis-⟨4''-(Chloro)-phenyl⟩-methoxy]-ethyl}-4-{prop-2'''-enyl}-piperazine,

1-{2'-[⟨3'',4''-di-(chloro)-phenyl⟩-⟨phenyl⟩-methoxy]-ethyl}-4-{prop-2'''-enyl}-piperazine,

1-{2'-[1''-bis-⟨4'''-(fluoro)-phenyl⟩-ethoxy]-ethyl}-4-{prop-2''''-enyl}-piperazine,

1-{2'-[bis-⟨4''-(fluoro)-phenyl⟩-methoxy]-ethyl}-4-{prop-2'''-inyl}-piperazine,

1-{2'-[bis-⟨4''-(fluoro)-phenyl⟩-methoxy]-ethyl}-4-{2'''-(methyl)-prop-2'''-enyl}-piperazine,

1-{2'-[⟨3''-(nitro)-4''-(1'''-[prop-2''''-enyl]-piperazin-4'''-yl)-phenyl⟩-⟨phenyl⟩-methoxy]-ethyl}-4-{prop-2'''''-enyl}-piperazine,

1-{2'-[1''-⟨2''',5'''-di-(methyl)-phenyl⟩-1''-⟨phenyl⟩-propoxy]-ethyl}-4-{prop-2''''-enyl}-piperazine,

1-{2'-[⟨di-(phenyl)⟩-methoxy]-ethyl}-4-{prop-2''-enyl}-piperazine,

1-{2'-[⟨2''-(chloro)-phenyl⟩-⟨4'''-(fluoro)-phenyl⟩-methoxy]-ethyl}-4-{prop-2''''-enyl}-piperazine,

1-{2'-[⟨4''-(trifluoromethyl)-phenyl⟩-⟨4'''-(fluoro)-phenyl⟩-methoxy]-ethyl}-4-{prop-2''''-enyl}-piperazine,

1-{2'-[⟨2''-(methyl)-phenyl⟩-⟨4'''-(fluoro)-phenyl⟩-methoxy]-ethyl}-4-{prop-2''''-enyl}-piperazine,

1-{2'-[⟨3''-(ethoxy)-4''-(hydroxy)-phenyl⟩-⟨4'''-(fluoro)-phenyl⟩-methoxy]-ethyl}-4-{prop-2''''-enyl}-piperazine,

1-{2'-[⟨3''-(trifluoromethyl)-phenyl⟩-⟨4'''-(fluoro)-phenyl⟩-methoxy]-ethyl}-4-{prop-2''''-enyl}-piperazine,

1-{2'-[⟨3''-(trifluoromethyl)-phenyl⟩-⟨phenyl⟩-methoxy]-ethyl}-4-{prop-2'''-enyl}-piperazine,

1-{2'-[⟨4''-(benzyloxy)]-phenyl⟩-⟨4'''-(fluoro)-phenyl⟩-methoxy]-ethyl}-4-{prop-2''''-enyl}-piperazine,

1-{2'-[⟨4''-(chloro)-phenyl⟩-⟨4'''-(fluoro)-phenyl⟩-methoxy]-ethyl}-4-{prop-2''''-inyl}-piperazine,

1-{2'-[⟨4''-(fluoro)-phenyl⟩-⟨phenyl⟩-methoxy]-ethyl}-4-{prop-2'''-inyl}-piperazine,

1-{2'-[⟨3'',4''-di-(chloro)-phenyl⟩-⟨phenyl⟩-methoxy]-ethyl}-4-{prop-2'''-inyl}-piperazine,

1-{2'-[⟨4''-(bromo)-phenyl⟩-⟨4'''-(fluoro)-phenyl⟩-methoxy]-ethyl}-4-{prop-2''''-inyl}-piperazine,

1-{2'-[⟨4''-(trifluoromethyl)-phenyl⟩-⟨4'''-(fluoro)-phenyl⟩-methoxy]-ethyl}-4-{prop-2''''-inyl}-piperazine,

1-{2'-[bis-⟨4''-(chloro)-phenyl⟩-methoxy]-ethyl}-4-{prop-2'''-inyl}-piperazine, and

1-{2'-[bis-⟨4''-(chloro)-phenyl⟩-methoxy]-ethyl}-4-{prop-2'''-enyl}-piperazine.

**8.** Process for preparing piperazine compounds according to Claims 1 to 7, characterized in that
a) optionally substituted benzhydrols or their alcoholates of the general formula

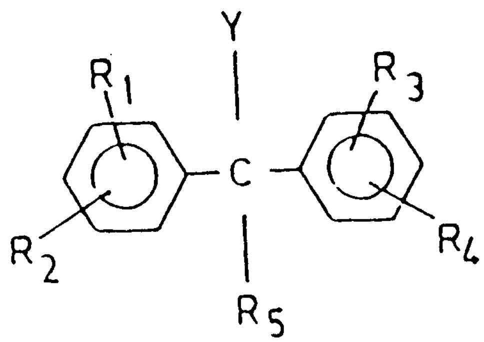

II

wherein

| | |
|---|---|
| $R_1$, $R_2$, $R_3$, $R_4$ and $R_5$ | have the meanings given in Claims 1 to 6, and |
| Y | denotes a hydroxy group or a radical of the general formula |

-OM

in which latter

M denotes an alkali metal atom or a radical of the formula

-MgHlg,

in which latter

Hlg denotes a halogen atom,

are reacted with a piperazine compound of the general formula

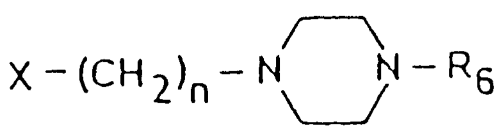

III

wherein

$R_6$ and n have the meanings given in Claims 1 or 5, and

X denotes a halogen atom, a hydroxy group, an alkylsulphonyloxy radical or an arylsulphonyloxy radical, with the restriction that,

X is different from a hydroxy group if Y denotes a radical of the general formula -OM, or
b) optionally substituted benzhydryl halides of the general formula

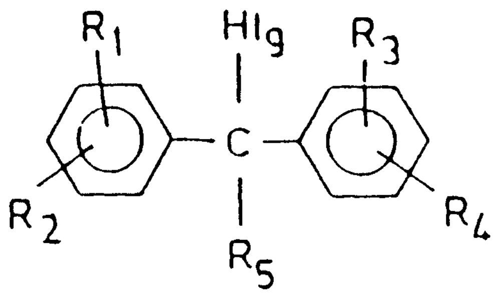

IV

wherein

| | |
|---|---|
| $R_1$, $R_2$, $R_3$, $R_4$ and $R_5$ | have the meanings given in Claims 1 to 6, and |
| Hlg | has the above meaning, |

are reacted with a piperazine compound of the general formula V or an alcoholate thereof

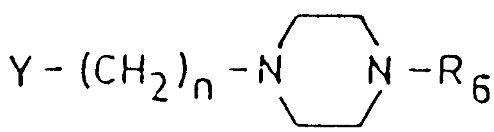

V

wherein

| | |
|---|---|
| $R_6$ and n | have the meanings given in Claims 1 or 5, and |
| Y | denotes a hydroxy group or a metal oxy radical of the general formula |

-OM'

in which latter

| | |
|---|---|
| M' | denotes an alkali metal atom, or |

c) optionally substituted diphenyl compounds of the general formula VI

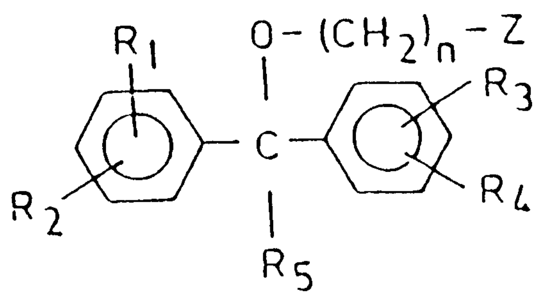

VI

wherein

| | |
|---|---|
| $R_1$, $R_2$, $R_3$, $R_4$ and $R_5$ as well as n | have the meanings given in Claims 1 to 6 and, |
| Z | denotes a halogen atom or an alkylsulphonyloxy or arylsulphonyloxy radical, |

are reacted with 1-(alkenyl)-piperazines or 1-(alkinyl)-piperazines of the general formula

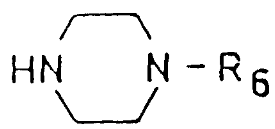

VII

wherein

$R_6$ has the meanings given in Claims 1 or 5, or

d) optionally substituted 1-{ω-[bis-(phenyl)-alkoxy]-alk-ω-yl} piperazines of the general formula

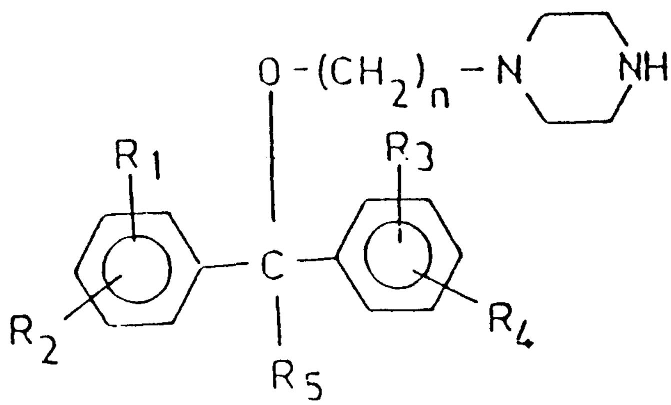

VIII

wherein

$R_1$, $R_2$, $R_3$, $R_4$ and $R_5$ as well as n have the meanings given in Claims 1 to 6,

are reacted with a compound of the general formula

Z - $R_6$ IX

wherein

$R_6$ has the meanings given in Claims 1 or 5, and

Z has the above-specified meaning, or

e) optionally substituted 1-[ω-[bis-(phenyl)-alkoxy]-alkanoyl]-4-[alkenyl]-piperazines or -4-[alkinyl]-piperazines of the general formula

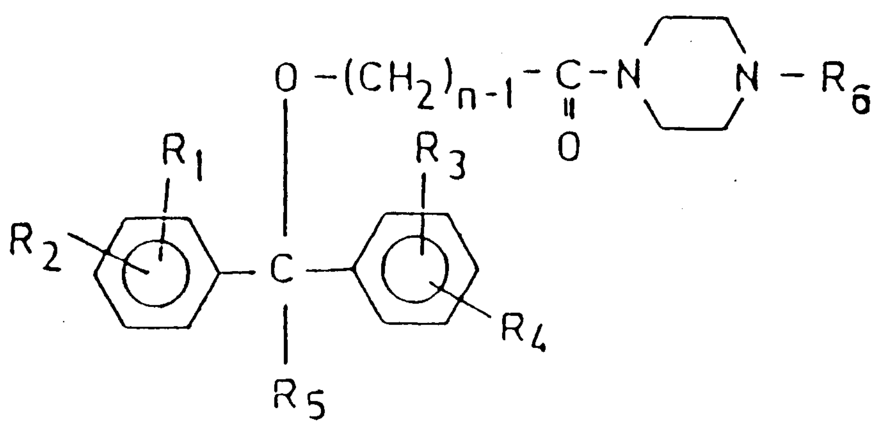

XI

wherein

$R_1$, $R_2$, $R_3$, $R_4$ and $R_5$ as well as n have the meanings given in Claims 1 to 6, and

$R_6$ denotes an alkenyl or alkinyl radical with 3 to 6 carbon atoms,

are reduced,

as well as in a manner known per se optionally the 1-[ω-[bis-(phenyl)-alkoxy]-alkyl]-4-[alkenyl]-piperazines or -4-[alkinyl]-piperazines of the general formula I obtained according to one of variants a)

to e) are converted with inorganic or organic acids into acid addition salts or with quaternization agents into quaternary salts and/or the obtained acid addition salts or quaternary salts of the 1-[ -[bis-(phenyl)-alkoxy]-alkyl]-4-[alkenyl]-piperazines or -4-[alkinyl]-piperazines of the general formula I are converted into the corresponding free 1-[ -[bis-(phenyl)-alkoxy]-alkyl]-4-[alkenyl]-piperazines or -4-[alkinyl)-piperazine bases of the general formula I or into other acid addition salts and/or quaternary salts, and/or optionally the obtained racemic piperazines of the general formula I or their acid addition salts or quaternary salts are resolved or the corresponding optically active compounds are racemized into stereoisomers.

**9.** Process according to Claim 8, characterized in that optionally substituted 1-[ω-[bis-(phenyl)-alkoxy]-alkyl]-4-[alkinyl]-piperazines of the general formula I in which $R_1$, $R_2$, $R_3$, $R_4$ and $R_5$ as well as n have the meanings given in Claims 1 to 6 and $R_6$ denotes an alkinyl radical, are reduced to 1-[ - [bis-(phenyl)-alkoxy]-alkyl]-4-[alkenyl]-piperazines of the general formula I in which $R_6$ denotes an alkenyl radical.

**10.** Process according to Claim 8, characterized in that 1-[ω-[bis-(phenyl)-alkoxy]-alkyl]-4-[alkenyl]-piperazines or -4-[alkinyl]-piperazines of the general formula

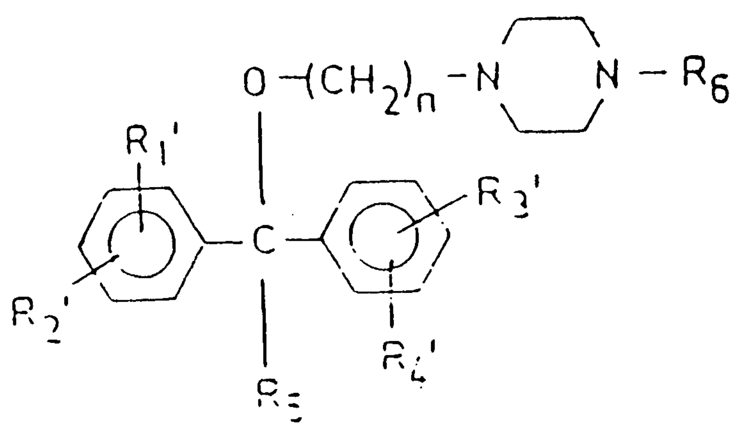

Ia ,

substituted by one or more benzyloxy radicals, wherein

| | |
|---|---|
| $R_5$, $R_6$ and n | have the meanings in Claims 1 to 6, and in which |
| at least one of $R'_1$, $R'_2$, $R'_3$, and $R_4'$ | of the phenyl $C_1$-$C_4$ alkoxy radical is a benzyloxy radical, |
| and the remaining $R'_1$, $R'_2$, $R'_3$ and $R_4'$ | have the meanings given in Claims 1 to 6, |

are reduced to those piperazines of the general formula I in which at least one of $R_1$, $R_2$, $R_3$ and $R_4$ denotes a hydroxy group.

**11.** Process according to Claim 8, characterized in that aluminium complexes are used as reducing agent.

**12.** Process according to one of Claims 8 to 11, characterized in that the process is performed in solvents inert with respect to the reactants.

**13.** Medicaments, characterized by a content of one or more compounds according to Claims 1 to 7 as active constituent(s), optionally together with one or more conventional pharmaceutical carriers and/or adjuvants.

**14.** Use of 1-{ω-[bis-(phenyl)-alkoxy]-alkyl]-4-[alkyl}-piperazines and 4-[acyl]-piperazines of the general formula

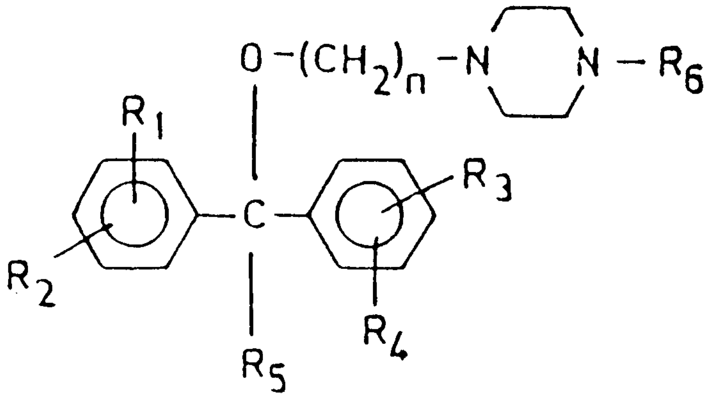

I

wherein

| | |
|---|---|
| $R_1$ | denotes a hydrogen atom or a halogen atom in the o- or p-position, |
| $R_2$, $R_3$, $R_4$ and $R_5$ | denote hydrogen, and |
| $R_6$ | denotes an alkyl radical with 3 to 6 carbon atoms or an acyl radical of the general formula |

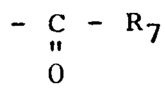

in which latter $R_7$ denotes an alkyl radical with 2 to 5 carbon atoms, and

| | |
|---|---|
| n | is 2 or 3 |

for the preparation of medicaments having a dopaminergic action on the central nervous system.

**Claims for the following Contracting State : AT**

**1**. Process for preparing optionally substituted 1-[ω-[bis-(phenyl)-alkoxy]-alkyl]-4-[alkenyl]-piperazinesand -4-[alkinyl]-piperazines of the general formula I

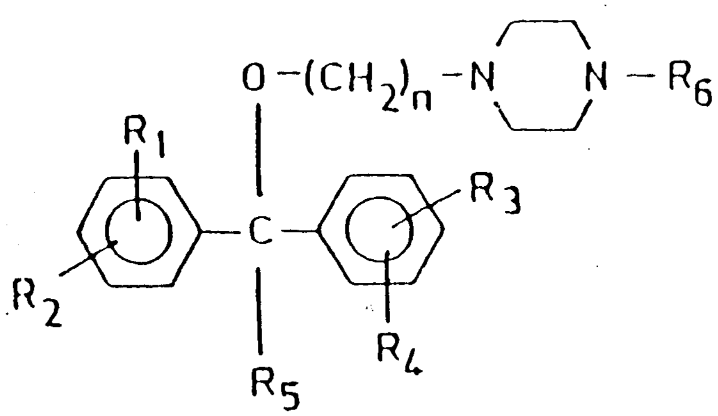

I

wherein

| | |
|---|---|
| $R_1$, $R_2$, $R_3$ and $R_4$ | independently of one another denote hydrogen atoms, halogen atoms, trihalomethyl radicals, $C_1$-$C_4$ alkyl radicals, $C_1$-$C_4$ alkoxy radicals, nitrogroups, hydroxy groups, 1-(prop-2'-enyl)-piperazin-4-yl radicals or phenyl $C_1$-$C_4$ alkoxy radicals, |
| $R_5$ | denotes a hydrogen atom or an alkyl radical with 1 to 4 carbon atoms, |

$R_6$ is an alkenyl or alkinyl radical with 3 to 6 carbon atoms, and
n is equal to 2 or 3;
as well as their acid addition salts, quaternary salts, and optically active isomers and solvates,

characterized in that

a) optionally substituted benzhydrols or their alcoholates of the general formula

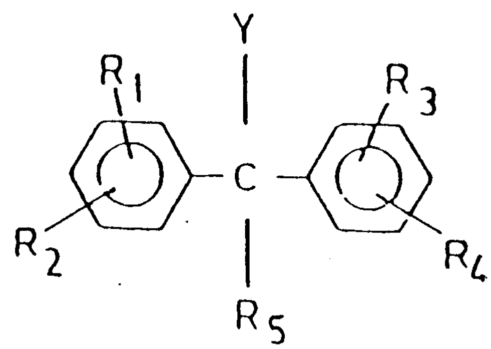

II

wherein

$R_1$, $R_2$, $R_3$, $R_4$ and $R_5$ have the meanings given in Claims 1 to 6, and
Y denotes a hydroxy group or a radical of the general formula

-OM

in which latter

M denotes an alkali metal atom or a radical of the formula

-MgHlg,

in which latter

Hlg denotes a halogen atom,

are reacted with a piperazine compound of the general formula

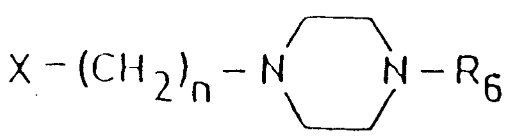

III

wherein

$R_6$ and n have the meanings given in Claims 1 or 5, and

X denotes a halogen atom, a hydroxy group, an alkylsulphonyloxy radical or an arylsulphonyloxy radical, with the restriction that,

X is different from a hydroxy group if Y denotes a radical of the general formula -OM, or

b) optionally substituted benzhydryl halides of the general formula

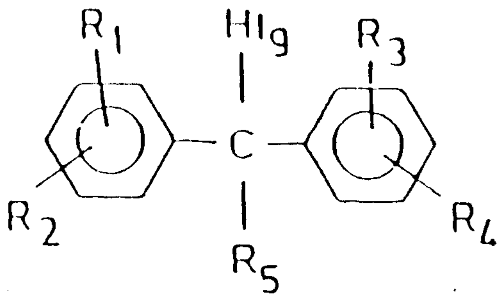

IV

wherein

$R_1$, $R_2$, $R_3$, $R_4$ and $R_5$ have the meanings given in Claims 1 to 6, and

Hlg has the above meaning,

are reacted with a piperazine compound of the general formula V or an alcoholate thereof

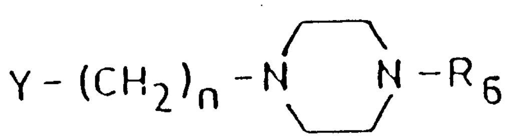

V

wherein

$R_6$ and n have the meanings given in Claims 1 or 5, and

Y denotes a hydroxy group or a metal oxy radical of the general formula

-OM'

in which latter

M' denotes an alkali metal atom, or

c) optionally substituted diphenyl compounds of the general formula VI

wherein

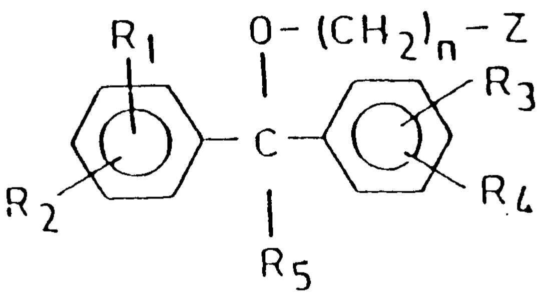

VI

$R_1$, $R_2$, $R_3$, $R_4$ and $R_5$ as well as n have the meanings given in Claims 1 to 6, and

Z denotes a halogen atom or an alkylsulphonyloxy or arylsulphonyloxy radical,

are reacted with 1-(alkenyl)-piperazines or 1-(alkinyl)-piperazines of the general formula

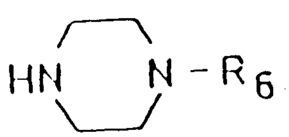

VII

wherein

$R_6$ has the meanings given in Claims 1 or 5, or

d) optionally substituted 1-[ω-[bis-(phenyl)-alkoxy]-alk-ω-yl]-piperazines of the general formula

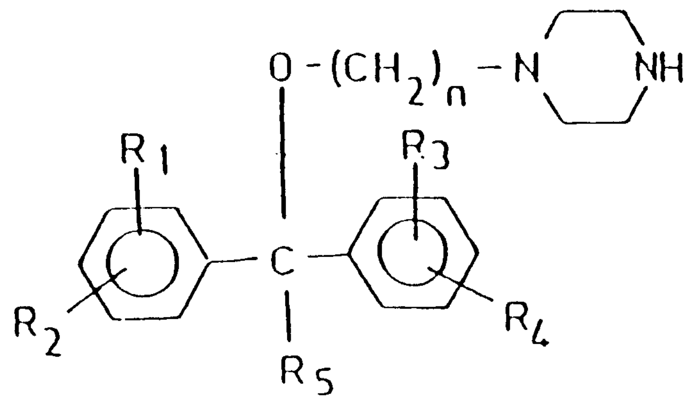

VIII

wherein

$R_1$, $R_2$, $R_3$, $R_4$ and $R_5$ as well as n have the meanings given in Claims 1 to 6

are reacted with a compound of the general formula

Z - $R_6$ IX

wherein

$R_6$ has the meanings given in Claims 1 or 5, and

Z has the above-specified meaning, or

e) optionally substituted 1-[ω-[bis-(phenyl)-alkoxy]-alkanoyl]-4-[alkenyl]-piperazines or -4-[alkinyl]-piperazines of the general formula

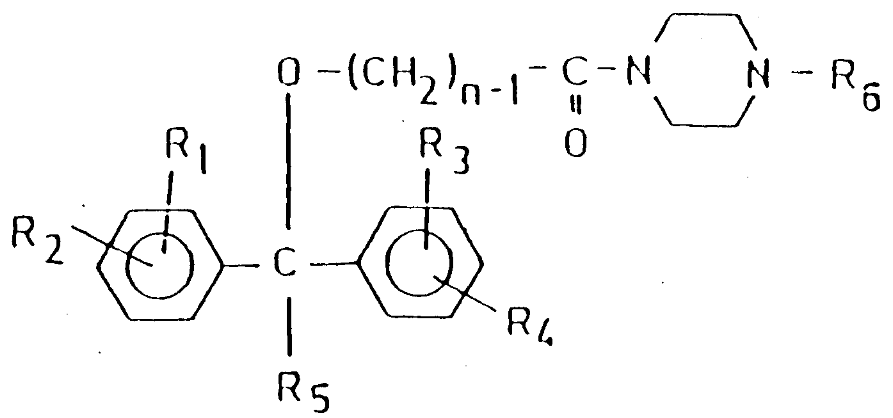

XI

wherein

$R_1$, $R_2$, $R_3$, $R_4$ and $R_5$ as well as n have the meanings given in Claims 1 to 6, and

$R_6$ denotes an alkenyl or alkinyl radical with 3 to 6 carbon atoms are reduced,

as well as in a manner known per se optionally the 1-[ω-[bis-(phenyl)-alkoxy]-alkyl]-4-[alkenyl]-piperazines or -4-[alkinyl]-piperazines of the general formula I obtained according to one of variants a) to e) are converted with inorganic or organic acids into acid addition salts or with quaternization agents into quaternary salts and/or the obtained acid addition salts or quaternary salts of the 1-[ -[bis-(phenyl)-alkoxy]-alkyl]-4-[alkenyl]-piperazines or -4-[alkinyl]-piperazines of the general formula I are converted into the corresponding free 1-[ -[bis-(phenyl)-alkoxy]-alkyl]-4-[alkenyl]-piperazines or -4-[alkinyl]-piperazine bases of the general formula I or into other acid addition salts and/or quaternary salts, and/or optionally the obtained racemic piperazines of the general formula I or their acid addition salts or quaternary salts are resolved or the corresponding optically active compounds are racemized into stereoisomers.

**2**. Process according to Claim 1, characterized in that as optionally substituted benzhydrols or their alcoholates of the general formula

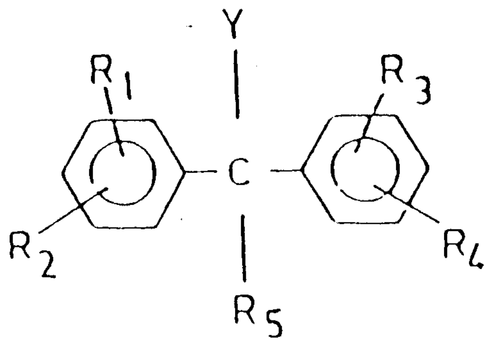

II

or as optionally substituted benzhydryl halides of the general formula

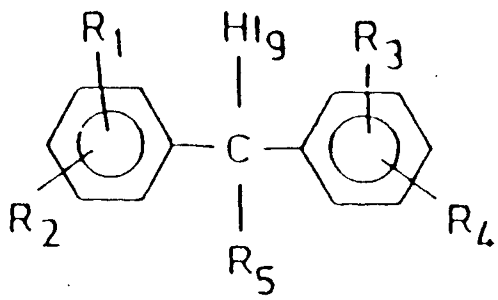

IV

or as optionally substituted diphenyl compounds of the general formula VI

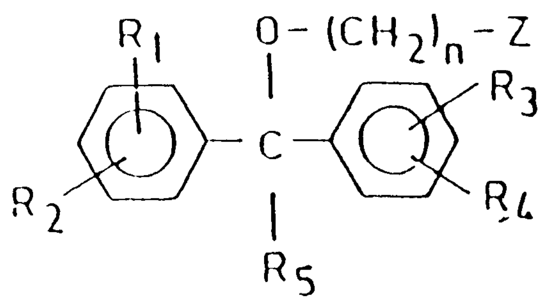

VI

or as optionally substituted 1-[ω-[bis-(phenyl)-alkoxy]-alk-ω-yl]-piperazines of the general formula

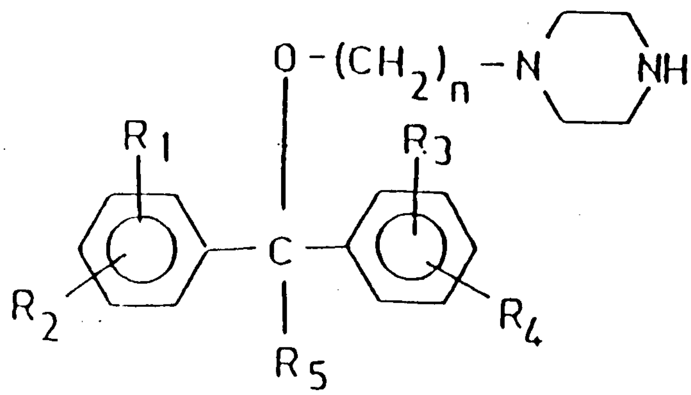

VIII

or as optionally substituted 1-[ω-[bis-(phenyl)-alkoxy]-alkanoyl]-4-[alkenyl]-piperazines or -4-[alkinyl]-piperazines of the general formula

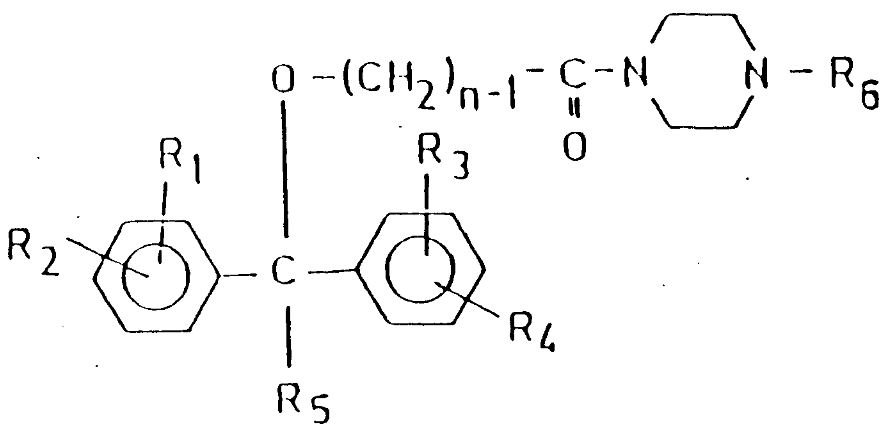

XI

those compounds are used in which the halogen atom(s) and/or the halogen atoms of the trihalomethyl radical(s) of $R_1$, $R_2$, $R_3$ and/or $R_4$ is fluorine, chlorine and/or bromine.

**3**. Process according to Claim 1 or 2, characterized in that, as optionally substituted benzhydrols or their alcoholates of the general formula

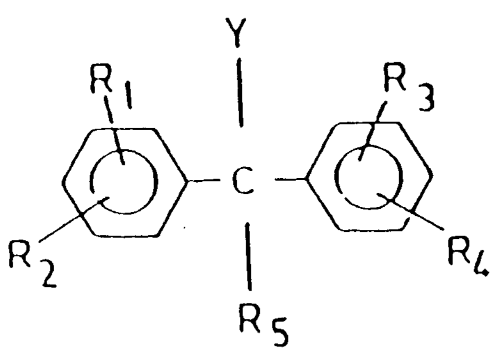

II

or as optionally substituted benzhydryl halides of the general formula

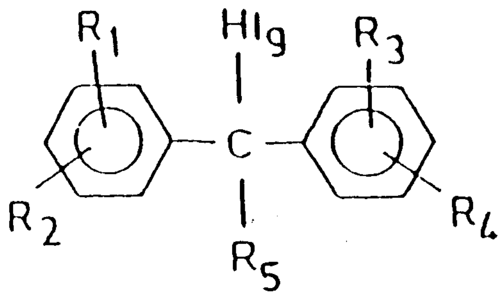

IV

or as optionally substituted diphenyl compounds of the general formula VI

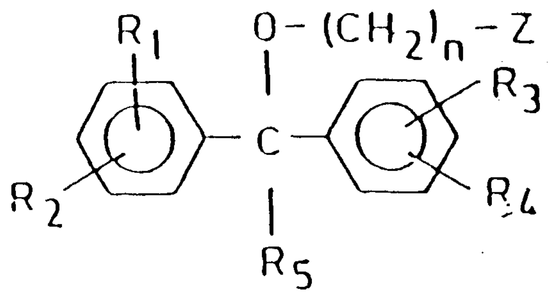

VI

or as optionally substituted 1-[ω-[bis-(phenyl)-alkoxy]-alk-ω-yl]-piperazines of the general formula

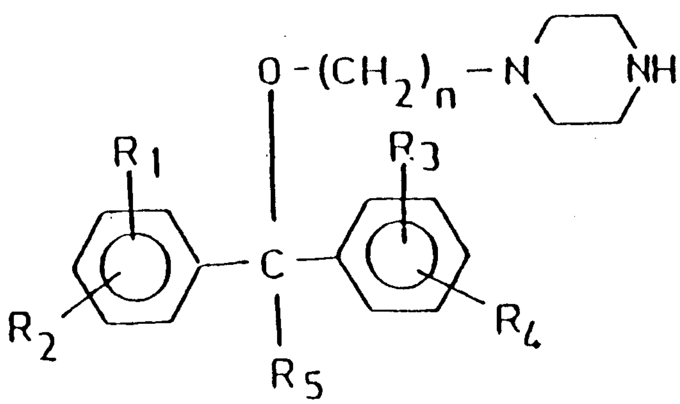

VIII

or as optionally substituted 1-[ω-[bis-(phenyl)-alkoxy]-alkanoyl]-4-[alkenyl]-piperazines or -4-[alkinyl]-piperazines of the general formula

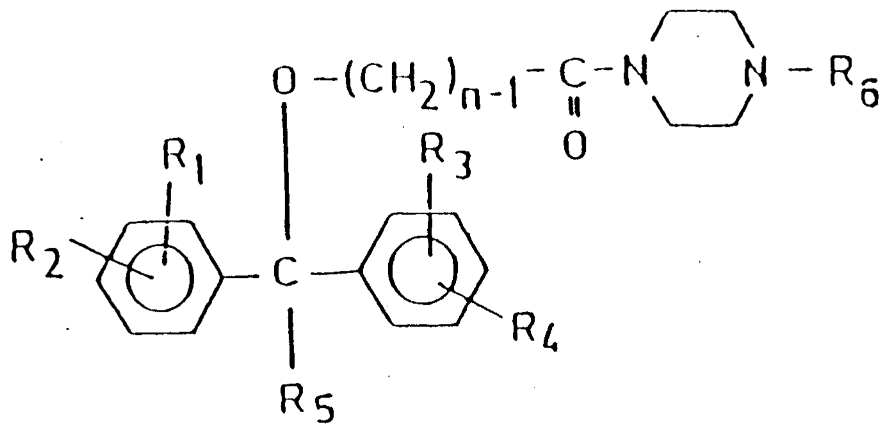

XI

those compounds are used in which the $C_1$-$C_4$ alkyl radical(s), $C_1$-$C_4$ alkoxy radical(s) and/or the alkyl part of the phenyl $C_1$-$C_4$ alkoxy radical(s) of $R_1$, $R_2$, $R_3$ and/or $R_4$ is/are a $C_1$ or $C_2$ radical.

**4**. Process according to Claims 1 to 3, characterized in that, as optionally substituted benzhydrols or their alcoholates of the general formula

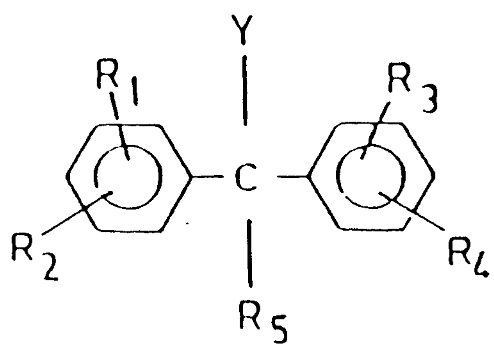

II

or as optionally substituted benzhydryl halides of the general formula

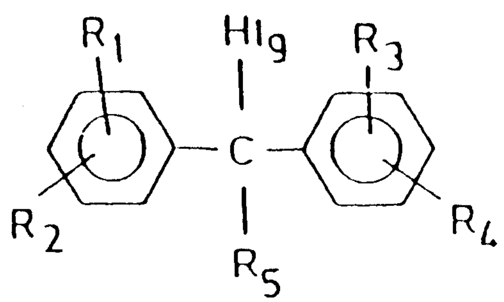

IV

or as optionally substituted diphenyl compounds of the general formula VI

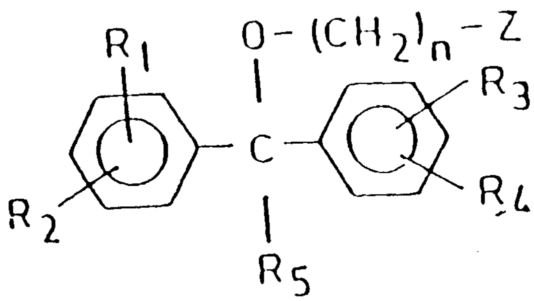

VI

or as optionally substituted 1-[ω-[bis-(phenyl)-alkoxy]-alk-ω-yl]-piperazines of the general formula

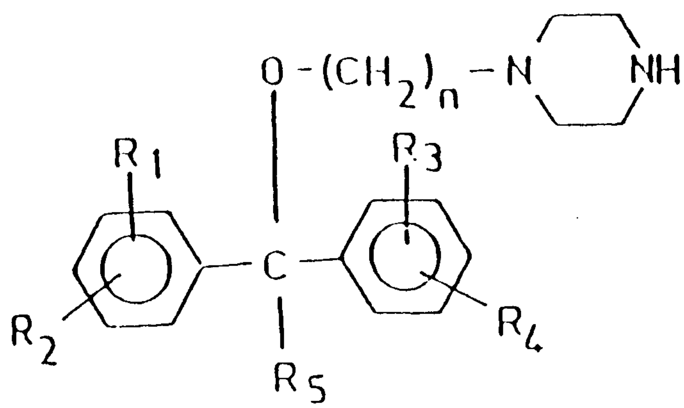

VIII

or as optionally substituted 1-[ω-[bis-(phenyl)-alkoxy]-alkanoyl]-4-[alkenyl]-piperazines or -4-[alkinyl]-piperazines of the general formula

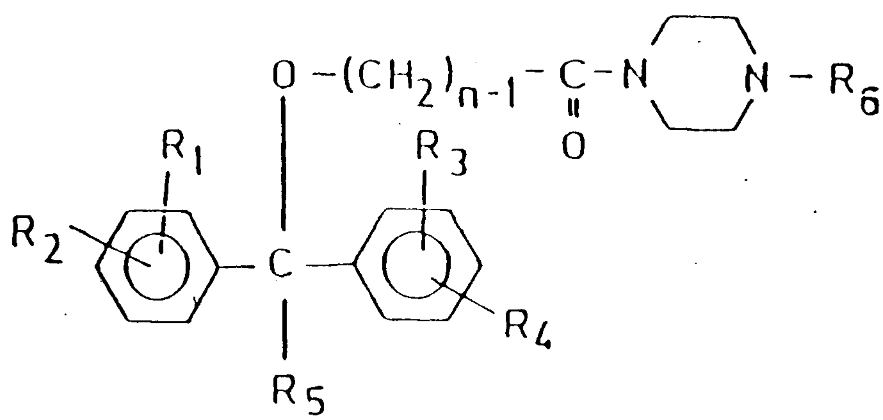

XI

those compounds are used in which $R_5$ is an alkyl radical with 1 to 3 carbon atoms.

**5**. Process according to Claims 1 to 4, characterized in that as optionally substituted benzhydrols or their alcoholates of the general formula

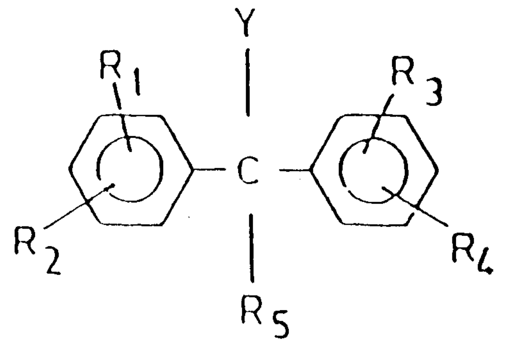

II

or as optionally substituted benzhydryl halides of the general formula

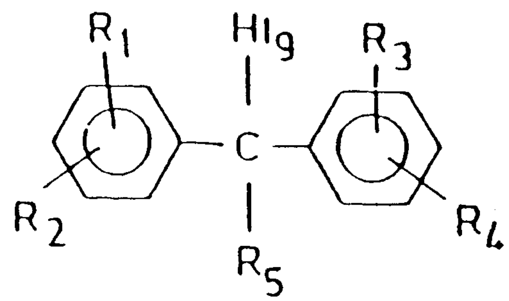

IV

or as optionally substituted diphenyl compounds of the general formula VI

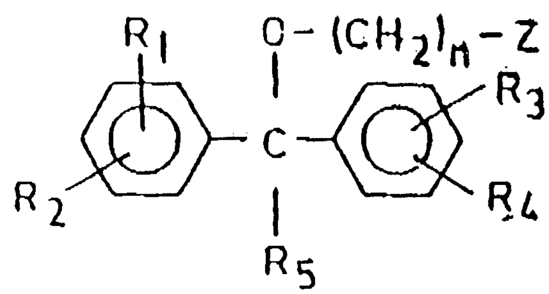

VI

or as optionally substituted 1-[ω-[bis-(phenyl)-alkoxy]-alk-ω-yl]-piperazines of the general formula

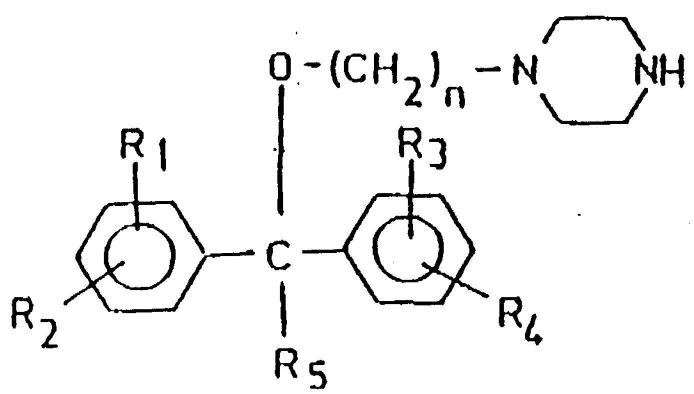

VIII

or as optionally substituted 1-[ω-[bis-(phenyl)-alkoxy]-[alkanoyl]-4-[alkenyl]-piperazines or -4-[alkinyl]-piperazines of the general formula

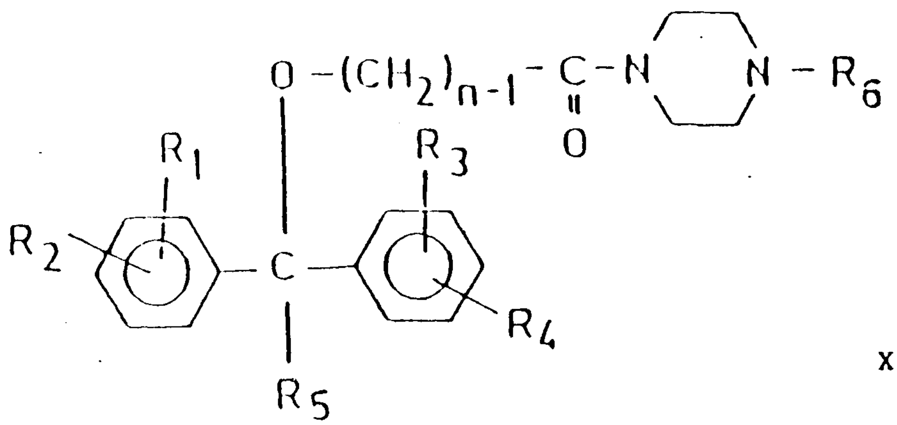

XI

those compounds are used in which $R_6$ is an alkenyl or alkinyl radical with 3 or 4 carbon atoms.

**6**. Process according to Claims 1 to 5, characterized in that as optionally substituted benzhydrols or their alcoholates of the general formula

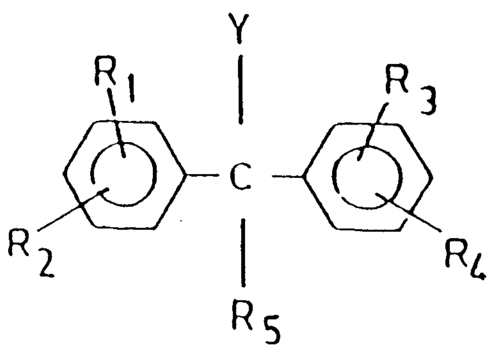

II

or as optionally substituted benzhydryl halides of the general formula

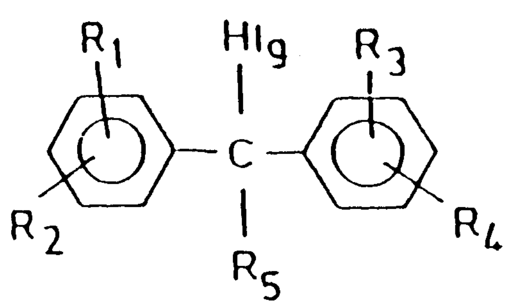

IV

or as optionally substituted diphenyl compounds of the general formula VI

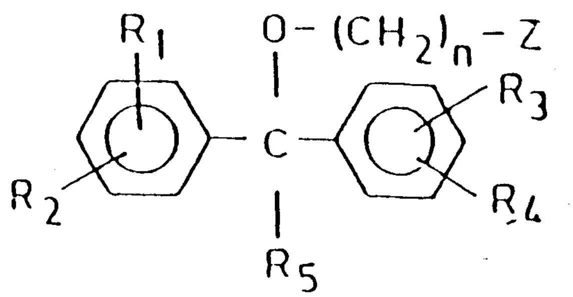

VI

or as optionally substituted 1-[ω-[bis-(phenyl)-alkoxy]-alk-ω-yl]piperazines of the general formula

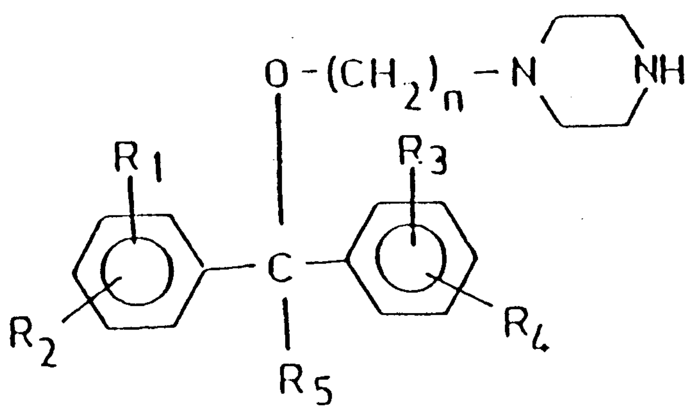

VIII

or as optionally substituted 1-[ω-[bis-(phenyl)-alkoxy]-[alkanoyl]-4-[alkenyl]-piperazines or -4-[alkinyl]-piperazines of the general formula

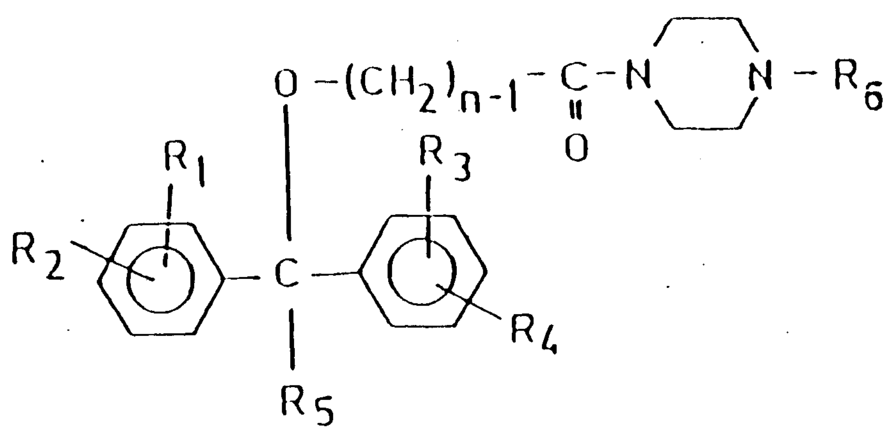

XI,

those compounds are used in which the phenyl $C_1$-$C_4$ alkoxyradical is a benzyloxy radical.

**7**. Process according to Claims 1 to 6, characterized in that the following compounds are prepared as optionally substituted 1-[ω-[bis-(phenyl)-alkoxy]-alkyl]-4-[alkenyl]-piperazines and -4-[alkinyl]-piperazines

**7**. 1-{2'-[bis-⟨4''-(fluoro)-phenyl⟩-methoxy]-ethyl}-4-{prop-2'''-enyl}-piperazine,

1-{2'-[⟨4''-(fluoro)-phenyl⟩-⟨phenyl⟩-methoxy]-ethyl}-4-{prop-2'''-enyl}-piperazine,

1-{2'-[⟨4''-(chloro)-phenyl⟩-⟨4'''-(fluoro)-phenyl⟩-methoxy]-ethyl}-4-{prop-2''''-enyl}-piperazine,

1-{2'-[⟨4''-(bromo)-phenyl⟩-⟨4'''-(fluoro)-phenyl⟩-methoxy]-ethyl}-4-{prop-2''''-enyl}-piperazine,

1-{2'-[bis-⟨4''-(Chloro)-phenyl⟩-methoxy]-ethyl}-4-{prop-2'''-enyl}-piperazine,

1-{2'-[⟨3'',4''-di-(chloro)-phenyl⟩-⟨phenyl⟩-methoxy]-ethyl}-4-{prop-2'''-enyl}-piperazine,

1-{2'-[1''-bis-⟨4'''-(fluoro)-phenyl⟩-ethoxy]-ethyl}-4-{prop-2''''-enyl}-piperazine,

1-{2'-[bis-⟨4''-(fluoro)-phenyl⟩-methoxy]-ethyl}-4-{prop-2'''-inyl}-piperazine,

1-{2'-[bis-⟨4''-fluoro)-phenyl⟩-methoxy]-ethyl}-4-{2'''-(methyl)prop-2'''-enyl}-piperazine,

1-{2'-[⟨3''-(nitro)-4''-(1'''-[prop-2''''-enyl]-piperazin-4'''-yl)-phenyl⟩-⟨phenyl⟩-methoxy]-ethyl}-4-{prop-2'''''-enyl}-piperazine,

1-{2'-[1''-⟨2''',5'''-di-(methyl)-phenyl⟩-1''-⟨phenyl⟩-propoxy]-ethyl}-4-{prop-2''''-enyl}-piperazine,

1-{2'-[⟨di-(phenyl)⟩-methoxy]-ethyl}-4-{prop-2''-enyl}-piperazine,

1-{2'-[⟨2''-(chloro)-phenyl⟩-⟨4'''-(fluoro)-phenyl⟩-methoxy]-ethyl}-4-{prop-2''''-enyl}-piperazine,

1-{2'-[⟨4''-(trifluoromethyl)-phenyl⟩-⟨4'''-(fluoro)-phenyl⟩-methoxy]-ethyl}-4-{prop-2''''-enyl}-piperazine,

1-{2'-[⟨2''-(methyl)-phenyl⟩-⟨4'''-(fluoro)-phenyl⟩-methoxy]-ethyl}-4-{prop-2''''-enyl}-piperazine,

1-{2'-[⟨3''-(ethoxy)-4''-(hydroxy)-phenyl⟩-⟨4'''-(fluoro)-phenyl⟩-methoxy]-ethyl}-4-{prop-2''''-enyl}-piperazine,

1-{2'-[⟨3''-(trifluoromethyl)-phenyl⟩-⟨4'''-(fluoro)-phenyl⟩-methoxy]-ethyl}-4-{prop-2''''-enyl}-piperazine,

1-{2'-[⟨3''-(trifluoromethyl)-phenyl⟩-⟨phenyl⟩-methoxy]-ethyl}-4-{prop-2'''-enyl}-piperazine,

1-[2'-[⟨4''-(benzyloxy)-phenyl⟩-⟨4'''-(fluoro)-phenyl⟩-methoxy]-ethyl}-4-{prop-2''''-enyl}-piperazine,

1-{2'-[⟨4''-(chloro)-phenyl⟩-⟨4'''-(fluoro)-phenyl⟩-methoxy]-ethyl}-4-{prop-2''''-inyl}-piperazine,

1-{2'-[⟨4''-(fluoro)-phenyl⟩-⟨phenyl⟩-methoxy]-ethyl}-4-{prop-2'''-inyl}-piperazine,

1-{2'-[⟨3'',4''-di-(chloro)-phenyl⟩-⟨phenyl⟩-methoxy]-ethyl}-4-{prop-2'''-inyl}-piperazine,

1-{2'-[⟨4''-(bromo)-phenyl⟩-⟨4'''-(fluoro)-phenyl⟩-methoxy]-ethyl}-4-{prop-2''''-inyl}-piperazine,

1-{2'-[⟨4''-(trifluoromethyl)-phenyl⟩-⟨4'''-(fluoro)-phenyl⟩-methoxy]-ethyl}-4-{prop-2''''-inyl}-piperazine,

1-{2'-[bis-⟨4''-(chloro)-phenyl⟩-methoxy]-ethyl}-4-{prop-2'''-inyl}-piperazine, and

1-{2'-[bis-⟨4''-(chloro)-phenyl⟩-methoxy]-ethyl}-4-{prop-2'''-enyl}-piperazine.

**8**. Process according to Claim 1, characterized in that optionally substituted 1-[ω-[bis-(phenyl)-alkoxy]-alkyl]-4-[alkinyl]-piperazines of the general formula I in which $R_1$, $R_2$, $R_3$, $R_4$ and $R_5$ as well as n have the meanings given in Claims 1 to 6 and $R_6$ denotes an alkinyl radical, are reduced to 1-[ω-[bis-(phenyl)-alkoxy]-alkyl]-4-[alkenyl]-piperazines of the general formula I in which $R_6$ denotes an alkenyl radical.

**9**. Process according to Claim 1, characterized in that 1-[ω-[bis-(phenyl)-alkoxy]-alkyl]-4-[alkenyl]-piperazines or -4-[alkinyl]-piperazines of the general formula Ia, substituted by one or more benzyloxy radicals

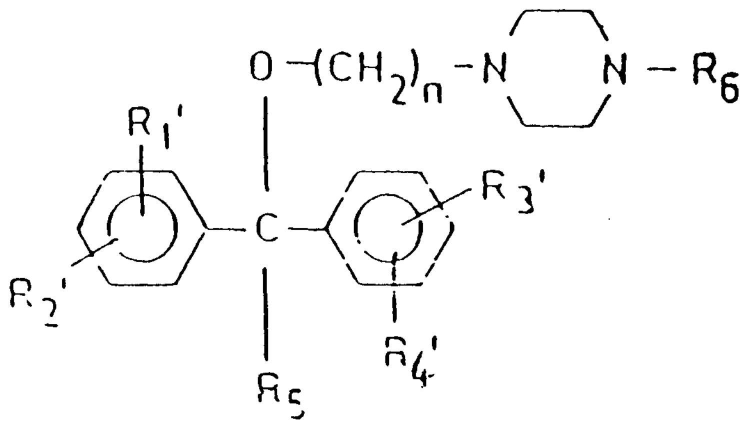

Ia

wherein

$R_5$, $R_6$ and n have the meanings given in Claims 1 to 6 and in at least one of

$R'_1$, $R'_2$, $R'_3$ and $R'_4$ the phenyl $C_1$-$C_4$ alkoxy radical is a benzyloxy radical, and the remaining others of

$R'_1$, $R'_2$, $R'_3$ and $R'_4$ have the meanings defined in Claims 1 to 6,

are reduced to those piperazines of the general formula I in which at least one of $R_1$, $R_2$, $R_3$ and $R_4$ denotes a hydroxy group.

**10**. Process according to Claim 1, characterised in that aluminium complexes are used as reducing agents.

**11**. Process according to one of the Claims 1 to 10, characterized in that the process is carried out in solvents inert with respect to the reactants.

**Revendications**

**Revendications pour les Etats contractants suivants : BE, CH, DE, FR, GB, IT, LI, LU, NL, SE**

**1.** 1-[ω-[bis-(phényl)-alcoxy-alcoyl]-4-[alcényl]-pipérazines et -4-[alcynyl]-pipérazines, éventuellement substituées, répondant à la formule générale I

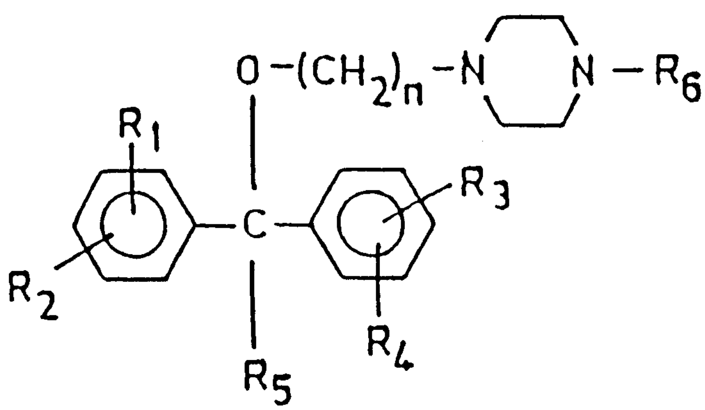

(I)

dans laquelle

$R_1$, $R_2$, $R_3$ et $R_4$ représentent, indépendamment les uns des autres, un atome d'hydrogène, un atome d'halogène, un reste trihalogénométhyle, un reste alcoyle en $C_1$-$C_4$, un reste alcoxy en $C_1$-$C_4$, un groupe nitro, un groupe hydroxy, un groupe 1-(prop-2'-ényl)-pipérazin-4-yle ou un reste phényl-alcoxy en $C_1$-$C_4$,

$R_5$ représente un atome d'hydrogène ou un reste alcoyle comportant 1 à 4 atomes de carbone,

$R_6$ représente un reste alcényle ou alcynyle comportant 3 à 6 atomes de carbone, et

n vaut 2 ou 3 ;

ainsi que leurs sels d'addition d'acides, des sels quaternaires et des isomères et solvates optiquement actifs.

2. Pipérazines selon la revendication 1, caractérisées en ce que le ou les atome(s) d'halogène et/ou les atomes d'halogène du ou des reste(s) trihalogénométhyle représenté(s) par $R_1$, $R_2$, $R_3$ et/ou $R_4$ est/sont du fluor, du chlore et/ou du brome.

3. Pipérazines selon la revendication 1 ou 2, caractérisées en ce que le ou les reste(s) alcoyle en $C_1$-$C_4$, le ou les reste(s) alcoxy en $C_1$-$C_4$ et/ou le motif alcoyle du ou des reste(s) phényl-alcoxy en $C_1$-$C_4$, représenté(s) par $R_1$, $R_2$, $R_3$ et/ou $R_4$ est/sont un ou des reste(s) en $C_1$ ou $C_2$.

4. Pipérazines selon l'une des revendications 1 à 3, caractérisées en ce que $R_5$ représente un reste alcoyle comportant 1 à 3 atomes de carbone.

5. Pipérazines selon l'une des revendications 1 à 4, caractérisées en ce que $R_6$ représente un reste alkényle ou alkinyle comportant 3 ou 4 atomes de carbone.

6. Pipérazines selon l'une des revendications 1 à 5, caractérisées en ce que le reste phényl-alcoxy en $C_1$-$C_4$ est un reste benzyloxy.

7. 1-{2'-[bis-⟨4''-(fluoro)-phényl⟩-méthoxy]-éthyl} -4-{prop-2'''-ényl}-pipérazine,
1-{2'-[⟨4''-(fluoro)-phényl⟩-⟨phényl⟩-méthoxy]-éthyl}-4-{prop-2'''-ényl}-pipérazine,
1-{2'-[⟨4''-(chloro)-phényl⟩-⟨4'''-(fluoro)-phényl⟩-méthoxy]-éthyl}-4-{prop-2'''-ényl}-pipérazine,
1-{2'-[⟨4''-bromo)-phényl⟩-⟨4'''-(fluoro)-phényl⟩-méthoxy]-éthyl}-4-{prop-2'''-ényl}-pipérazine,
1-{2'-[bis-⟨4''-(chloro)-phényl⟩-méthoxy]-éthyl} -4-{prop-2'''-ényl}-pipérazine,
1-{2'-[⟨3'',4''-di-(chloro)-phényl⟩-⟨phényl⟩-méthoxy]-éthyl}-4-{prop-2'''-ényl}-pipérazine,
1-{2'-[1''-bis-⟨4'''-(fluoro)-phényl⟩-éthoxy]-éthyl}-4-{prop-2'''-ényl}-pipérazine,
1-{2'-[bis-⟨4''-(fluoro)-phényl⟩-méthoxy]-éthyl} -4-{prop-2'''-inyl}-pipérazine,
1-{2'-[bis-⟨4''-(fluoro)-phényl⟩-méthoxy]-éthyl} -4-{2'''-(méthyl)-prop-2'''-ényl}-pipérazine,
1-{2'-[⟨3''-(nitro)-4''-(1'''-[prop-2''''-ényl]-pipérazin-4'''-yl)-phényl⟩-⟨phényl⟩-méthoxy]-éthyl}-4-{prop-2'''''-ényl)-pipérazine,
1-{2'-[1''-⟨2''',5'''-di-(méthyl)-phényl⟩-1''-⟨phényl⟩-propoxy]-éthyl}-4-{prop-2''''-ényl}-pipérazine,
1-{2'-[⟨di-(phényl)⟩-méthoxy]-éthyl}-4-{prop-2''-ényl}-pipérazine,
1-{2'-[⟨2''-(chloro)-phényl⟩-⟨4'''-(fluoro)-phényl⟩-méthoxy]-éthyl}-4-{prop-2''''-ényl}-pipérazine,
1-{2-[⟨4''-(trifluorométhyl)-phényl⟩-⟨4'''-(fluoro)-phényl⟩-méthoxy]-éthyl}-4-{prop-2''''-ényl}-pipérazine,
1-{2'-[⟨2''-(méthyl)-phényl⟩-⟨4'''-(fluoro)-phényl⟩-méthoxy]-éthyl}-4-{prop-2''''-ényl}-pipérazine,
1-{2'-[⟨3''-(éthoxy)-4''-(hydroxy)-phényl⟩-⟨4'''-(fluoro)-phényl⟩-méthoxy]-éthyl}-4-{prop-2''''-ényl}-pipérazine,
1-{2'-[⟨3''-(trifluorométhyl)-phényl⟩-⟨4'''-(fluoro)-phényl⟩-méthoxy]-éthyl}-4-{prop-2''''-ényl}-pipérazine,
1-{2'-[⟨3''-(trifluorométhyl)-phényl⟩-⟨phényl⟩-méthoxy]-éthyl}-4-{prop-2'''-ényl}-pipérazine,
1-{2'-[⟨4''-(benzyloxy)-phényl⟩-⟨4'''-(fluoro)-phényl⟩-méthoxy]-éthyl}-4-{prop-2''''-ényl}-pipérazine,
1-{2'-[⟨4''-(chloro)-phényl⟩-⟨4'''-(fluoro)-phényl⟩-méthoxy]-éthyl}-4-{prop-2''''-inyl}-pipérazine,
1-{2'-[⟨4''-(fluoro)-phényl⟩-⟨phényl⟩-méthoxy]-éthyl}-4-{prop-2'''-inyl}-pipérazine,
1-{2'-[⟨3'',4''-di-(chloro)-phényl⟩-⟨phényl⟩-méthoxy]-éthyl}-4-{prop-2'''-inyl}-pipérazine,
1-{2'-[⟨4''-(bromo)-phényl⟩-⟨4'''-(fluoro)-phényl⟩-méthoxy]-éthyl}-4-{prop-2''''-inyl}-pipérazine,
1-{2'-[⟨4''-(trifluorométhyl)-phényl⟩-⟨4'''-(fluoro)-phényl⟩-méthoxy]-éthyl}-4-{prop-2''''-inyl}-pipérazine,
1-{2'-[bis-⟨4''-(chloro)-phényl⟩-méthoxy]-éthyl} -4-{prop-2'''-inyl}-pipérazine, et
1-{2'-[bis-⟨4''-(chloro)-phényl⟩-méthoxy]-éthyl} -4-{prop-2'''-ényl}-pipérazine.,

8. Procédé de préparation de composés pipérazines selon l'une des revendications 1 à 7, caractérisé en ce que
a) l'on fait réagir des benzhydrols, éventuellement substitués, ou leurs alcoolates, répondant à la formule générale

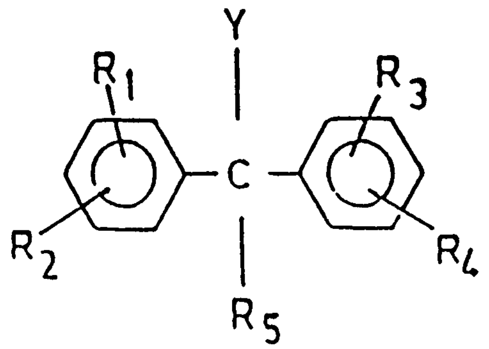

(II)

dans laquelle

| | |
|---|---|
| $R_1$, $R_2$, $R_3$, $R_4$ et $R_5$ | ont les significations indiquées dans les revendications 1 à 6, et |
| Y | représente un groupe hydroxy ou un reste répondant à la formule<br>-OM<br>dans laquelle M représente un atome de métal alcalin ou un reste répondant à la formule<br>-MgHlg<br>dans laquelle Hlg représente un atome d'halogène, |

avec un composé pipérazine répondant à la formule générale

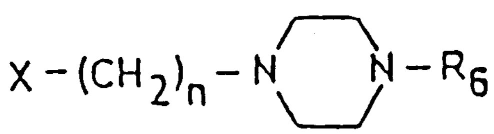

(III)

dans laquelle

| | |
|---|---|
| $R_6$ et n | ont les significations indiquées dans la revendication 1 ou 5, et |
| X | représente un atome d'halogène, un groupe hydroxy, un reste alcoylsulfonyloxy ou un reste arylsulfonyloxy,<br>à condition que<br>X soit différent d'un groupe hydroxy quand<br>Y représente un reste répondant à la formule générale -OM, |

ou

b) l'on fait réagir des halogénures de benzhydryle, éventuellement substitués, répondant à la formule générale

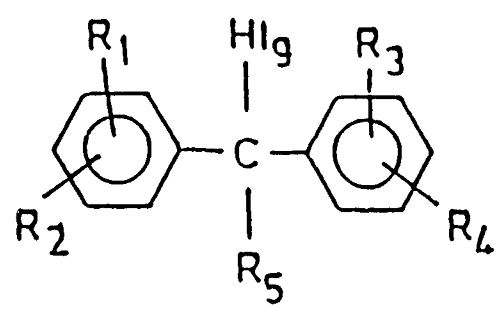

(IV)

dans laquelle

| | |
|---|---|
| $R_1$, $R_3$, $R_3$, $R_4$ et $R_5$ | ont les significations indiquées dans les revendications 1 à 6, et |
| Hlg | a la signification ci-dessus, |

avec un composé pipérazine, ou un alcoolate d'un tel composé, répondant à la formule générale

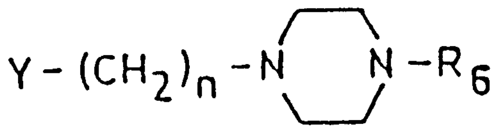

(V)

dans laquelle

$R_6$ et n ont les significations indiquées dans la revendication 1 ou 5, et

Y représente un groupe hydroxy ou un reste oxy-métal répondant à la formule générale

-OM'

dans laquelle M' représente un atome de métal alcalin,

ou

c) l'on fait réagir des composés diphényliques, éventuellement substitués, répondant à la formule générale VI

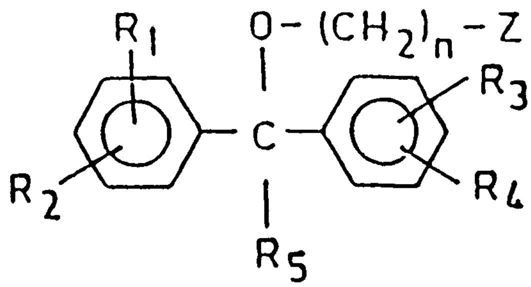

(VI)

dans laquelle

$R_1$, $R_2$, $R_3$, $R_4$ et $R_5$ ainsi que n ont les significations indiquées dans les revendications 1 et 6, et

Z représente un atome d'halogène ou un reste alcoyl- ou arylsulfonyloxy,

avec des 1-(alcényl)- ou 1-(alcynyl)-pipérazines répondant à la formule générale

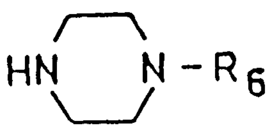

(VII)

dans laquelle

$R_6$ a les significations dans la revendication 1 ou 5,

ou

d) l'on fait réagir des 1-{ω-[bis-(phényl)-alcoxy])alk-ω-yl}-pipérazines, éventuellement substituées, répondant à la formule générale

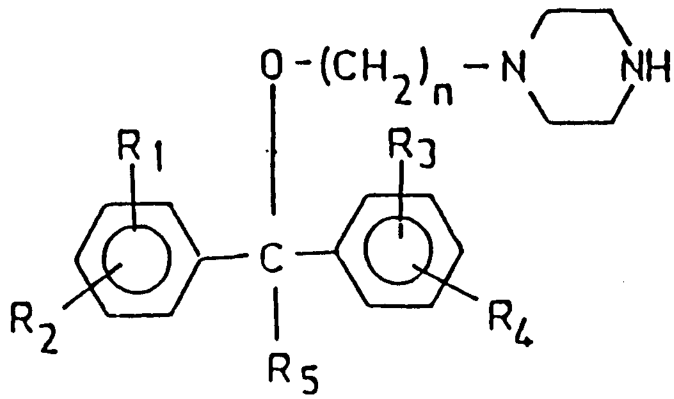

(VIII)

dans laquelle

$R_1$, $R_2$, $R_3$, $R_4$ et $R_5$ ainsi que n ont les significations indiquées dans les revendications 1 à 6, avec un composé répondant à la formule générale

Z - $R_6$ (IX)

dans laquelle

$R_6$ a les significations indiquées dans la revendication 1 ou 5, et

Z a la signification ci-dessus,

ou

e) l'on réduit des 1-{ω-[bis-(phényl)-alcoxy]-alcanoyl}-4-{alcényl}-pipérazines ou -4-{alcynyl}-pipérazines, éventuellement substituées, répondant à la formule générale

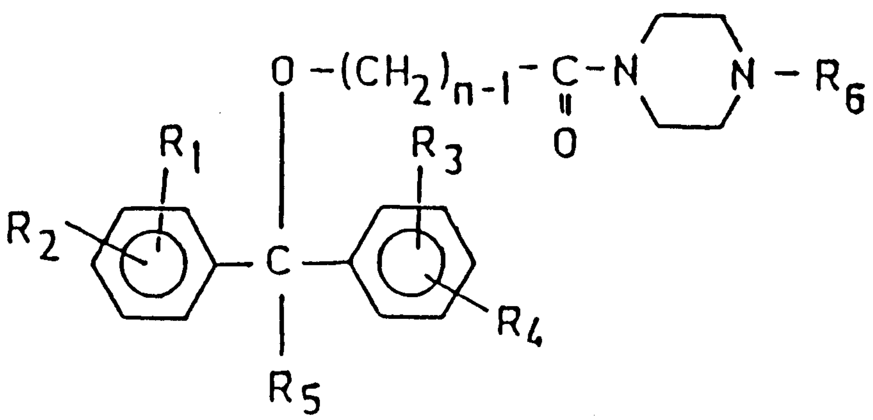

(XI)

dans laquelle

$R_1$, $R_2$, $R_3$, $R_4$ et $R_5$ ainsi que n ont les significations indiquées dans les revendications 1 à 6,

$R_6$ représente un reste alcényle ou alcynyle comportant 3 à 6 atomes de carbone,

et, d'une manière connue en soi, éventuellement on transforme les 1-{ω-[bis-(phényl)-alcoxy]-alcoyl}-4-{alcényl}-pipérazines ou -{alcynyl}-pipérazines répondant à la formule générale I, obtenues d'après une des variantes a) à e), en sels d'addition d'acides avec des acides inorganiques ou organiques, ou en sels quaternaires avec des agents de quaternisation, et/ou on transforme les sels d'addition d'acides ou les sels quaternaires obtenus des 1-{ω-[bis-(phényl)-alcoxy]-alcoyl}-4-{alcényl}-pipérazines ou -{alcynyl}-pipérazines répondant à la formule générale I en les bases 1-{ω-[bis-(phényl)-alcoxy]-alcoyl}-4-{alcényl}-pipérazines ou -{alcynyl}-pipérazines libres correspondantes, répondant à la formule générale I, ou en d'autres sels d'addition d'acides et/ou sels quaternaires, et/ou éventuellement on réalise un clivage des pipérazines racémiques obtenues, répondant à la formule générale I, ou de leurs sels d'addition d'acides ou de leurs sels quaternaires, ou on réalise une racémisation des composés optiquement actifs correspondants, pour les transformer en stéréoisomères.

**9.** Procédé selon la revendication 8, caractérisé en ce qu'on réduit des 1-{ω-[bis-(phényl)-alcoxy]-alcoyl}-4-{alcynyl}-pipérazines répondant à la formule générale I, dans lesquelles $R_1$, $R_2$, $R_3$, $R_4$, $R_5$ et n ont les significations indiquées dans les revendications 1 à 6 et $R_6$ représente un reste alcynyle, en 1-{ω-[bis-(phényl)-alcoxy]-alcoyl}-4-{alcényl}-pipérazines répondant à la formule générale I, dans lesquelles $R_6$ représente un reste alcényle.

**10.** Procédé selon la revendication 8, caractérisé en ce qu'on réduit des 1-{ω-[bis-(phényl)-alcoxy]-alcoyl}-4-{alcényl}-pipérazines ou -4-{alcynyl}-pipérazines, substituées par 1 ou un plusieurs restes benzyloxy et répondant à la formule générale

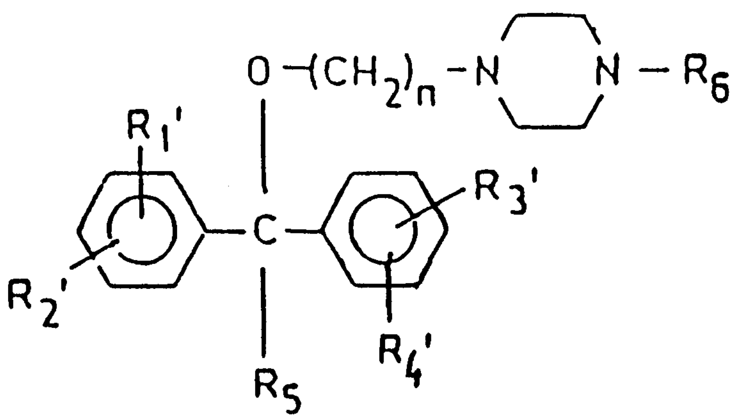

(Ia)

dans laquelle

$R_5$, $R_6$ et n — ont les significations indiquées dans les revendications 1 à 6, et

au moins un des restes $R_1'$, $R_2'$, $R_3'$ et $R_4'$ — représente le reste phényl-alcoxy en $C_1$-$C_4$

et les autres restes éventuels $R_1'$, $R_2'$, $R_3'$ et $R_4'$ — ont les significations indiquées dans les revendications 1 à 6,

en pipérazines de formule générale I, dans lesquelles au moins un des restes $R_1$, $R_2$, $R_3$ et $R_4$ représente un groupe hydroxy.

**11.** Procédé selon la revendication 8, caractérisé en ce qu'on utilise des complexes d'aluminium comme agent de réduction.

**12.** Procédé selon l'une des revendications 8 à 11, caractérisé en ce qu'on travaille avec des solvants inertes vis-à-vis des partenaires réactionnels.

**13.** Médicaments, caractérisés en ce qu'ils contiennent un ou plusieurs composés selon l'une des revendications 1 à 7, sous forme de substance(s) active(s), éventuellement avec un ou plusieurs excipients et/ou adjuvants pharmaceutiques usuels.

**14.** Utilisation de 1-{ω-[bis-(phényl)-alcoxy)-alcoyl}-4-{alcoyl}-pipérazines et -4-{acyl}-pipérazines répondant à la formule générale

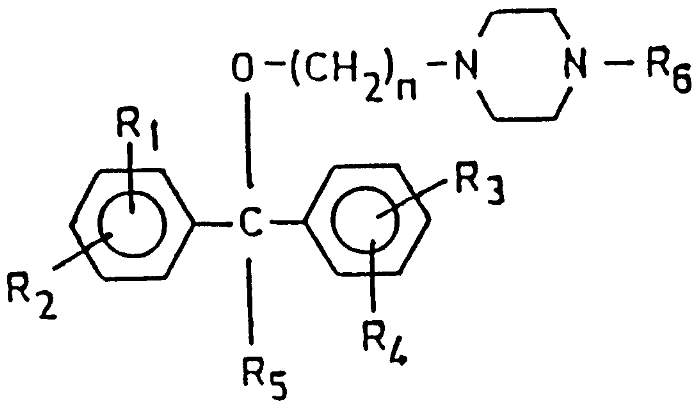

(I)

dans laquelle

| | |
|---|---|
| $R_1$ | représente un atome d'hydrogène ou un atome d'halogène en position ortho ou para, |
| $R_2$, $R_3$, $R_4$ et $R_5$ | représentent un atome d'hydrogène ou un reste alcoyle comportant 3 à 6 atomes de carbone, ou un reste acyle répondant à la formule générale 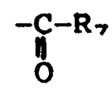 dans laquelle $R_7$ représente un reste alcoyle comportant 2 à 5 atomes de carbone, et |
| n | vaut 2 ou 3, |

pour préparer des médicaments ayant une action dopaminergique sur le système nerveux central.

**Revendications pour l'Etat contractant suivant : AT**

**1.** Procédé de préparation de 1-[ω-[bis-(phényl)-alcoxy-alcoyl]-4-[alcényl]-pipérazines et -4-[alcynyl]-pipérazines, éventuellement substituées, répondant à la formule générale I

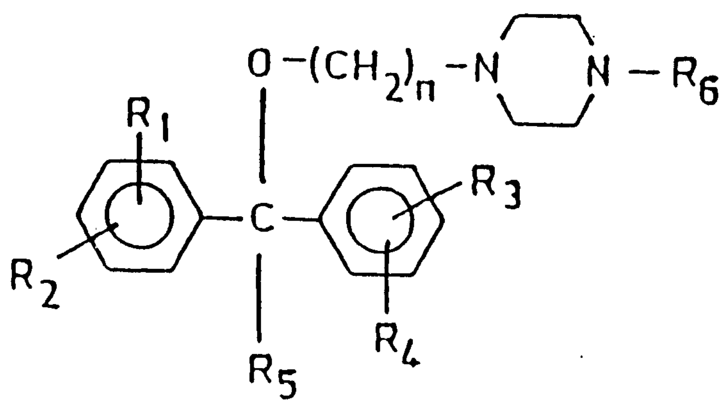

(I)

dans laquelle

| | |
|---|---|
| $R_1$, $R_2$, $R_3$ et $R_4$ | représentent, indépendamment les uns des autres, un atome d'hydrogène, un atome d'halogène, un reste trihalogénométhyle, un reste alcoyle en $C_1$-$C_4$, un reste alcoxy en $C_1$-$C_4$, un groupe nitro, un groupe hydroxy, un groupe 1-(prop-2'-ényl)-pipérazin-4-yle ou un reste phényl-alcoxy en $C_1$-$C_4$, |
| $R_5$ | représente un atome d'hydrogène ou un reste alcoyle comportant 1 à 4 atomes de carbone, |
| $R_6$ | représente un reste alcényle ou alcynyle comportant 3 à 6 atomes de carbone, et |

n vaut 2 ou 3 ;

ainsi que leurs sels d'addition d'acides, des sels quaternaires et des isomères et solvates optiquement actifs, caractérisé en ce que

a) l'on fait réagir des benzhydrols, éventuellement substitués, ou leurs alcoolates, répondant à la formule générale

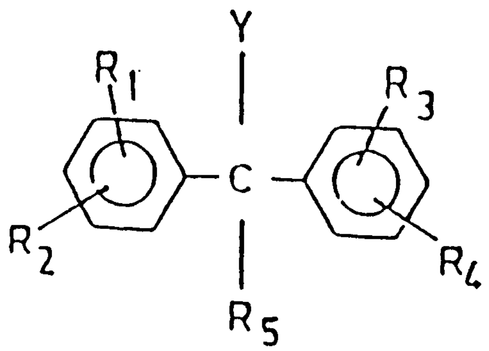

(II)

dans laquelle

$R_1$, $R_2$, $R_3$, $R_4$ et $R_5$ ont les significations indiquées dans les revendications 1 à 6, et

Y représente un groupe hydroxy ou un reste répondant à la formule

-OM

dans laquelle M représente un atome de métal alcalin ou un reste répondant à la formule

-MgHlg

dans laquelle Hlg représente un atome d'halogène,

avec un composé pipérazine répondant à la formule générale

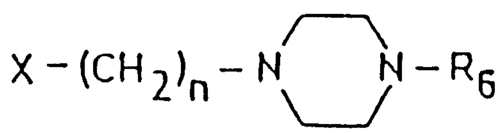

(III)

dans laquelle

$R_6$ et n ont les significations indiquées dans la revendication 1 ou 5, et

X représente un atome d'halogène, un groupe hydroxy, un reste alcoylsulfonyloxy ou un reste arylsulfonyloxy,

à condition que

X soit différent d'un groupe hydroxy quand

Y représente un reste répondant à la formule générale -OM,

ou

b) l'on fait réagir des halogénures de benzhydryle, éventuellement substitués, répondant à la formule générale

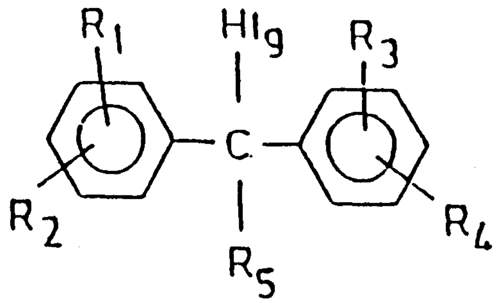

(IV)

dans laquelle

$R_1$, $R_2$, $R_3$, $R_4$ et $R_5$ ont les significations indiquées dans les revendications 1 à 6, et

Hlg a la signification ci-dessus,

avec un composé pipérazine, ou un alcoolate d'un tel composé, répondant à la formule générale

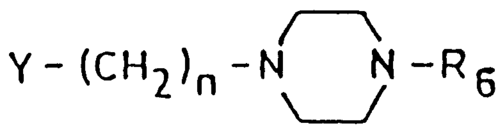

(V)

dans laquelle

$R_6$ et n ont les significations indiquées dans la revendication 1 ou 5, et

Y représente un groupe hydroxy ou un reste oxy-métal répondant à la formule générale

-OM'

dans laquelle M' représente un atome de métal alcalin ; ou

c) l'on fait réagir des composés diphényliques, éventuellement substitués, répondant à la formule générale VI

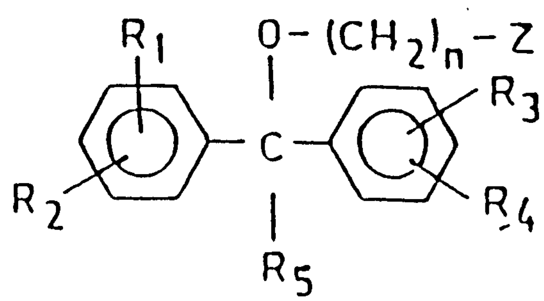

(VI)

dans laquelle

$R_1$, $R_2$, $R_3$, $R_4$ et $R_5$ ainsi que n ont les significations indiquées dans les revendications 1 et 6, et

Z représente un atome d'halogène ou un reste alcoyl- ou arylsulfonyloxy,

avec des 1-(alcényl)- ou 1-(alcynyl)-pipérazines répondant à la formule générale

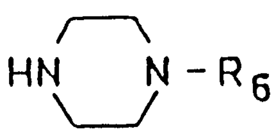

(VII)

dans laquelle

$R_6$ a les significations dans la revendication 1 ou 5 ; ou

d) l'on fait réagir des 1-{ω-[bis-(phényl)-alcoxy])alk-ω-yl}-pipérazines, éventuellement substituées, répondant à la formule générale

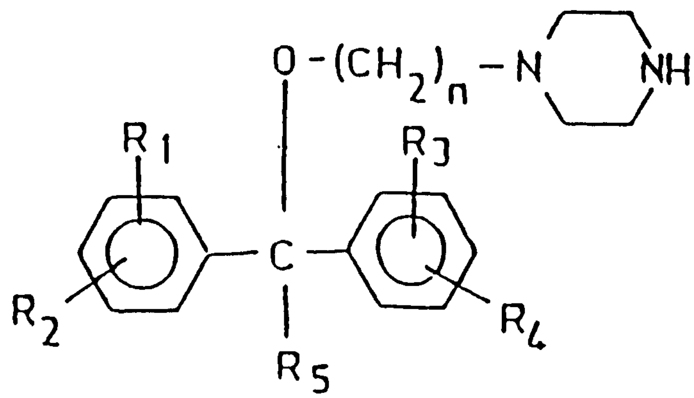

(VIII)

dans laquelle

$R_1$, $R_2$, $R_3$, $R_4$ et $R_5$ ainsi que n ont les significations indiquées dans les revendications 1 à 6,

avec un composé répondant à la formule générale

Z - $R_6$ (IX)

dans laquelle

$R_6$ a les significations indiquées dans la revendication 1 ou 5, et

Z a la signification ci-dessus,

ou

e) l'on réduit des 1-{ω-[bis-(phényl)-alcoxy]-alcanoyl}-4-{alcényl}-pipérazines ou -4-{alcynyl}-pipérazines, éventuellement substituées, répondant à la formule générale

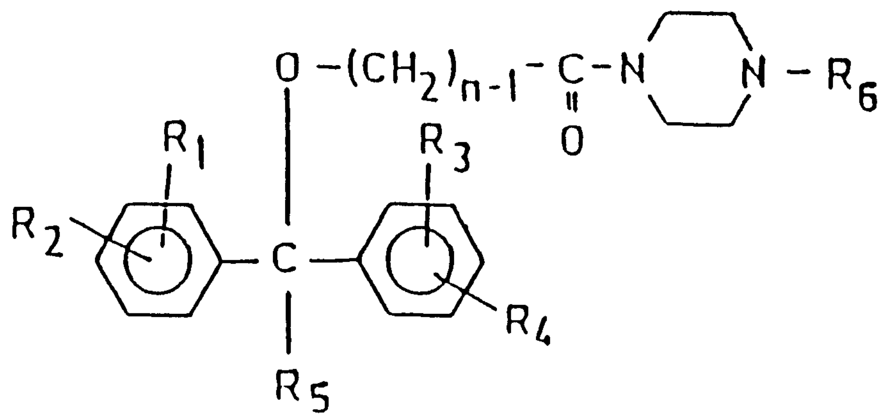

(XI)

dans laquelle

$R_1$, $R_2$, $R_3$, $R_4$ et $R_5$ ainsi que n ont les significations indiquées dans les revendications 1 à 6,

$R_6$ représente un reste alcényle ou alcynyle comportant 3 à 6 atomes de carbone,

et, d'une manière connue en soi, éventuellement on transforme les 1-{ω-[bis-(phényl)-alcoxy]-alcoyl}-4-{alcényl}-pipérazines ou -{alcynyl}-pipérazines répondant à la formule générale I, obtenues d'après une des variantes a) à e), en sels d'addition d'acides avec des acides inorganiques ou organiques, ou en sels quaternaires avec des agents de quaternisation, et/ou on transforme les sels d'addition d'acides ou les sels quaternaires obtenus des 1-{ω-[bis-(phényl)-alcoxy]-alcoyl}-4-{alcényl}-pipérazines ou -{alcynyl}-pipérazines répondant à la formule générale I en les bases 1-{ω-[bis-(phényl)-alcoxy]-alcoyl}-4-{alcényl}-pipérazines ou -{alcynyl}-pipérazines libres correspondantes, répondant à la formule générale I, ou en d'autres sels d'addition d'acides et/ou sels quaternaires, et/ou éventuellement on réalise un clivage des pipérazines racémiques obtenues, répondant à la formule générale I, ou de leurs sels d'addition d'acides ou de leurs sels quaternaires, ou on réalise une racémisation des composés optiquement actifs correspondants, pour les transformer en stéréoisomères.

**2.** Procédé selon la revendication 1, caractérisé en ce qu'on utilise, comme benzhydrols ou leurs alcoolates, éventuellement substitués, répondant à la formule générale

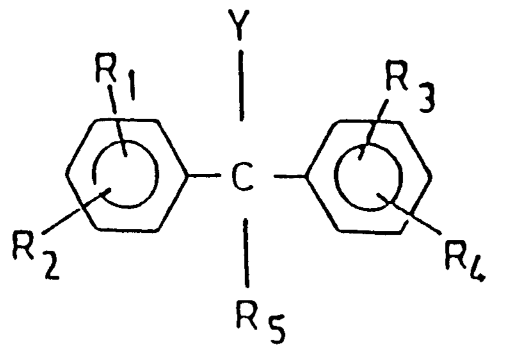

(II)

ou, comme halogénures de benzhydryle éventuellement substitués, répondant à la formule générale

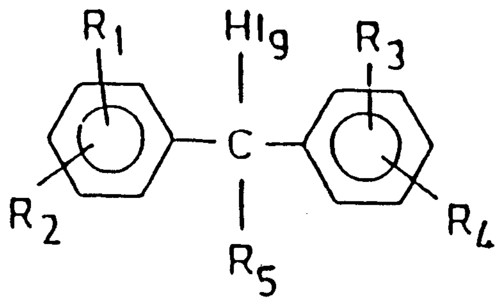

(IV)

ou, comme composés diphényliques éventuellement substitués, répondant à la formule générale VI

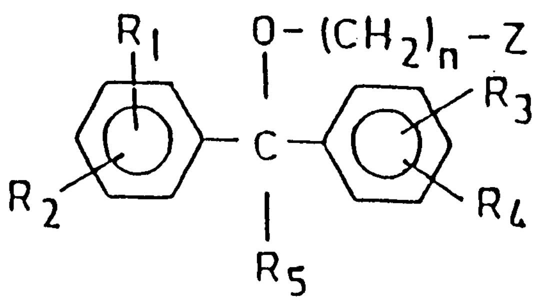

(VI)

ou, comme 1-{ω-[bis-(phényl)-alcoxy]-alk-ω-yl}-pipérazines éventuellement substituées, répondant à la formule générale

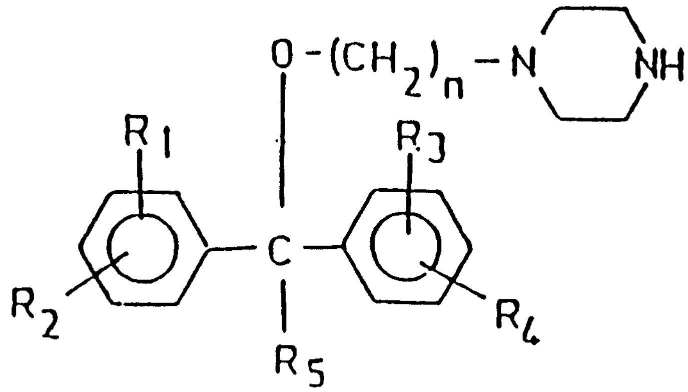

(VIII)

ou, comme 1-{ω-[bis-(phényl)-alcoxy]-alcanoyl}-4-{alcényl}-pipérazines ou -4-{alcynyl}-pipérazines éventuellement substituées, répondant à la formule générale

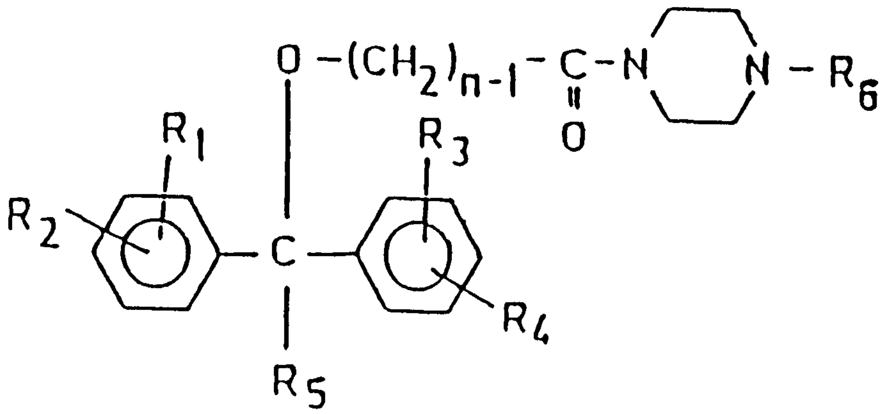

(XI)

ceux/celles dans lesquel(le)s le ou les atomes d'halogène et/ou les atomes d'halogène du ou des restes trihalogénométhyle, représenté(s) par $R_1$, $R_2$, $R_3$ et/ou $R_4$, est/sont du fluor, chlore et/ou brome.

**3.** Procédé selon la revendication 1 ou 2, caractérisé en ce qu'on utilise comme benzhydrols ou leurs alcoolates, éventuellement substitués, répondant à la formule générale

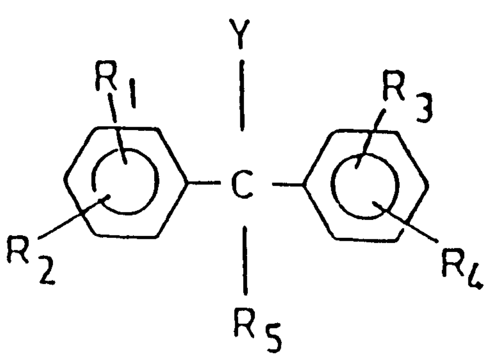

(II)

ou, comme halogénures de benzhydryle éventuellement substitués, répondant à la formule générale

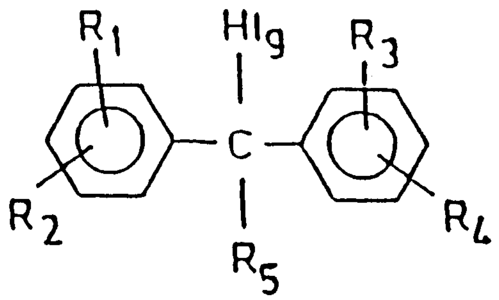

(IV)

ou, comme composés diphényliques éventuellement substitués, répondant à la formule générale VI

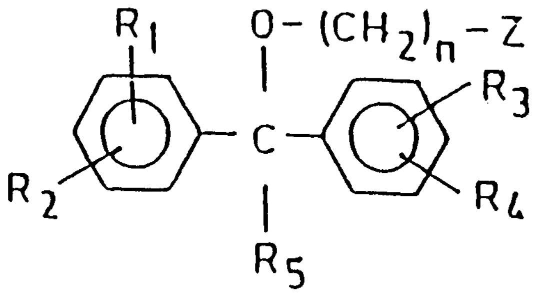

(VI)

ou, comme 1-{ω-[bis-(phényl)-alcoxy]-alk-ω-yl}-pipérazines éventuellement substituées, répondant à la formule générale

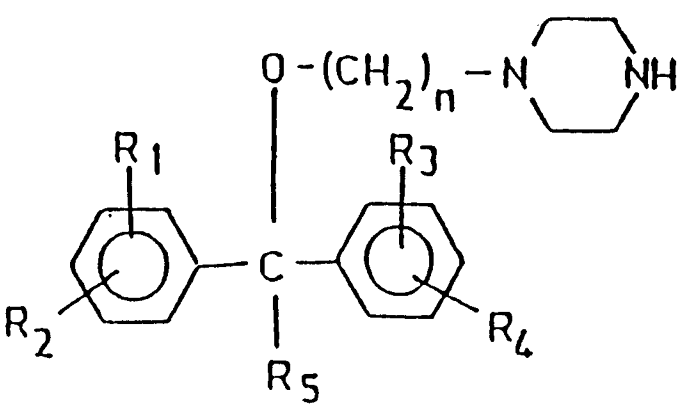

(VIII)

ou, comme 1-{ω-[bis-(phényl)-alcoxy]-alcanoyl}-4-{alcényl}-pipérazines ou -4-{alcynyl}-pipérazines éventuellement substituées, répondant à la formule générale

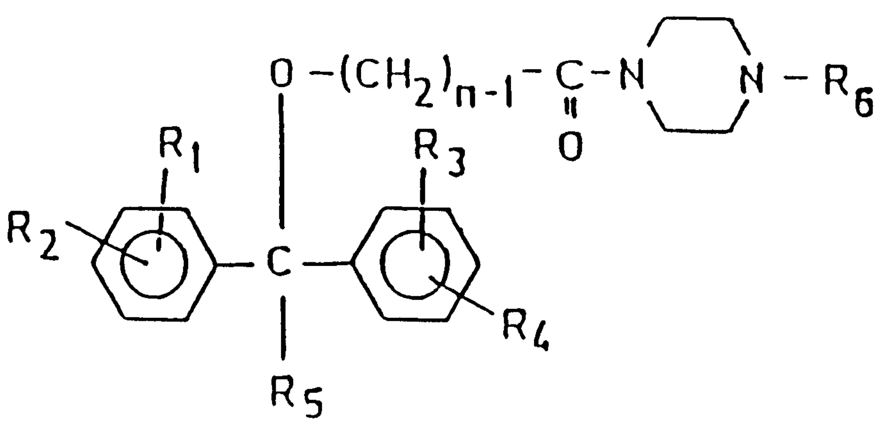

(XI)

ceux/celles dans lesquel(le)s le ou les restes alcoyle en $C_1$-$C_4$, le ou les restes alcoxy en $C_1$-$C_4$ et/ou le motif alcoyle du/ des restes phényl-alcoxy en $C_1$-$C_4$, représenté(s) par $R_1$, $R_2$, $R_3$, et/ou $R_4$, est/sont un ou des restes en $C_1$ ou en $C_2$.

4. Procédé selon l'une des revendications 1 à 3, caractérisé en qu'on utilise comme benzhydrols ou leurs alcoolates, éventuellement substitués, répondant à la formule générale

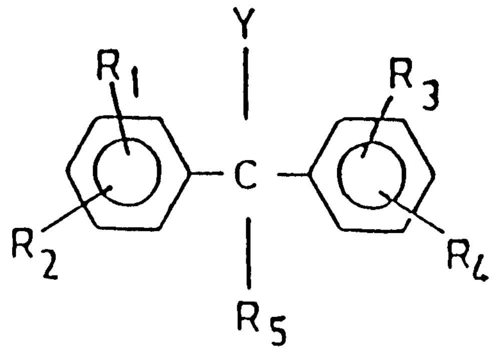

(II)

ou, comme halogénures de benzhydryle éventuellement substitués, répondant à la formule générale

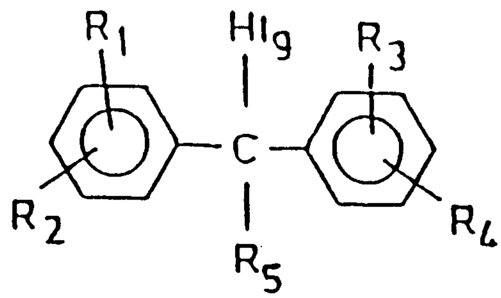

(IV)

ou, comme composés diphényliques éventuellement substitués, répondant à la formule générale VI

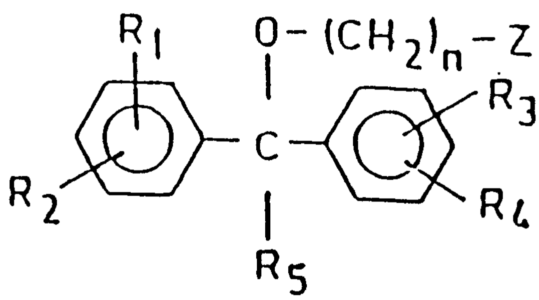

(VI)

ou, comme 1-{ω-[bis-(phényl)-alcoxy]-alk-ω-yl}-pipérazines éventuellement substituées, répondant à la formule générale

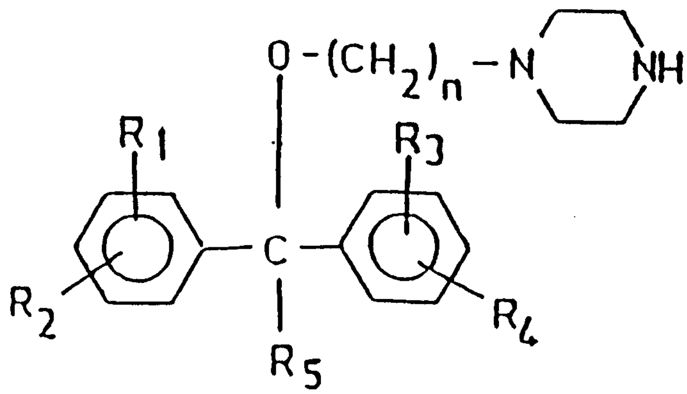

(VIII)

ou, comme 1-{ω-[bis-(phényl)-alcoxy]-alcanoyl}-4-{alcényl}-pipérazines ou -4-{alcynyl}-pipérazines éventuellement substituées, répondant à la formule générale

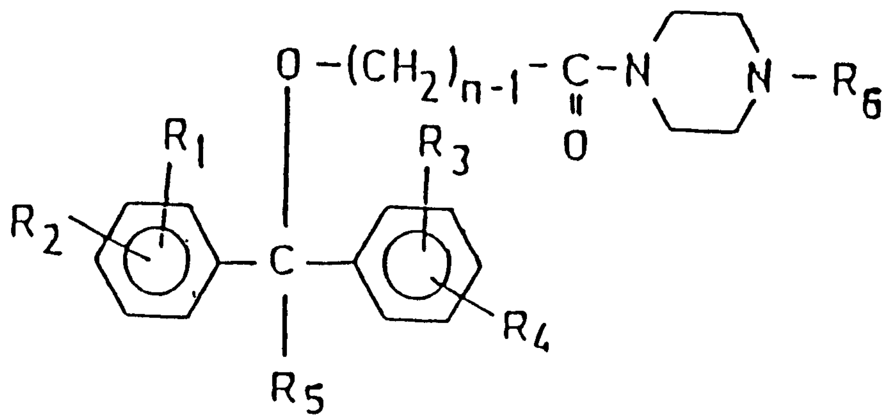

(XI)

ceux/celles dans lesquel(le)s $R_5$ représente un reste alcoyle comportant 1 à 3 atomes de carbone.

**5.** Procédé selon l'une des revendications 1 à 4, caractérisé en ce qu on utilise comme benzhydrols ou leurs alcoolates, éventuellement substitués, répondant à la formule générale

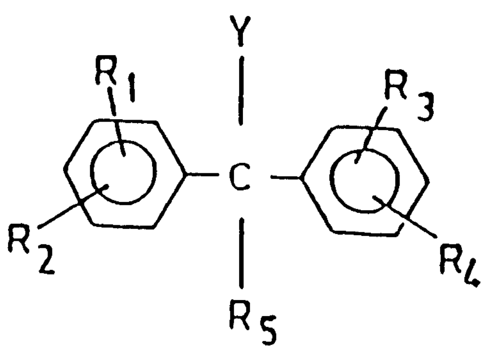

(II)

ou, comme halogénures de benzhydryle éventuellement substitués, répondant à la formule générale

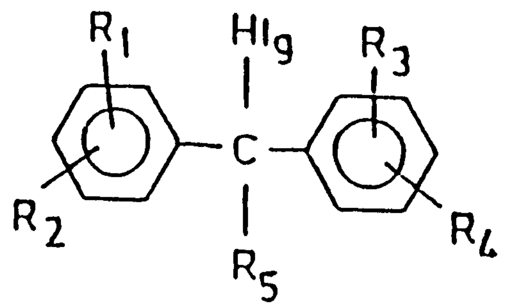

(IV)

ou, comme composés diphényliques éventuellement substitués, répondant à la formule générale VI

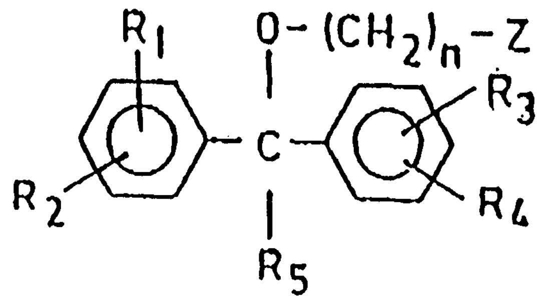

(VI)

ou, comme 1-{ω-[bis-(phényl)-alcoxy]-alk-ω-yl}-pipérazines éventuellement substituées, répondant à la formule générale

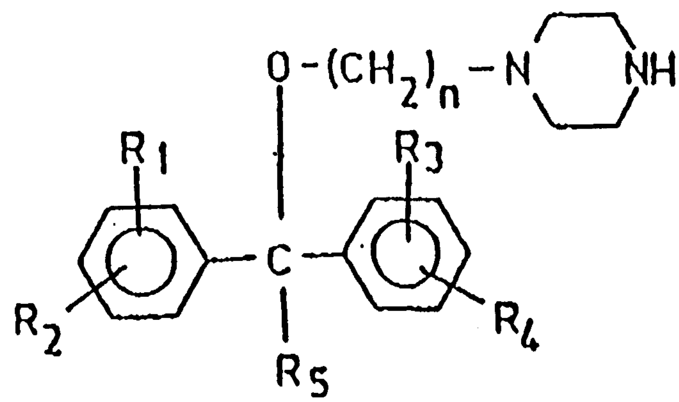

(VIII)

ou, comme 1-{ω-[bis-(phényl)-alcoxy]-alcanoyl}-4-{alcényl}-pipérazines ou -4-{alcynyl}-pipérazines éventuellement substituées, répondant à la formule générale

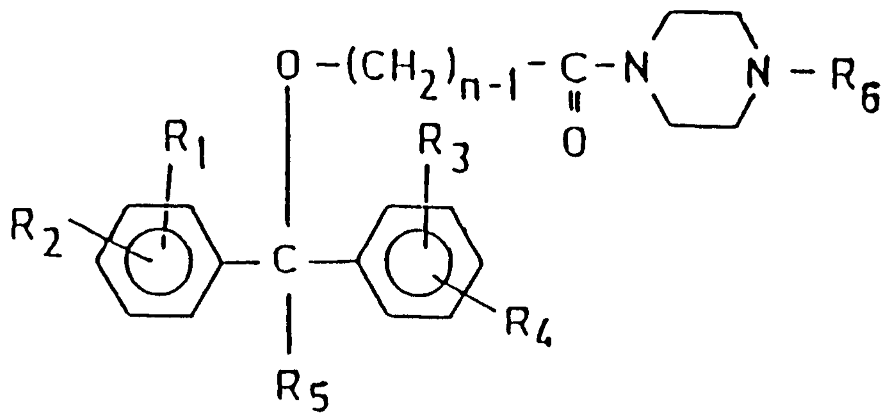

(XI)

ceux/celles dans lesquel(le)s $R_6$ représente un reste alcényle ou alcynyle comportant 3 à 4 atomes de carbone.

6. Procédé selon l'une des revendications 1 à 5, caractérisé en ce qu'on utilise comme benzhydrols ou leurs alcoolates, éventuellement substitués, répondant à la formule générale

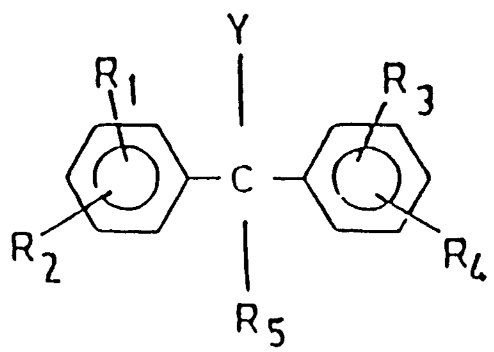

(II)

ou, comme halogénures de benzhydryle éventuellement substitués, répondant à la formule générale

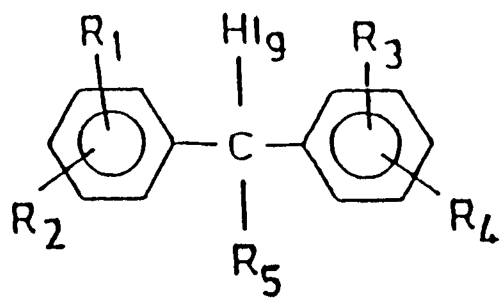

(IV)

ou, comme composés diphényliques éventuellement substitués, répondant à la formule générale VI

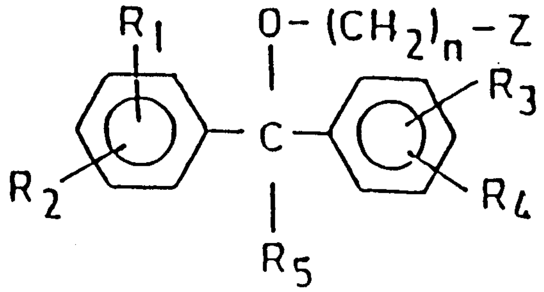

(VI)

ou, comme 1-{ω-[bis-(phényl)-alcoxy]-alk-ω-yl}-pipérazines éventuellement substituées, répondant à la formule générale

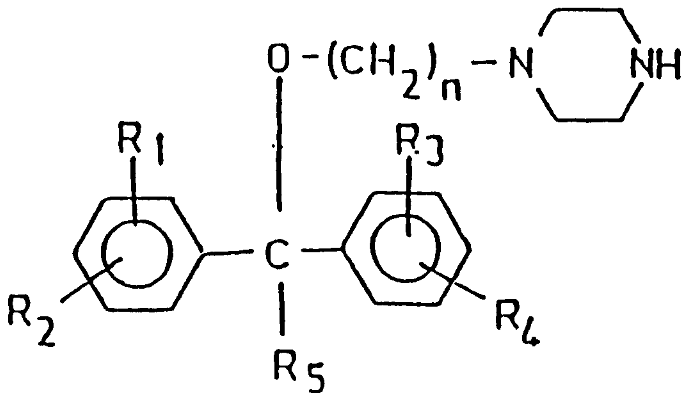

(VIII)

ou, comme 1-{ω-[bis-(phényl)-alcoxy]-alcanoyl}-4-{alcényl}-pipérazines ou -4-{alcynyl}-pipérazines éventuellement substituées, répondant à la formule générale

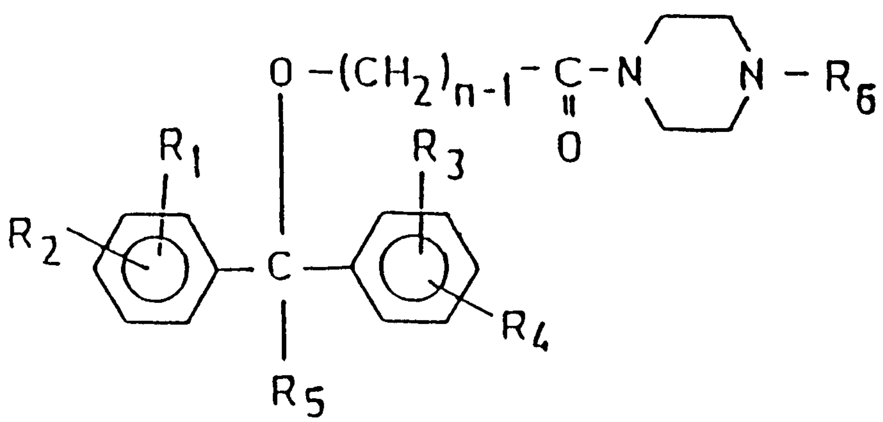

(XI)

ceux/celles dans lesquel(le)s le reste phényl-alcoxy en $C_1$-$C_4$ est un reste benzyloxy.

**7.** Procédé selon l'une des revendications 1 à 6, caractérisé en ce qu'on prépare, comme 1-{ω-[bis-(phényl)-alcoxy]-alcoyl}-4-{alcényl}-pipérazines ou -4-{alcynyl}-pipérazines, les composés suivants :

1-{2'-[bis-⟨4''-(fluoro)-phényl⟩-méthoxy]-éthyl} -4-{prop2'''-ényl}-pipérazine,
1-{2'-[⟨4''-(fluoro)-phényl⟩-⟨phényl⟩-méthoxy]-éthyl}-4-{prop-2'''-ényl}-pipérazine,
1-{2'-[⟨4''-(chloro)-phényl⟩-⟨4'''-(fluoro)-phényl⟩-méthoxy]-éthyl}-4-{prop-2'''-ényl}-pipérazine,
1-{2'-[⟨4''-bromo)-phényl⟩-⟨4'''-(fluoro)-phényl⟩-méthoxy]-éthyl}-4-{prop-2'''-ényl}-pipérazine,
1-{2'-[bis-⟨4''-(chloro)-phényl⟩-méthoxy]-éthyl}-4-{prop-2'''-ényl}-pipérazine,
1-{2'-[⟨3'',4,,-di-(chloro)-phényl⟩-⟨phényl⟩-méthoxy]-éthyl}-4-{prop-2'''-ényl}-pipérazine,

1-{2'-[1''-bis-(4'''-(fluoro)-phényl)-éthoxy]-éthyl}-4-{prop-2'''-ényl}-pipérazine,
1-{2'-[bis-(4''-(fluoro)-phényl)-méthoxy]-éthyl} -4-{prop-2'''-inyl}-pipérazine,
1-{2'-[bis-(4''-(fluoro)-phényl)-méthoxy]-éthyl} -4-{2'''-(méthyl)-prop-2'''-ényl}-pipérazine,
1-{2'-[(3''-(nitro)-4''-(1'''-[prop-2''''-ényl]-pipérazin-4'''-yl)-phényl)-(phényl)-méthoxy]-éthyl}-4-{prop-2'''''-ényl}-pipérazine,
1-{2'-[1''-(2''',5'''-di-(méthyl)-phényl)-1''-(phényl)-propoxy]-éthyl}-4-{prop-2''''-ényl}-pipérazine,
1-{2'-[(di-(phényl))-méthoxy]-éthyl}-4-{prop-2''-ényl}-pipérazine,
1-{2'-[(2''-(chloro)-phényl)-(4'''-(fluoro)-phényl)-méthoxy]-éthyl}-4-{prop-2''''-ényl}-pipérazine,
1-{2-[(4''-(trifluorométhyl)-phényl)-(4'''-(fluoro)-phényl)-méthoxy]-éthyl}-4-{prop-2''''-ényl}-pipérazine,
1-{2'-[(2''-(méthyl)-phényl)-(4'''-(fluoro)-phényl)-méthoxy]-éthyl}-4-{prop-2''''-ényl}-pipérazine,
1-{2'-[(3''-(éthoxy)-4''-(hydroxy)-phényl)-(4'''-(fluoro)-phényl)-méthoxy]-éthyl}-4-{prop-2''''-ényl}-pipérazine,
1-{2'-[(3''-(trifluorométhyl)-phényl)-(4'''-(fluoro)-phényl)-méthoxy]-éthyl}-4-{prop-2''''-ényl}-pipérazine,
1-{2'-[(3''-(trifluorométhyl)-phényl)-(phényl)-méthoxy]-éthyl}-4-{prop-2'''-ényl}-pipérazine,
1-{2'-[(4''-(benzyloxy)-phényl)-(4'''-(fluoro)-phényl)-méthoxy]-éthyl}-4){prop-2''''-ényl}-pipérazine,
1-{2'-[(4''-(chloro)-phényl)-(4'''-(fluoro)-phényl)-méthoxy]-éthyl}-4-{prop-2''''-inyl}-pipérazine,
1-{2'-[(4''-(fluoro)-phényl)-(phényl)-méthoxy]-éthyl}-4-{prop-2'''-inyl}-pipérazine,
1-{2'-[(3'',4''-di-(chloro)-phényl)-(phényl)-méthoxy]-éthyl}-4-{prop-2'''-inyl}-pipérazine,
1-{2'-[(4''-(bromo)-phényl)-(4'''-(fluoro)-phényl)-méthoxy]-éthyl}-4-{prop-2''''-inyl}-pipérazine,
1-{2'-[(4''-(trifluorométhyl)-phényl)-(4'''-(fluoro)-phényl)-méthoxy]-éthyl}-4-{prop-2''''-inyl}-pipérazine,
1-{2'-[bis-(4''-(chloro)-phényl)-méthoxy]-éthyl} -4-{prop-2'''-inyl}-pipérazine, et
1-{2'-[bis-(4''-(chloro)-phényl)-méthoxy]-éthyl} -4-{prop-2'''-ényl}-pipérazine.

**8.** Procédé selon la revendication 1, caractérisé en ce qu'on réduit des 1-{ω-[bis-(phényl)-alcoxy]-alcoyl}-4-{alcynyl}-pipérazines répondant à la formule générale I, dans lesquelles $R_1$, $R_2$, $R_3$, $R_4$, $R_5$ et n ont les significations indiquées dans les revendications 1 à 6 et $R_6$ représente un reste alcynyle, en 1-{ω-[bis-(phényl)-alcoxy]-alcoyl}-4-{alcényl}-pipérazines répondant à la formule générale I, dans lesquelles $R_6$ représente un reste alcényle.

**9.** Procédé selon la revendication 1, caractérisé en ce qu'on réduit des 1-{ω-[bis-(phényl)-alcoxy]-alcoyl}-4-{alcényl}-pipérazines ou -4-{alcynyl}-pipérazines, substituées par 1 ou un plusieurs restes benzyloxy et répondant à la formule générale

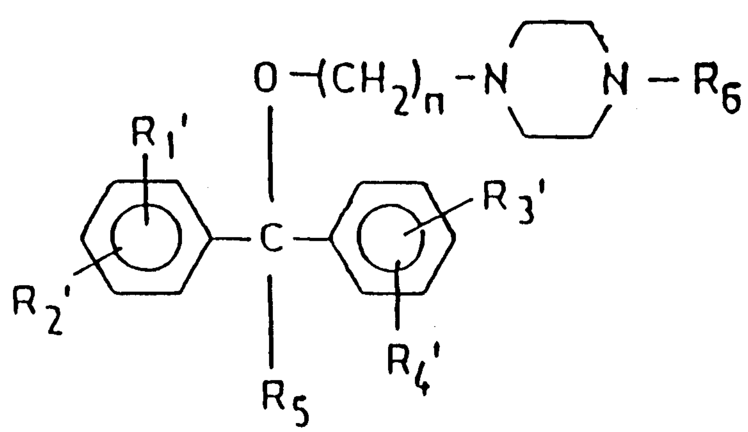

(Ia)

dans laquelle

| | |
|---|---|
| $R_5$, $R_6$ et n | ont les significations indiquées dans les revendications 1 à 6, et |
| au moins un des restes $R_1'$, $R_2'$, $R_3'$ et $R_4'$ | représente le reste phényl-alcoxy en $C_1$-$C_4$ |
| et les autres restes éventuels $R_1'$, $R_2'$, $R_3'$ et $R_4'$ | ont les significations indiquées dans les revendications 1 à 6, |

en pipérazines de formule générale I, dans lesquelles au moins un des restes $R_1$, $R_2$, $R_3$ et $R_4$

représente un groupe hydroxy.

**10.** Procédé selon la revendication 1, caractérisé en ce qu'on utilise des complexes d'aluminium comme agent de réduction.

**11.** Procédé selon l'une des revendications 1 à 10, caractérisé en ce qu'on travaille avec des solvants inertes vis-à-vis des partenaires réactionnels.